# EUROPEAN PATENT APPLICATION

(11) **EP 4 140 997 A1**
(43) Date of publication of application: **01.03.2023**
(21) Application number: 21804225.7
(22) Date of filing: 30.04.2021
(51) Int. Cl.: C07D 401/14, C07D 519/00, C07D 487/02, A61K 31/4439, A61P 43/00, A61P 35/00, A61P 35/02, A61P 35/04

(54) **PYRIDINE ACETAMIDE DERIVATIVE SERVING AS CDK INHIBITOR, AND PREPARATION METHOD THEREFOR AND USE THEREOF**

(30) Priority: 12.05.2020 CN 202010398063
(71) Applicant: Suzhou Alphama Biotechnology Co., Ltd., Suzhou, Jiangsu 215000 (CN)
(72) Inventor: ZHENG, Suxin, Suzhou, Jiangsu 215000 (CN); XIE, Chengying, Shanghai 201203 (CN); ZHENG, Mingyue, Shanghai 201203 (CN); LU, Xiaojie, Suzhou, Jiangsu 215000 (CN); QIAO, Gang, Suzhou, Jiangsu 215000 (CN)
(74) Representative: Ipside
(86) International application number: PCT/CN2021/091707
(87) International publication number: WO 2021/227906

(57) **Abstract**

The present invention relates to the technical field of pyridine acetamide derivatives, and specifically relates to a pyridine acetamide derivative serving as a CDK inhibitor, and a preparation method therefor and a use thereof. The pyridine acetamide derivative exhibits excellent CDK9/CDK7 enzyme inhibitory activity, and can be used for preparing a medication for treating cancers; and the cancers particularly are blood cancers, including acute myeloid leukemia, multiple myeloma, chronic lymphocytic leukemia, follicular lymphoma and the like, and solid tumors, including breast cancer, prostate cancer, ovarian cancer, hepatocellular carcinoma, pancreatic cancer, kidney cancer, stomach cancer, colorectal cancer, lung cancer and the like.

## Description

### TECHNICAL FIELD

The present disclosure belongs to the technical field of pyridine acetamide derivatives, and particularly relates to a pyridine acetamide derivative as a CDK inhibitor, a preparation method therefor and use thereof.

### BACKGROUND OF THE INVENTION

Cyclin dependent protein kinases (CDKs) are a type of serine/threonine kinase, and play a key role in cell cycle regulation and cell transcription process, which have gained more and more attention as important targets for treating cancers and other diseases. The CDKs family has 13 different subtypes with similar structures, and can be activated by about 10 cyclins, exerting different biological functions. According to different effecting mechanisms, they are generally classified into cell-cycle CDK (CDK1-6) and transcriptional CDK (CDK7-9). CDK9 mainly participates in the transcriptional regulation process, and the heterodimer composed of CDK9 and cyclin (T1, T2a, T2b, K) participates in forming a positive transcription elongation factor (P-TEFb), playing a key role in the transcriptional regulation process. CDK9 has a classical protein kinase fold, consisting of C-terminal and N-terminal kinase domains and a small C-terminal extension. CDK9 participates in the elongation process of transcription as a subunit of P-TEFb, and plays an important role in the transcription process. CDK9 regulates RNA transcription of short-life anti-apoptotic proteins. CDK9 regulates the expression of anti-apoptotic proteins by phosphorylating RNApol II. The transcription catalyzed by RNApol II is a multi-step process. The phosphorylation of Ser and Thr on the landem heptapeptide repeat sequence contained in the C-terminal domain of RNApol II large subunit plays an important role in the transcription process. While CDK9 in the P-TEFb complex phosphorylates RNApol II CTD Ser2, it also phosphorylates the sensitivity inducing factor DSIF and the negative elongation factor NELF. Phosphorylation makes NELF leave while DSIF converted to a positive transcription factor, followed by RNApol II entering the transcription elongation mode and the transcription elongation process starting¹.

CDK7, cyclin H and Mat1 form the catalytic center of CDK-activating kinase (CAK), thereby phosphorylating CDK1, 2, 4 and 6 to maintain the process of the cell cycle. On the other hand, CAK is a component of transcription factor TFIIH in RNA polymerase II (RNAPII), capable of phosphorylating the Rbp1 subunit of RNAPII C-terminal domain(CTD), while phosphorylation of CTD plays a role in regulating the transcription of RNAPII. Moreover, CDK7 is capable of activating CDK9 by the phosphorylation of T-loop, further regulating the transcription².

Inhibition of CDK9 leads to down-regulation of anti-apoptotic proteins Mcl-1, XIAP and the like, so that these anti-apoptotic proteins lose the ability to maintain the stability of tumor cells, thereby inducing the apoptosis of tumor cells. CDK9 also participates in the regulation of many cell functions. The selective inhibition of CDK9 can also act as a potential therapeutic strategy for tumor invasion and metastasis. Recently, inhibitors of CDK9 have been reported to down-regulate MYC proteins by inhibiting transcription and post-transcriptional modification, and thus have an inhibitory effect on MYC-driven tumors³. In addition, inhibition of CDK9 can increase the number of CD45⁺ cells in the tumor environment, increase the proportion of CD3⁺T cells, and activate dendritic cells, and therefore combining with CDK9 inhibitors can enhance the immune response of the tumor immune checkpoint blockage⁴. And inhibition of CDK7 can also cause cell cycle disruption and instability, activate the immune response of T cells at the same time, and also enhance the immune response of the tumor immune checkpoint blockage⁵.

CDKs are important targets for the treatment of tumors and other diseases, in the recent 20 years many CDK inhibitors have entered the clinic, and there have been selective CDK4/6 inhibitors on the market, which brings confidence and hope to develop selective CDK inhibitors. CDK9 as a novel anti-tumor drug target with great potential has gained more and more attention, there have been a plurality of selective CDK9 inhibitors entering the clinic (Fadraciclib, Zotitraciclib, KB-130742, AZD-4573, etc.), and CDK9 as an anti-tumor drug target has a broad application prospect.

At present, some CDK inhibitors have higher selectivity to CDK7, CDK9 or the like (WO2017001354, WO2018192273, WO2019154177, etc.). However, the compounds and test drugs disclosed in the prior art still have uncertainty in terms of efficacy, safety, selectivity, etc. Therefore, it is necessary to study and develop new selective CDK inhibitors.

Reference:
1. Boffo et al. CDK9 inhibitors in acute myeloid leukemia. J. Eep. Clin. Cancer Res. 37, 36 (2018) .
2. Kwak et al. Control of transcriptional elongation. Annu. Rev. Genet. 47, 483 (2013).
3. Blake et al. Application of a MYC degradation screen identifies sensitivity to CDK9 inhibitors in KRAS-mutant pancreatic cancer. Sci. Signal. 12, eeav7259 (2019).
4. Zhang et al. Targeting CDK9 Reactivates Epigenetically Silenced Genes in Cancer. Cell 175, 1244-1258 (2018).
5. Zhang et al. CDK7 Inhibition Protentiates Genome Instability Triggering Anti-tumor Immunity in Small Cell Lung Cancer. Cancer Cell. 37, 37-54 (2020).

### SUMMARY OF THE INVENTION

In order to solve the above problems in the prior art, an object of the present disclosure is to provide a pyridine acetamide derivative, a pharmaceutically acceptable salt thereof, a tautomer thereof or a stereoisomer thereof, so as to select a compound used as an inhibitor for CDK families, including but not limited to CDK9 or CDK7, with excellent properties in terms of efficacy, safety, selectivity and the like.

Another object of the present disclosure is to provide a method for preparing the derivative, the pharmaceutically acceptable salt thereof, the tautomer thereof, or the stereoisomer thereof.

In order to achieve the objects of the present disclosure, the present disclosure adopts the following technical solutions:
In a first aspect, the present disclosure provides a pyridine acetamide derivative, a pharmaceutically acceptable salt thereof, a tautomer thereof or a stereoisomer thereof, and the structure of the pyridine acetamide derivative is represented by formula (I): 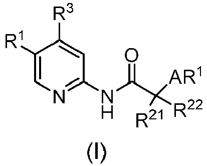 wherein:
R¹ is selected from hydrogen, halogen, cyano, substituted or unsubstituted C₁-C₃ alkyl, or substituted or unsubstituted C₁-C₃ alkoxy, wherein "substituted" refers to optionally further substituted with 1-3 halogens;
R²¹ and R²² are independently selected from hydrogen, halogen, hydroxyl, cyano, amino, substituted or unsubstituted C₁-C₃ alkyl, and substituted or unsubstituted C₁-C₃ alkoxy, wherein substituted refers to optionally substituted with 1-3 halogens, hydroxyl groups, cyano groups, or amino groups;
AR¹ is selected from phenyl, 5-6 membered heteroaryl, 8-10 membered fused aryl, and 8-10 membered fused heteroaryl, wherein phenyl, 5-6 membered heteroaryl, 8-10 membered fused aryl, and 8-10 membered fused heteroaryl are optionally further substituted with one or more R^{a};
R^{a} is independently selected from C₁-C₃ alkyl, hydroxyl, halogen, cyano, amino, C₁-C₃ alkoxy, C₃-C₆ cycloalkyl, C₃-C₆ heterocyclyl, phenyl, 5-6 membered heteroaryl, S(O)R^{b1}, S(O)₂R^{b1}, S(O)NH₂, S(O)NHR^{b1}, S(O)NR^{b1}R^{b2}, S(O)₂NH₂, S(O)₂NHR^{b1}, S(O)₂NR^{b1}R^{b2}, NHS(O)R^{b1}, NR^{b1}S(O)R^{b2}, NHS(O)₂R^{b1}, NR^{b1}S(O)₂R^{b2}, C(O)R^{b1}, C(O)OR^{b1}, OC(O)R^{b1}, NHC(O)R^{b1}, NR^{b1}C(O)R^{b2}, NHC(O)OR^{b1}, NR^{b1}C(O)OR^{b2}, C(O)NH₂, C(O)NHR^{b1} or C(O)NR^{b1}R^{b2}, wherein alkyl, alkoxy, cycloalkyl, heterocyclyl, phenyl, and 5-6 membered heteroaryl are optionally further substituted with 1-3 alkyl groups, hydroxyl groups, halogens, cyano groups, amino groups, alkoxy groups, acryloyl groups or acryloyl methylene groups;
R^{b1} and R^{b2} are independently selected from C₁-C₃ alkyl, 3-6 membered cycloalkyl or heterocyclyl, wherein alkyl, cycloalkyl, and heterocyclyl are optionally further substituted with 1-3 substituents selected from alkyl, hydroxyl, halogen, cyano, amino or alkoxy, or R^{b1} and R^{b2} together with the N atom to which they are bonded form a 3-7 membered heterocyclyl group;
R³ is selected from: 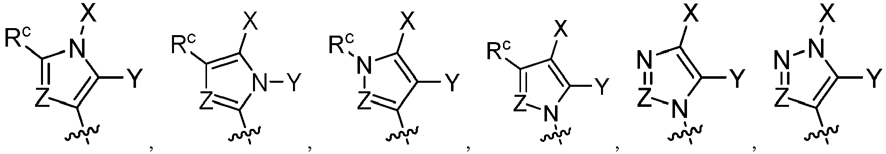 or 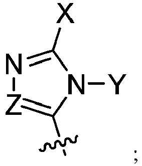
Z is N or CR^{c};
R^{c} is independently selected from hydrogen, halogen, cyano, C(O)NH₂, C(O)NHR^{b}, C(O)NR^{b1}R^{b2}, C(O)R^{b}, or C₁-C₃ alkyl, wherein alkyl is optionally further substituted with 1-3 substituents selected from alkyl, hydroxyl, halogen, cyano, amino, or alkoxy;
X and Y together with the atoms to which they are bonded form a 5-7 membered heterocyclyl or cycloalkyl group, wherein heterocyclyl comprises 1-2 heteroatoms selected from N, O, or S; the 5-7 membered heterocyclyl or cycloalkyl group is saturated or partially saturated and the ring carbon or ring S atom is optionally further substituted with 1-3 R^{d};
R^{d} is independently selected from halogen, hydroxyl, cyano, =O, or C₁-C₃ alkyl, wherein alkyl is optionally further substituted with 1-3 substituents selected from alkyl, hydroxyl, halogen, cyano, amino, or alkoxy;

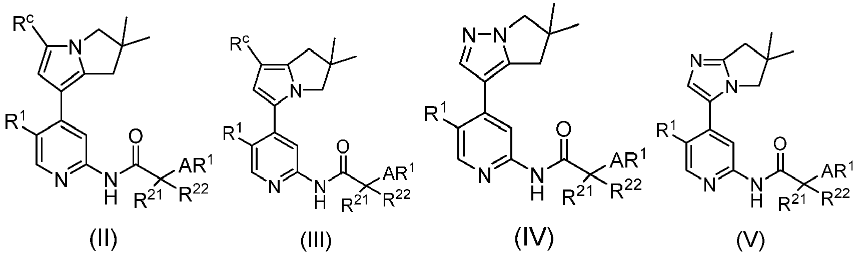

Preferably, the structure of the pyridine acetamide derivative is represented by formula (II):
wherein R¹, R²¹, R²², AR¹, and R^{c} have the same defined ranges as in the above general formula (I).

Preferably, the structure of the pyridine acetamide derivative is represented by formula (III):
wherein R¹, R²¹, R²², AR¹, and R^{c} have the same defined ranges as in the above general formula (I).

Preferably, the structure of the pyridine acetamide derivative is represented by formula (IV):
wherein R¹, R²¹, R²², and AR¹ have the same defined ranges as in the above general formula (I).

Preferably, the structure of the pyridine acetamide derivative is represented by formula (V):
wherein R¹, R²¹, R²², and AR¹ have the same defined ranges as in the above general formula (I).

Further preferably, the pyridine acetamide derivative is selected from any one of the following structures:

| Compound No. | Structure | Naming |
|---|---|---|
| 1 | 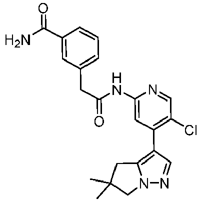 | 3-(2-((5-chloro-4-(5,5-dimethyl-5,6-dihydr o-4*H*-pyrrolo[1,2-*b*]pyrazol-3-yl)pyridin-2 -yl)amino)-2-oxoethyl)benzamide |
| 2-1 | 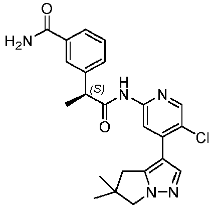 | (*S*)-3-(1-((5-chloro-4-(5,5-dimethyl-5,6-di hydro-4*H*-pyrrolo[1,2-*b*]pyrazol-3-yl)pyrid in-2-yl)amino)-1-oxopropan-2-yl)benzami de (assumed) |
| 2-2 | 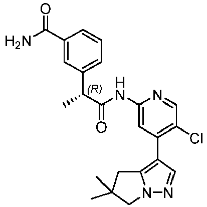 | (*R*)-3-(1-((5-chloro-4-(5,5-dimethyl-5,6-di hydro-4*H*-pyrrolo[1,2-*b*]pyrazol-3-yl)pyrid in-2-yl)amino)-1-oxopropan-2-yl)benzami de (assumed) |
| 3 | 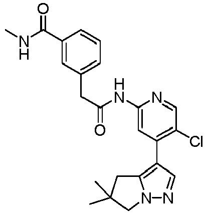 | 3-(2-((5-chloro-4-(5,5-dimethyl-5,6-dihydr o-4*H*-pyrrolo[1,2-*b*]pyrazol-3-yl)pyridin-2 -yl)amino)-2-oxoethyl)-*N*-methylbenzamid e |
| 4 | 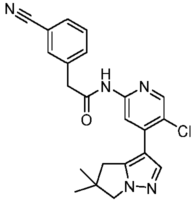 | *N*-(5-chloro-4-(5,5-dimethyl-5,6-dihydro-4 *H*-pyrrolo[1,2-*b*]pyrazol-3-yl)pyridin-2-yl) -2-(3-cyanophenyl)acetamide |
| 5 | 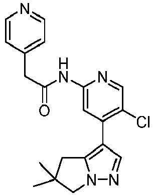 | *N*-(5-chloro-4-(5,5-dimethyl-5,6-dihydro-4 *H*-pyrrolo[1,2-*b*]pyrazol-3-yl)pyridin-2-yl) -2-(pyridin-4-yl)acetamide |
| 6 | 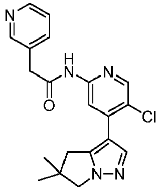 | *N*-(5-chloro-4-(5,5-dimethyl-5,6-dihydro-4 *H*-pyrrolo[1,2-*b*]pyrazol-3-yl)pyridin-2-yl) -2-(pyridin-3-yl)acetamide |
| 7 | 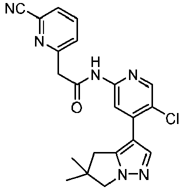 | *N*-(5-chloro-4-(5,5-dimethyl-5,6-dihydro-4 *H*-pyrrolo[1,2-*b*]pyrazol-3-yl)pyridin-2-yl) -2-(6-cyanopyridin-2-yl)acetamide |
| 8 | 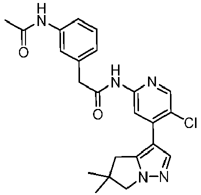 | 2-(3-acetylaminophenyl)-*N*-(5-chloro-4-(5, 5-dimethyl-5,6-dihydro-4*H*-pyrrolo[1,2-b] pyrazol-3-yl)pyridin-2-yl)acetamide |
| 9 | 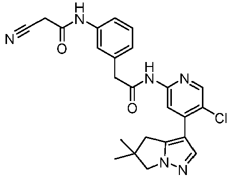 | *N*-(5-chloro-4-(5,5-dimethyl-5,6-dihydro-4 *H*-pyrrolo[1,2-*b*]pyrazol-3-yl)pyridin-2-yl) -2-(3-(2-cyanoacetylamino)phenyl)acetami de |
| 10 | 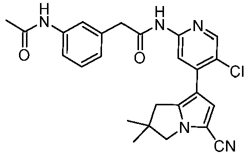 | 2-(3-acetylaminophenyl)-*N*-(5-chloro-4-(5 -cyano-2,2-dimethyl-2,3-dihydro-1*H*-pyrro lizin-7-yl)pyridin-2-yl)acetamide |
| 11-1 | 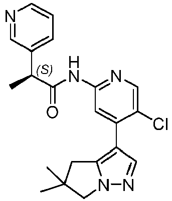 | (*S*)-*N*-(5-chloro-4-(5,5-dimethyl-5,6-dihyd ro-4*H*-pyrrolo[1,2-*b*]pyrazol-3-yl)pyridin-2-yl)-2-(pyridin-3-yl)propionamide (assumed) |
| 11-2 | 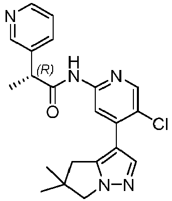 | (*R*)-*N*-(5-chloro-4-(5,5-dimethyl-5,6-dihyd ro-4*H*-pyrrolo[1,2-*b*]pyrazol-3-yl)pyridin-2-yl)-2-(pyridin-3-yl)propionamide (assumed) |
| 12 | 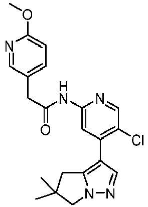 | *N*-(5-chloro-4-(5,5-dimethyl-5,6-dihydro-4 *H*-pyrrolo[1,2-b]pyrazol-3-yl)pyridin-2-yl) -2-(6-methoxypyridin-3-yl)acetamide |
| 13-1 | 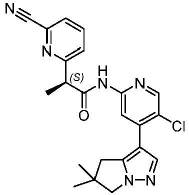 | (*S*)-*N*-(5-chloro-4-(5,5-dimethyl-5,6-dihyd ro-4*H*-pyrrolo[1,2-*b*]pyrazol-3-yl)pyridin-2-yl)-2-(6-cyanopyridin-2-yl)propionamid e (assumed) |
| 13-2 | 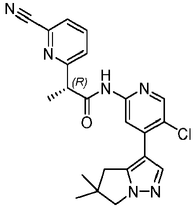 | (*R*)-*N*-(5-chloro-4-(5,5-dimethyl-5,6-dihyd ro-4*H*-pyrrolo[1,2-*b*]pyrazol-3-yl)pyridin-2-yl)-2-(6-cyanopyridin-2-yl)propionamid e (assumed) |
| 14-1 | 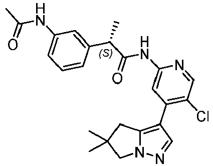 | (*S*)-2-(3-acetylaminophenyl)-*N*-(5-chloro-4-(5,5-dimethyl-5,6-dihydro-4*H*-pyrrolo[1, 2-*b*]pyrazol-3-yl)pyridin-2-yl)propionamid e (assumed) |
| 14-2 | 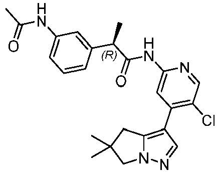 | (*R*)-2-(3-acetylaminophenyl)-*N*-(5-chloro-4-(5,5-dimethyl-5,6-dihydro-4*H*-pyrrolo[1, 2-b]pyrazol-3-yl)pyridin-2-yl)propionamid e (assumed) |
| 15-1 | 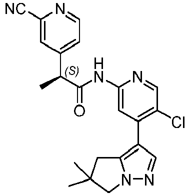 | (*S*)-*N*-(5-chloro-4-(5,5-dimethyl-5,6-dihyd ro-4*H*-pyrrolo[1,2-*b*]pyrazol-3-yl)pyridin-2-yl)-2-(2-cyanopyridin-4-yl)propionamid e (assumed) |
| 15-2 | 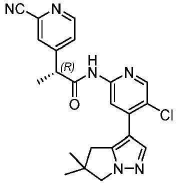 | (*R*)-*N*-(5-chloro-4-(5,5-dimethyl-5,6-dihyd ro-4*H*-pyrrolo[1,2-*b*]pyrazol-3-yl)pyridin-2-yl)-2-(2-cyanopyridin-4-yl)propionamid e (assumed) |
| 16-1 | 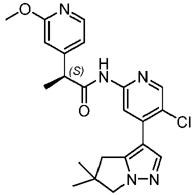 | (*S*)-*N*-(5-chloro-4-(5,5-dimethyl-5,6-dihyd ro-4*H*-pyrrolo[1,2-*b*]pyrazol-3-yl)pyridin-2-yl)-2-(2-methoxypyridin-4-yl)propionam ide (assumed) |
| 16-2 | 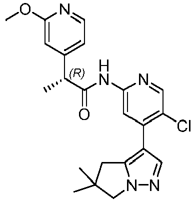 | (*R*)-*N*-(5-chloro-4-(5,5-dimethyl-5,6-dihyd ro-4*H*-pyrrolo[1,2-*b*]pyrazol-3-yl)pyridin-2-yl)-2-(2-methoxypyridin-4-yl)propionam ide (assumed) |
| 17-1 | 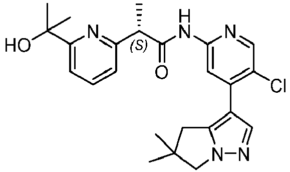 | (*S*)-*N*-(5-chloro-4-(5,5-dimethyl-5,6-dihyd ro-4*H*-pyrrolo[1,2-*b*]pyrazol-3-yl)pyridin-2-yl)-2-(6-(2-hydroxypropan-2-yl)pyridin-2-yl)propionamide (assumed) |
| 17-2 | 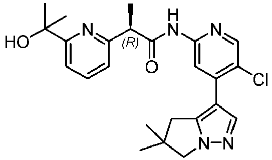 | (*R*)-*N*-(5-chloro-4-(5,5-dimethyl-5,6-dihyd ro-4*H*-pyrrolo[1,2-*b*]pyrazol-3-yl)pyridin-2-yl)-2-(6-(2-hydroxypropan-2-yl)pyridin-2-yl)propionamide (assumed) |
| 18-1 | 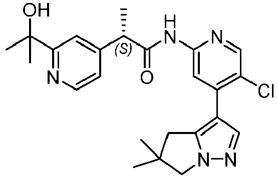 | (*S*)-*N*-(5-chloro-4-(5,5-dimethyl-5,6-dihyd ro-4*H*-pyrrolo[1,2-*b*]pyrazol-3-yl)pyridin-2-yl)-2-(2-(2-hydroxypropan-2-yl)pyridin-4-yl)propionamide (assumed) |
| 18-2 | 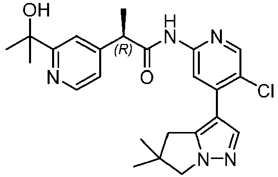 | (*R*)-*N*-(5-chloro-4-(5,5-dimethyl-5,6-dihyd ro-4*H*-pyrrolo[1,2-*b*]pyrazol-3-yl)pyridin-2-yl)-2-(2-(2-hydroxypropan-2-yl)pyridin-4-yl)propionamide (assumed) |
| 19-1 | 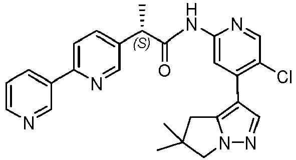 | (*S*)-2-([2,3'-bipyridin]-5-yl)-*N*-(5-chloro-4-(5,5-dimethyl-5,6-dihydro-4*H*-pyrrolo[1,2-*b*]pyrazol-3-yl)pyridin-2-yl)propionamide (assumed) |
| 19-2 | 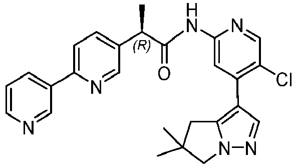 | (*R*)-2-([2,3'-bipyridin]-5-yl)-*N*-(5-chloro-4 -(5,5-dimethyl-5,6-dihydro-4*H*-pyrrolo[1,2 -*b*]pyrazol-3-yl)pyridin-2-yl)propionamide (assumed) |
| 20-1 | 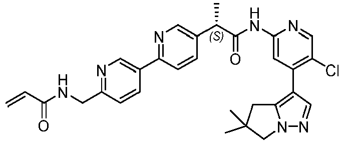 | (*S*)-*N*-((5-(1-((5-chloro-4-(5,5-dimethyl-5, 6-dihydro-4*H*-pyrrolo[1,2-*b*]pyrazol-3-yl)p yridin-2-yl)amino)-1-oxopropan-2-yl)-[2,3' -bipyridin]-6'-yl)methyl)acrylamide (assumed) |
| 20-2 | 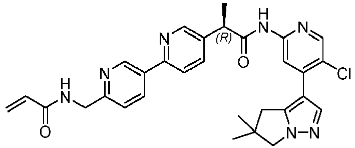 | (*R*)-*N*-((5-(1-((5-chloro-4-(5,5-dimethyl-5, 6-dihydro-4*H*-pyrrolo[1,2-*b*]pyrazol-3-yl)p yridin-2-yl)amino)-1-oxopropan-2-yl)-[2,3' -bipyridin]-6'-yl)methyl)acrylamide (assumed) |
| 21-1 | 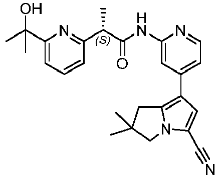 | (*S*)-*N*-(4-(5-cyano-2,2-dimethyl-2,3-dihydr o-1*H*-pyrrolizin-7-yl)pyridin-2-yl)-2-(6-(2-hydroxypropan-2-yl)pyridin-2-yl)propiona mide (assumed) |
| 21-2 | 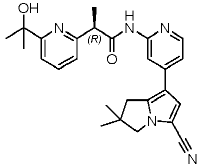 | (*R*)-*N*-(4-(5-cyano-2,2-dimethyl-2,3-dihyd ro-1*H*-pyrrolizin-7-yl)pyridin-2-yl)-2-(6-(2 -hydroxypropan-2-yl)pyridin-2-yl)propion amide (assumed) |
| 22-1 | 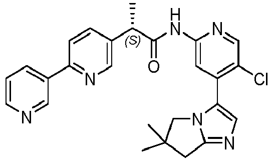 | (*S*)-2-([2,3'-bipyridin]-5-yl)-*N*-(5-chloro-4-(6,6-dimethyl-6,7-dihydro-5*H*-pyrrolo[1,2-a]imidazol-3-yl)pyridin-2-yl)propionamide (assumed) |
| 22-2 | 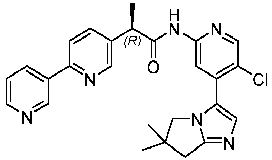 | (*R*)-2-([2,3'-bipyridin]-5-yl)-*N*-(5-chloro-4 -(6,6-dimethyl-6,7-dihydro-5*H*-pyrrolo[1,2 -a]imidazol-3-yl)pyridin-2-yl)propionamid e (assumed) |

In a second aspect, the present disclosure provides a preparation method of the pyridine acetamide derivative, the pharmaceutically acceptable salt thereof, the tautomer thereof, or the stereoisomer thereof as described in the first aspect, selected from one of the following three solutions:
Solution 1 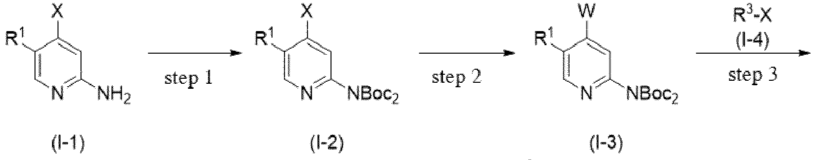 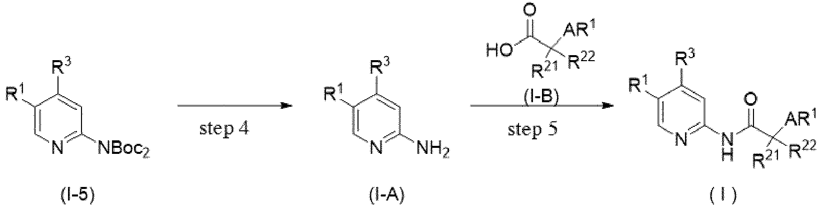
In step 1, the amine of the compound of general formula (I-1) is protected with Boc under an alkaline condition to obtain a compound of general formula (I-2);
In step 2, the compound of general formula (I-2) is in the presence of a metal catalyst under an alkaline condition to obtain a compound of general formula (I-3);
In step 3, the compound of general formula (I-3) and the compound of general formula (I-4) undergo Suzuki reaction in the presence of a metal catalyst and a ligand under an alkaline condition to obtain a compound of general formula (I-5);
In step 4, the Boc protecting group of the compound of general formula (I-5) is removed under an acidic condition to obtain a compound of general formula (I-A);
In step 5, the compound of general formula (I-A) and the compound of general formula (I-B) undergo a condensation reaction under an alkaline condition to obtain a compound of general formula (I).

Wherein R¹, AR¹, R²¹, R²², and R³ have the same defined ranges as in the above general formula (I).
Solution 2 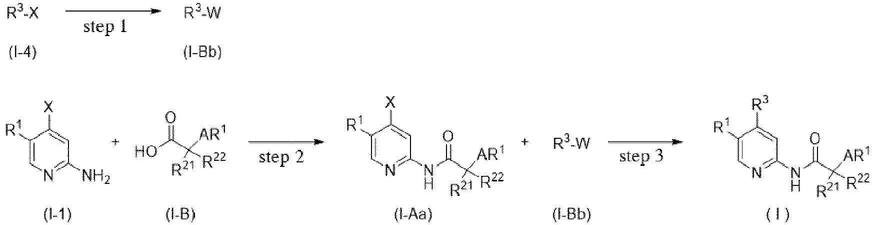
In step 1, the compound of general formula (I-4) is in the presence of a metal catalyst under an alkaline condition to obtain a compound of general formula (I-Bb);
In step 2, the compound of general formula (I-1) and the compound of general formula (I-B) undergo a condensation reaction under an alkaline condition to obtain a compound of general formula (I-Aa);
In step 3, the compound of general formula (I-Aa) and the compound of general formula (I-Bb) undergo a Suzuki reaction in the presence of a metal catalyst and a ligand under an alkaline condition to obtain a compound of general formula (I).

Wherein X is halogen, preferably bromine; and W is 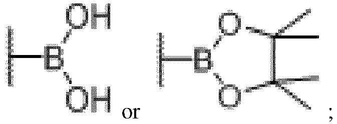 R¹, AR¹, R²¹, R²² and R³ have the same defined ranges as in the above general formula (I).
Solution 3 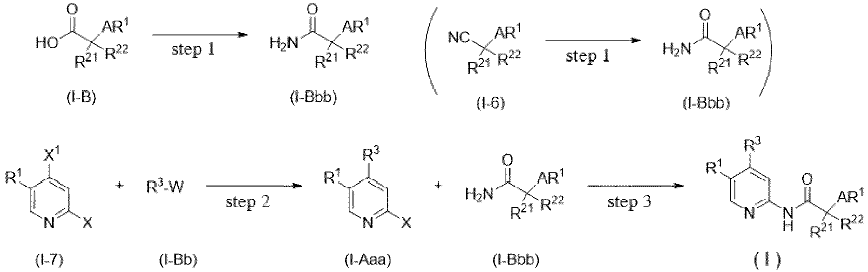
In step 1, the compound of general formula (I-B) and ammonium chloride undergo a condensation reaction under an alkaline condition to obtain a compound of general formula (I-Bbb); or the compound of general formula (I-6) is oxidized under an alkaline condition to obtain a compound of general formula (I-Bbb);
In step 2, the compound of general formula (I-7) and the compound of general formula (I-Bb) undergo Suzuki reaction in the presence of a catalyst under an alkaline condition to obtain a compound of general formula (I-Aaa);
In step 3, the compound of general formula (I-Aaa) and the compound of general formula (I-Bbb) undergo a condensation reaction under an alkaline condition to obtain a compound of general formula (I).

Wherein X is halogen, preferably chlorine; R¹, AR¹, R²¹, R²² and R³ have the same defined ranges as in the above general formula (I).
In the above preparation method, the reagent providing the alkaline condition is selected from an organic base or an inorganic base, the organic base includes, but is not limited to, one or more of triethylamine, N,N-diisopropylethylamine, n-butyllithium, lithium diisopropylamide, lithium bis(trimethylsilyl)amide, potassium acetate, sodium acetate, sodium tert-butoxide, sodium methoxide and potassium tert-butoxide, and the inorganic base is one or more of sodium hydride, potassium phosphate, sodium carbonate, potassium carbonate, potassium acetate, cesium carbonate, sodium hydroxide, potassium hydroxide, sodium bicarbonate and lithium hydroxide;
The reagent providing the acidic condition includes, but are not limited to, one or more of hydrogen chloride, 1,4-dioxane solution of hydrogen chloride, trifluoroacetic acid, formic acid, acetic acid, hydrochloric acid, sulfuric acid, methanesulfonic acid, nitric acid, and phosphoric acid;
The metal catalyst includes, but is not limited to, one or more of palladium/carbon, Raney nickel, tetrakis(triphenylphosphine)palladium, palladium dichloride, palladium acetate,
[1,1'-bis(diphenylphosphino)ferrocene]dichloridepalladium(Pd(dppf)Cl₂),
[1,1'-bis(diphenylphosphino)ferrocene]dichloridepalladium dichloromethane complex, bis(triphenylphosphine)palladium dichloride(Pd(PPh₃)Cl₂), and tris(dibenzylideneacetone)dipalladium(Pd₂(dba)₃)
The ligand includes, but is not limited to, one or more of 2-dicyclohexylphosphino-2,6'-dimethoxybiphenyl (SPhos), 4,5-bis(diphenylphosphino)-9,9-dimethylxanthene(XantPhos), 2-dicyclohexylphosphino-2,4,6-triisopropylbiphenyl(XPhos), 2-dicyclohexylphosphino-2'-(*N,N*-dimethylamino)biphenyl(DavePhos), 1,1'-bis(diphenylphosphino)ferrocene(Dppf) and 1,1'-binaphthyl-2.2'-diphemyl phosphine(BINAP), preferably 4,5-bis(diphenylphosphino)-9,9-dimethylxanthene(XantPhos);
The condensing agent includes, but is not limited to, one or more of dicyclohexylcarbodiimide(DCC), diisopropylcarbodiimide(DIC), 1-(3-dimethylaminopropyl)-3-ethylcarbodiimide(EDC), 1-(3-dimethylaminopropyl)-3-ethylcarbodiimide(EDCI), 2-(7-oxidobenzotriazol)-*N,N,N',N*'-tetramethyluronium hexafluorophosphate(HATU), 2-(1*H*-benzotriazol-1-yl)-1,1,3,3-tetramethyluronium tetrafluoroborate(TBTU), 1-hydroxybenzotriazole(HOBt) and 1-propylphosphonic anhydride(T3P).

The above reaction is preferably carried out in a solvent, and the solvent used includs, but not limited to: *N,N*-dimethylformamide, dimethyl sulfoxide, 1,4-dioxane, water, tetrahydrofuran, dichloromethane, 1,2-dichloroethane, acetic acid, methanol, ethanol, toluene, petroleum ether, ethyl acetate, n-hexane, acetone, diethyl ether, diethylene glycol, and mixtures thereof.

In a third aspect, the present disclosure provides a pharmaceutical composition, and the pharmaceutical composition comprises the pyridine acetamide derivative, the stereoisomer, the tautomer, and the pharmaceutically acceptable salt as described above;
Preferably, the pharmaceutical composition further comprises a pharmaceutically acceptable carrier and/or an excipient.

In a fourth aspect, the present disclosure provides an application of the pyridine acetamide derivative, the stereoisomer, the tautomer, the pharmaceutically acceptable salt according to the first aspect, or the pharmaceutical composition according to the third aspect in the preparation of medicaments for treating cancers, or in preparing inhibitors for CDK families, wherein the cancers are preferably blood cancer, including acute myeloid leukemia, multiple myeloma, chronic lymphocytic leukemia, follicular lymphoma, etc., and solid tumors, including breast cancer, prostate cancer, ovarian cancer, hepatocellular carcinoma, pancreatic cancer, renal cancer, gastric cancer, colorectal cancer, lung cancer, etc.

The present disclosure also provides a method for using the pyridine acetamide derivative as a CDK inhibitor to treat cancers, by administering an effective amount of the compound to a subject suffering from cancer. These compounds of the present disclosure can be further administered in combination with a therapeutically effective amount of one or more reagents for the treatment of cancer, wherein examples of the reagents include, for example, radiation, alkylating agents, angiogenesis inhibitors, anti-mitotic agents, anti-proliferative agents, aurora kinase inhibitors, cell death activators(for example, inhibitors of Bcl-2, BclxL, Bcl-w, Bfl-1, or Mcl-1), activators of the death receptor pathway, Bcr-Abl kinase inhibitors, BET(bromodomain protein) inhibitors, inhibitors of the Ras signaling pathway(for example, inhibitors of MEK, Raf or Ras), antibodies, BiTE(bispecific T-cell engager) antibodies, antibody-drug conjugates, biological response modifiers, cyclin-dependent kinase inhibitors, cell cycle inhibitors, cyclooxygenase-2 inhibitors, DVD (dual variable domain) antibodies, leukemia viral oncogene homolog (ErbB2) receptor inhibitors, growth factor inhibitors, heat shock protein (HSP)-90 inhibitors, histone deacetylase(HDAC) inhibitors, hormone therapies, immunodrugs, inhibitors of apoptosis proteins(IAP), kinase inhibitors, tumor kinesin inhibitors, Jak2 inhibitors, mammalian target of rapamycin inhibitors, microRNAs, mitogen-activated extracellular signal-regulated kinase inhibitors, poly ADP (adenosine diphosphate)-ribose polymerase (PARP) inhibitors, platinum chemotherapeutic agents, polo-like kinase (Plk) inhibitors, phosphoinositol-3 kinase inhibitors, proteasome inhibitors, small interfering ribonucleic acid(siRNA), topoisomerase inhibitors, ubiquitin ligase inhibitors, and the like, as well as one or more combinations of these agents.

As used herein, the term "effective amount" refers to an amount of a compound or composition sufficient to significantly and positively alter the symptoms and/or conditions to be treated (eg, to provide a positive clinical response). An effective amount of an active ingredient for use in a pharmaceutical composition will vary with the particular symptom being treated, the severity of the symptom, the duration of the treatment, the nature of the synchronous treatment, the one or more specific active ingredients used, one or more pharmaceutically acceptable excipients/carriers used, the knowledge and the professional skills of the attending physician, and the like.

In particular, an effective amount of compounds of formula (I) used in cancer treatment is an amount sufficient to relieve cancer symptoms of human, to slow the progression of cancer, or to reduce the risk of symptom deterioration in patients suffering from cancer.

### Explanation of the Terms

Unless stated to the contrary, the terms used in the specification and the claims are defined as follows:
"Alkyl" refers to a saturated aliphatic hydrocarbon group, including a saturated linear or branched monovalent hydrocarbon group of 1-20 carbon atoms, or 1-10 carbon atoms, or 1-6 carbon atoms, or 1-4 carbon atoms, or 1-3 carbon atoms, or 1-2 carbon atoms, wherein the alkyl may be independently optionally substituted with one or more substituents described herein. Further examples of alkyl groups include, but are not limited to, methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, and the like. The alkyl group may be optionally substituted or unsubstituted.
"Alkenyl" refers to a linear or branched monovalent hydrocarbon group of 2-12 carbon atoms, or 2-8 carbon atoms, or 2-6 carbon atoms, or 2-4 carbon atoms, wherein at least one C-C is an sp² double bond, wherein the alkenyl group may be independently optionally substituted with one or more substituents described herein, wherein specific examples include, but are not limited to, vinyl, allyl, butenyl, and the like. The alkenyl group may be optionally substituted or unsubstituted.
"Cycloalkyl" refers to a saturated or partially unsaturated monocyclic or polycyclic ringlike hydrocarbon substituent, and the cycloalkyl group comprises 3-20 carbon atoms, preferably 3-12 carbon atoms, more preferably 3-6 carbon atoms. Non-limiting examples of monocyclic cycloalkyl include, but are not limited to, cyclopropyl, cyclobutyl, cyclopentyl, cyclopentenyl, cyclohexyl, cyclohexenyl, cyclohexadienyl, cycloheptyl, cycloheptatrienyl, cyclooctyl, and the like; the polycyclic cycloalkyl groups include spirocyclic, fused-ring, and bridged-ring cycloalkyl groups. The cycloalkyl group may be optionally substituted or unsubstituted. "Spirocycloalkyl" refers to a polycyclic group of 5-18 members with two or more cyclic structures and one carbon atom (referred to as spiro atom) shared between the monocyclic rings, and one or more double bonds are contained in the rings, but none of the rings have a completely conjugated π-electron aromatic system. Preferably, it is 6-14 membered, more preferably 7-10 membered. The spirocycloalkyl group is divided into single spirocyclic, double spirocyclic or polyspirocyclic alkyl according to the number of spiro atoms shared between rings, preferably it is single spirocyclic or double spirocyclic alkyl groups, and preferably it is 4 membered/5 membered, 4 membered/6 membered, 5membered/5 membered or 5 membered/6 membered.

Non-limiting examples of "spirocycloalkyl" include, but are not limited to:

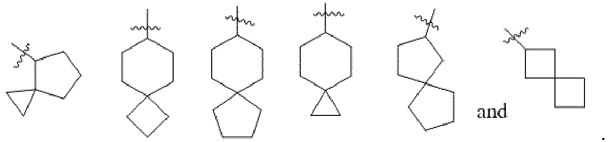

"Fused cycloalkyl" refers to an all-carbon polycyclic group of 5-18 members with two or more cyclic structures sharing a pair of carbon atoms with each other, and one or more rings may contain one or more double bonds, but none of the rings have a completely conjugated π-electron aromatic system. Preferably it is 6-12 members, more preferably 7-10 members. It can be divided into bicyclic, tricyclic, tetracyclic or polycyclic fused cycloalkyl group according to the number of constituting rings, preferably it is bicyclic or tricyclic, and more preferably it is 5 membered/5 membered or 5 membered/6 membered bicyclic alkyl groups. Non-limiting examples of "fused cycloalkyl" include, but are not limited to:

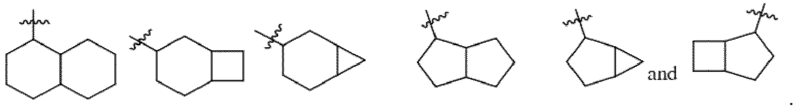

"Bridged cycloalkyl" refers to an all-carbon polycyclic group of 5-18 members with two or more cyclic structures sharing two non-directly-bonded carbon atoms with each other, and one or more rings may contain one or more double bonds, but none of the rings have a completely conjugated π-electron aromatic system. Preferably it is 6-12 members, more preferably 7-10 members. It can be divided into bicyclic, tricyclic, tetracyclic or polycyclic bridged cycloalkyl group according to the number of constituting rings, preferably it is bicyclic, tricyclic or tetracyclic, and more preferably it is bicyclic or tricyclic. Non-limiting examples of "bridged cycloalkyl" include, but are not limited to:

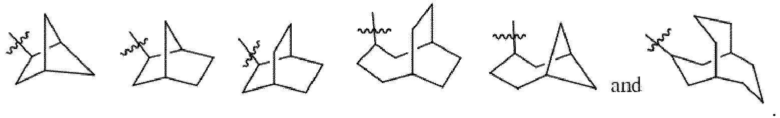

The cycloalkyl ring may be fused to an aryl, heteroaryl, or heterocyclic ring, wherein the ring bonded to the parent structure is the cycloalkyl group, and non-limiting examples include indanyl, tetrahydronaphthyl, benzocycloheptyl, and the like.

"Heterocyclyl", "heterocycle" or "heterocyclic" may be used interchangeably in the present disclosure, all referring to a saturated or partially unsaturated monocyclic, bicyclic, or tricyclic non-aromatic heterocyclic group comprising 3-12 ring atoms, wherein at least one ring atom is a heteroatom, such as oxygen, nitrogen, sulfur atom, and the like. It is preferred to have a 5-7 membered single ring or 7-10 membered double or triple rings, which may contain 1, 2, or 3 atoms selected from nitrogen, oxygen, and/or sulfur. Examples of "heterocyclyl" include, but are not limited to, morpholinyl, oxetanyl, thiomorpholinyl, tetrahydropyranyl, 1,1-dioxo-thiomorpholinyl, piperidinyl, 2-oxo-piperidinyl, pyrrolidinyl, 2-oxo-pyrrolidinyl, piperazine-2-one, 8-oxa-3-aza-bicyclo[3.2.1]octyl and piperazinyl. The heterocyclic ring may be fused to an aryl, heteroaryl, or cycloalkyl ring, wherein the ring bonded to the parent structure is the heterocyclyl group. The heterocyclyl group may be optionally substituted or unsubstituted.

"Spiroheterocyclyl" refers to a polycyclic group of 5-18 members with two or more cyclic structures sharing an atom with each other between the monocyclic rings, and one or more double bonds are contained in the rings, but none of the rings have a completely conjugated π-electron aromatic system, wherein one or more ring atoms are selected from nitrogen, oxygen, sulfur, or S(O)ₘ heteroatoms, with the remaining ring atoms being carbon, m=1 or 2. Preferably it is 6-14 membered, more preferably 7-10 membered. The spiroheterocyclyl group is divided into single spiroheterocyclyl, double spiroheterocyclyl or polyspiroheterocyclyl according to the number of spiro atoms shared between rings, and preferably it is single spiroheterocyclyl or double spiroheterocyclyl. More preferably it is 4 membered/4 membered, 4 membered/5 membered, 4 membered/6 membered, 5 membered/5 membered or 5 membered/6 membered single spiroheterocyclyl. Non-limiting examples of "spiroheterocyclyl" include, but are not limited to:

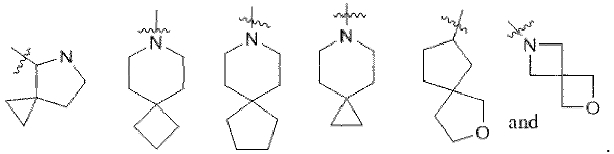

"Fused heterocyclyl" refers to an all-carbon polycyclic group comprising two or more cyclic structures sharing a pair of atoms with each other, and one or more rings may contain one or more double bonds, but none of the rings have a completely conjugated π-electron aromatic system, wherein one or more ring atoms are selected from nitrogen, oxygen, sulfur, or S(O)ₘ heteroatoms, with the remaining ring atoms being carbon, m=1 or 2. Preferably it is 6-14 membered, more preferably 7-10 membered. It can be divided into bicyclic, tricyclic, tetracyclic or polycyclic fused heterocyclic group according to the number of constituting rings, preferably it is bicyclic or tricyclic, and more preferably it is 5 membered/5 membered or 5 membered/6 membered bicyclic fused heterocyclyl. Non-limiting examples of "fused heterocyclyl" include, but are not limited to:

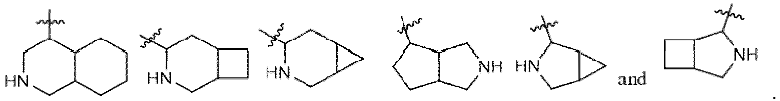

"Bridged heterocyclyl" refers to an polycyclic group of 5-18 members with two or more cyclic structures sharing two non-directly-bonded atoms with each other, and one or more rings may contain one or more double bonds, but none of the rings have a completely conjugated π-electron aromatic system, wherein one or more ring atoms are selected from nitrogen, oxygen, sulfur, or S(O)ₘ heteroatoms, with the remaining ring atoms being carbon, m=1 or 2. Preferably it is 6-14 membered, more preferably 7-10 membered. It can be divided into bicyclic, tricyclic, tetracyclic or polycyclic bridged heterocyclyl according to the number of constituting rings, preferably it is bicyclic, tricyclic or tetracyclic, and more preferably it is bicyclic or tricyclic.

Non-limiting examples of "bridge heterocyclyl" include, but are not limited to:

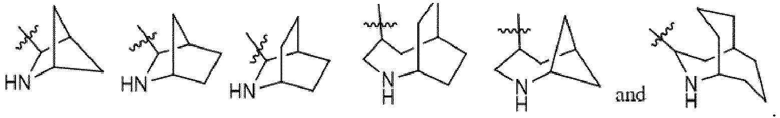

"Aryl" refers to a carbocyclic aromatic system comprising one or two rings, wherein the rings can be bonded together in a fused manner. The term "aryl" includes aromatic group such as phenyl, naphthyl, tetrahydronaphthyl. Preferred aryl groups are C₆-C₁₀ aryl groups, more preferably aryl groups are phenyl and naphthyl, and most preferably it is phenyl. The aryl group may be substituted or unsubstituted. The "aryl" group may be fused with heteroaryl, heterocyclyl, or cycloalkyl, wherein the ring bonded to the parent structure is the aryl ring. Non-limiting examples include, but is not limited to:

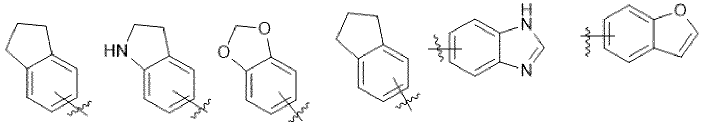

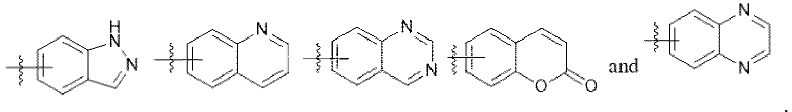

"Heteroaryl" refers to an aromatic 5-6 membered monocyclic or 9-10 membered bicyclic ring, which may contain 1 to 4 atoms selected from nitrogen, oxygen, and/or sulfur. Examples of "heteroaryl" include, but are not limited to, furyl, pyridyl, 2-oxo-1,2-dihydropyridyl, pyridazinyl, pyrimidinyl, pyrazinyl, thienyl, isoxazolyl, oxazolyl, oxadiazolyl, imidazolyl, pyrrolyl, pyrazolyl, triazolyl, tetrazolyl, thiazolyl, isoxazolyl, 1,2,3-thiadiazolyl, benzodioxolyl, benzimidazolyl, indolyl, isoindolyl, 1,3-dioxo-isoindolyl, quinolinyl, indazolyl, benzoisothiazolyl, benzoxazolyl, and benzisoxazolyl. Heteroaryl may be optionally substituted or unsubstituted. The heteroaryl ring may be fused to aryl, heterocyclyl, or cycloalkyl ring, wherein the ring bonded to the parent structure is the heteroaryl ring. Non-limiting examples include, but are not limited to:

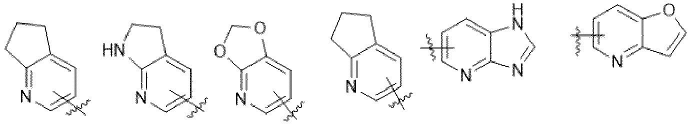

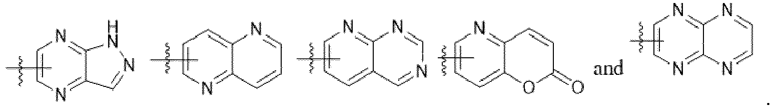

"Alkoxy" refers to a group of (alkyl-O-). Wherein, the alkyl group refers to the related defination herein. C₁-C₆ alkoxy is preferred. Its examples include, but are not limited to, methoxy, ethoxy, n-propoxy, isopropoxy, n-butoxy, isobutoxy, tert-butoxy, and the like.

"Haloalkyl" refers to an alkyl group having one or more halogen substituents, wherein the alkyl group has the meaning as described in the present disclosure. Examples of haloalkyl include, but are not limited to, fluoromethyl, difluoromethyl, trifluoromethyl, perfluoroethyl, 1,1-dichloroethyl, 1,2-dichloropropyl, and the like.

"Hydroxyl" refers to -OH group.

"Halogen" refers to fluorine, chlorine, bromine, and iodine, preferably fluorine, chlorine, and bromine. "Amino" refers to -NH₂.

"Cyano" refers to -CN.

"Nitro" refers to -NO₂.

"Benzyl" refers to -CH₂-phenyl.

"Carboxyl" refers to -C(O)OH.

"Acetyl" refers to -C(O)CH₃ or Ac.

"Carboxylate" refers to -C(O)O(alkyl) or (cycloalkyl), wherein the definition of alkyl and cycloalkyl is as described above.

"Optional" means that the event described may, but need not, occur. For example, the description "AR¹ is optionally substituted with one to more R^{c}" contains the case where the AR¹ group may be substituted with 1 to more R^{c} or not substituted with R^{c}.

"Substituted" means that one or more hydrogen atoms in a group, preferably at most 5, more preferably 1-3 hydrogen atoms are substituted with a corresponding number of substituents independently from each other. It goes without saying that the substituents are only at their possible chemical positions, and those skilled in the art can determine (through experimentation or theory) possible or impossible substitutions without excessive effort. For example, an amino or hydroxyl group with free hydrogen bonded to carbon atoms with an unsaturated (eg, olefinic) bond may be unstable.

As described herein, "substitution" or "substituted", as not particularly pointed out, means that the group can be substituted with one or more groups selected from the followings: alkyl, alkenyl, alkynyl, alkoxy, alkylthio, alkylamino, halogen, sulfhydryl, hydroxyl, nitro, cyano, cycloalkyl, heterocyclyl, aryl, heteroaryl, cycloalkoxy, heterocycloalkoxy, cycloalkylthio, heterocyclylthio, amino, haloalkyl, hydroxyalkyl, carboxyl, carboxylate, =O, -C(O)R^{b}, -OC(O)R^{b}, -NR^{b}R^{b}, -C(O)NR^{b}R^{b}, -NR^{b}C(O)R^{b}, -S(O)NR^{b}R^{b} or -S(O)₂NR^{b}R^{b}, wherein the definition of R^{b} is as described in general formula (I).

The use of stereochemical definitions and conventions in the present disclosure generally refers to the following document:
S.P.Parker,Ed.,McGraw-Hill Dictionary of Chemical Terms(1984)McGraw-HillBook Company,New York;and Eliel,E.and Wilen,S.,"Stereochemistry of Organic Compounds", John Wiley&Sons,Inc.,New York,1994. The compounds of the present disclosure may contain asymmetric centers or chiral centers, and therefore there are different stereoisomers. All stereoisomeric forms of the compounds disclosed herein include, but not limited to, diastereomers, enantiomers and atropisomers, as well as mixtures thereof such as racemic mixtures, forming part of the present invention. Diastereomers can be separated into their individual diastereoisomer on the basis of their physical and chemical differences by chromatography, crystallization, distillation, sublimation or the like. Enantiomers can be separated by converting the enantiomeric mixture into a diastereomeric mixture in a manner of reacting with an appropriate optically active compound (e.g., chiral auxiliary such as a chiral alcohol or Mosher's acid chloride), separating the diastereoisomers and converting the individual diastereoisomers to the corresponding pure enantiomers. The intermediates and compounds of the present disclosure may exist in different tautomeric forms and all such forms are encompassed within the scope of the present disclosure. Many organic compounds exist in optically active forms, i.e., they have the ability to rotate the plane of plane-polarized light. In describing an optically active compound, the prefixes D and L, or R and S, are used to denote the absolute configuration of the molecule about its chiral center(s). The prefixes d and 1, or (+) and (-) are employed to designate the sign of rotation of plane-polarized light by the compound, (-) or 1 meaning that the compound is levorotatory, (+) or d meaning that the compound is dextrorotatory. The atoms or atomic groups of these stereoisomers are bonded in the same order, but their three-dimensional structures are different. A specific stereoisomer may be an enantiomer, and a mixture of such isomers is often called an enantiomeric mixture. A 50:50 mixture of enantiomers is referred to as a racemic mixture or a racemate, which may result in no stereoselection or stereospecificity in a chemical reaction process. The term "racemic mixture" or "racemate" refers to an equimolar mixture of two enantiomers, devoid of optical activity.

"Tautomer" or "tautomeric form" refers to structural isomers of different energies which are interconvertible via a low energy barrier. For example, proton tautomers (i.e., prototropic tautomers) include interconversions via migration of a proton, such as keto-enol and imine-enamine isomerizations. Valence tautomers include interconversions by reorganization of the bonding electrons. Unless otherwise indicated, structure formulas depicted in the present disclosure include all isomeric forms (e.g., enantiomeric, diastereomeric, and geometric): for example, R and S configurations with asymmetric center, (Z) and (E) isomers of double bond, and (Z) and (E) conformational isomers. Therefore, a single stereochemical isomer as well as its enantiomer, diastereomer, or geometric isomer mixtures of the compounds of the present disclosure are within the scope of the present disclosure.

"Pharmaceutically acceptable salt" refers to a salt of the compound of the present disclosure that has safety and efficacy when used in humans or animals. The salt of the compound can be obtained by obtaining a corresponding addition salt with a sufficient amount of base or acid in a pure solution or a suitable inert dissolution. Pharmaceutically acceptable base addition salts include sodium, potassium, calcium, ammonium, organic ammonia, or magnesium salts, etc., pharmaceutically acceptable acid addition salts include inorganic acid salts and organic acid salts including hydrochloric acid, hydrobromic acid, carbonic acid, hydrogen carbonate, phosphoric acid, monohydrogen phosphate, dihydrogen phosphate, sulfuric acid, monohydrogen sulfate, acetic acid, maleic acid, malonic acid, succinic acid, fumaric acid, phthalic acid, benzenesulfonic acid, p-toluenesulfonic acid, citric acid, methanesulfonic acid, and the like (see Berge et al.,"Pharmaceutical Salts", Journal of Pharmaceutical Science 66:1-19(1977)).

Compared with the prior art, the pyridine acetamide derivative as a CDK inhibitor provided by the present disclosure has the following beneficial effects:
The present disclosure provides a novel structure CDK inhibitor. Test results show that the pyridine acetamide derivative exhibits excellent CDK7/CDK9 kinase inhibitory activity, and can be used for preparing medicaments for treating cancers, especially blood cancer, including acute myeloid leukemia, multiple myeloma, chronic lymphocytic leukemia, follicular lymphoma, etc,, and solid tumors, including breast cancer, prostate cancer, ovarian cancer, hepatocellular carcinoma, pancreatic cancer, kidney cancer, gastric cancer, colorectal cancer, lung cancer and other diseases.

### BRIEF DESCRIPTION OF THE DRAWINGS

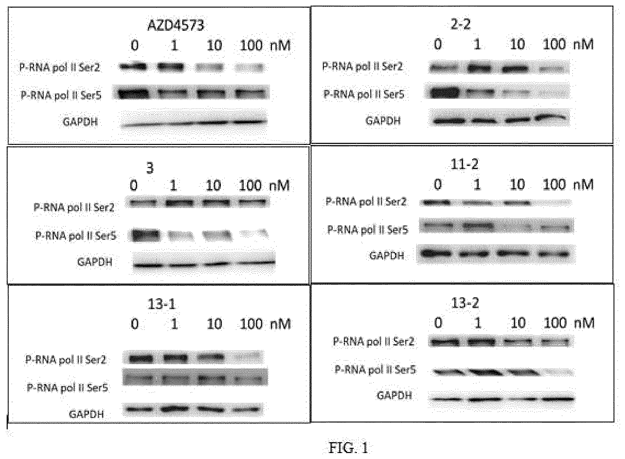

FIG. 1 is an SDS-PAGE electrophoresis detection diagram of the inhibition effect of the compounds 2-2, 3, 11-2, 13-1, 13-2, and AZD4573 on the phosphorylation of Mv4-11 cell RNA pol II Ser 2/5.

### DETAILED DESCRIPTION OF THE PREFERRED EMBODIMENTS

The method of the present disclosure is described below through specific embodiments, so that the technical solution of the present disclosure is easier to understand and master, but the present disclosure is not limited thereto. The ¹H NMR spectrum in the following embodiments was determined with a Bruker instrument (400 MHz), and the chemical shift was expressed in ppm. Tetramethylsilane internal standard (0.00 ppm) was used. Expression of ¹H NMR: s=singlet, d=doublet, t=triplet, q=quartet, m=multiplet, br=broadening, dd=doublet of doublet, dt = doublet of triplet. If a coupling constant is provided, its unit is Hz.

The mass spectrum was determined by LC/MS instrument, and the ionization mode was ESI.

High performance liquid chromatograph model: Agilent 1260, Thermo Fisher U3000; column model: Waters xbrige C18 (4.6*150 mm, 3.5 µm); mobile phase: A:ACN, B:Water (0.1% H₃PO₄); flow rate: 1.0 mL/min; gradient: 5% A for 1 min, increase to 20%A within 4 min, increase to 80%A within 8 min, 80%A for 2min, back to 5%A within 0.1 min; wavelength: 220 nm; column temperature box: 35°C.

Thin-layer chromatography silica gel plate uses Yantai Huanghai HSGF 254 or Qingdao GF254 silica gel plate, the size of the silica gel plate used for thin layer chromatography (TLC) is 0.2 mm to 0.3 mm, and the size for product separating and purifing thin-layer chromatography is 0.4 mm to 0.5 mm.

Column chromatography generally uses Yantai Huanghai silica gel 200-300 mesh silica gel as a carrier.

In the following embodiments, unless otherwise indicated, all temperatures are in degrees Celsius; unless otherwise indicated, all starting materials and reagents are commercially available or synthesized according to known methods, and commercially available starting materials and reagents are directly used without further purification; unless otherwise indicated, commercially available manufacturers include, but are not limited to, National Medicine Group, J&K Scientific Ltd., TCI (Shanghai) Development Co. Ltd., Shanghai Bide Pharmatech Ltd., Meryer (Shanghai) Chemical Technology Co., Ltd., et al.
CD₃OD: deuterated methanol
CDCl₃: deuterated chloroform.
DMSO-*d₆*: deuterated dimethyl sulfoxide
Pd₂(dba)₃: tris (dibenzylideneacetone) dipalladium
Pd(dppf)Cl₂: [1,1'-bis(diphenylphosphino)ferrocene]palladium dichloride
XantPhos: 4,5-bis-diphenylphosphine-9,9-dimethylxanthene
XPhos: 2-dicyclohexylphosphine-2,4,6-triisopropylbiphenyl
HATU: 2-(7-oxidobenzotriazol)-*N,N,N',N*'-tetramethyluronium hexafluorophosphate
TLC: Thin Layer Chromatography
HPLC: High Performance Liquid Chromatography
Purity: purity
&: and

The hydrogen atmosphere means that the reaction flask is connected to a hydrogen balloon with a volume of about 1 L.

If no special statement is given in the embodiments, the solution in the reaction refers to an aqueous solution. If no special statement is given in the embodiments, the temperature of the reaction is room temperature, and it is 20°C to 30°C.

Thin Layer Chromatography (TLC) was used for the monitoring of the reaction process in the embodiments. The expanding agent used in the reaction, the eluent system of column chromatography used for purifying the compound or the developing agent system of the thin-layer chromatography includes: A: petroleum ether and ethyl acetate system; B: dichloromethane and methanol system; C: n-hexane: ethyl acetate; wherein the volume ratio of the solvents is different depending on the different polarity of the compound, and a small amount of acidic or alkaline reagent such as acetic acid, triethylamine or the like can also be added for adjustment.

### Preparation of Intermediates

### Intermediate 1 5,5-dimethyl-3-(4,4,5,5-tetramethyl-1,3,2-dioxaborolan-2-yl)-5,6-dihydro-4H-pyrrolo[1,2-b]pyrazole IN -1

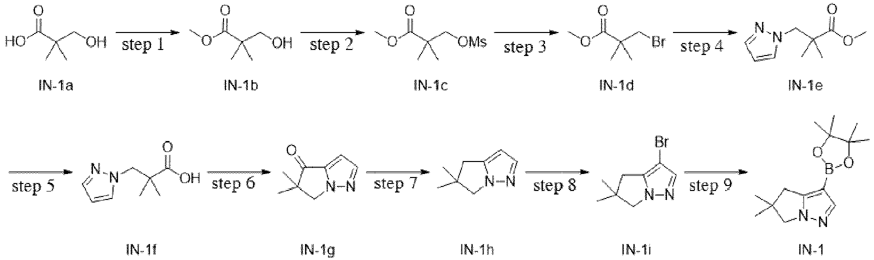

### Step 1: methyl 2,2-dimethyl-3-hydroxypropanoate IN-1b

2,2-dimethyl-3-hydroxypropionic acid **IN-1a** (100.0 g, 0.85 mol) was dissolved in methanol (1 L), concentrated sulfuric acid (91.1 g, 0.36 mol) was added dropwise at room temperature, the temperature was increased to 75°C for reaction for 4 hours, and the reaction was complete shown by TLC. The reaction solution was concentrated, a saturated sodium bicarbonate aqueous solution was added to adjust the pH to be weak basic, it was extracted with ethyl acetate, and the organic phases were combined, washed with saturated salt water, dried over anhydrous sodium sulfate, and concentrated to obtain the light yellow transparent liquid title compound **IN-1b** (93.0 g, crude product), which was directly used for the next step.

### Step 2: 2,2-dimethyl-3-((methylsulfonyl)oxy)propionic acid methyl ester IN-1c

Compound **IN-1b** (93.0 g, crude product) was dissolved in dichloromethane (500 mL), triethylamine (85.9 g, 0.85 mol) was added, the temperature was lowered to -5°C, under nitrogen protection, methylsulfonyl chloride (88.2 g, 0.77 mol) was added dropwise, after addition the temperature was lifted to room temperature for reaction for 2 hours, and the reaction was complete shown by TLC. The reaction solution was poured into water and extracted with ethyl acetate, the organic phases were combined, washed with saturated salt water, dried over anhydrous sodium sulfate and concentrated, and the crude product was separated and purified by silica gel column chromatography to obtain the light yellow transparent liquid title compound **IN-1c** (121.0 g, crude product), which was directly used for the next step.

### Step 3: 3-bromo-2,2-dimethylpropionic acid methyl ester IN-1d

Compound **IN-1c** (121.0 g, crude product) was dissolved in N,N-dimethylformamide (800 mL), lithium bromide (101.0 g, 1.16 mol) was added at room temperature, the temperature was lifted to 100°C for reaction overnight, and the reaction was complete shown by TLC. The reaction solution was cooled to room temperature, poured into water and extracted with ethyl acetate, the organic phases were combined, washed with saturated salt water, dried over anhydrous sodium sulfate and concentrated, and the crude product was separated and purified by silica gel column chromatography to obtain the light yellow transparent liquid title compound **IN-1d** (85.0 g, three-step yield 51%).

¹H NMR (400 MHz, CDCl₃) δ 3.69 (s, 3H), 3.48 (s, 2H), 1.29 (s, 6H)..

### Step 4: 2,2-dimethyl-3-(1H-pyrazol-1-yl)propionic acid methyl ester IN-1e

Compound **IN-1d** (85.0 g, 0.44 mol) was dissolved in *N,N*-dimethylformamide (500 mL), cesium carbonate (173.0 g, 0.53 mol) and imidazole (32.6 g, 0.48 mol) were added at room temperature, the temperature was lifted to 85°C for reaction overnight, and the reaction was complete shown by TLC. The reaction solution was cooled to room temperature, poured into water and extracted with ethyl acetate, the organic phases were combined, washed with saturated salt water, dried over anhydrous sodium sulfate and concentrated, and the crude product was separated and purified by silica gel column chromatography to obtain the light yellow transparent liquid title compound **IN-1e** (41.0 g, yield 52%).

LC-MS: m/z=183.2 [M+H]⁺

### Step 5: 2,2-dimethyl-3-(1H-pyrazol-1-yl)propionic acid IN-1f

Compound **IN-1e** (41.0 g, 0.22 mol) was dissolved in a mixed solvent of tetrahydrofuran (150 mL) and methanol (100 mL), a water (100 mL) solution of sodium hydroxide (18.0 g, 0.45 mol) was added, it was stirred at room temperature for 1 hour, and the reaction was complete shown by TLC. The reaction solution was concentrated to remove tetrahydrofuran and methanol, added with water and extracted with ethyl acetate, and the organic phases were combined, washed with saturated salt water, dried over anhydrous sodium sulfate and concentrated to obtain the white solid title compound **IN-1f** (33.0 g, crude product), which was directly used for the next step.

¹H NMR (400 MHz, DMSO-*d₆*) δ 12.44 (s, 1H), 7.61 (t, *J =* 1.6 Hz, 1H), 7.41 (d, *J =* 1.6 Hz, 1H), 6.21 (t, *J* = 2.0 Hz, 1H), 4.24 (s, 2H), 1.06 (s, 6H).

### Step 6: 5,5-dimethyl-5,6-dihydro-4H-pyrrolo[1,2-b]pyrazol-4-one IN-1g

Compound **IN-1f** (33.0 g, crude product) was dissolved in anhydrous tetrahydrofuran (300 mL), under nitrogen protection, the temperature was lowered to -65°C, n-butyllithium (160 mL, 0.4 mol, 2.5 M tetrahydrofuran solution) was slowly added dropwise, after dropwise addition the temperature was lifted to -45°C for reaction for 1 hour, then the temperature was lifted to room temperature, it was stirred overnight, and the reaction was complete shown by TLC. The reaction solution was slowly poured into water and extracted with ethyl acetate, the organic phases were combined, washed with saturated salt water, dried over anhydrous sodium sulfate and concentrated, and the crude product was separated and purified by silica gel column chromatography to obtain the light yellow liquid title compound **IN-1g** (18.1 g, two-step yield 55%).

### Step 7: 5,5-dimethyl-5,6-dihydro-4H-pyrrolo[1,2-b]pyrazole IN-1h

Compound **IN-1g** (18.1 g, 0.12 mol) was dissolved in diethylene glycol (300 mL), hydrazine hydrate (30 mL, 0.6 mol, 85%) was added at room temperature, the temperature was lifted to 180°C (internal temperature 156°C) for reaction for 2 hours, the temperature was lowered to 150°C, potassium hydroxide (23.6 g, 0.42 mol) was slowly added, after addition the temperature was lifted to 180°C for reaction for 5 hours, and the reaction was complete shown by TLC. The reaction solution was cooled to room temperature, added with water, adjusted to pH=6-7 with a hydrochloric acid (3M) solution and extracted with ethyl acetate, the organic phases were combined, washed with saturated salt water, dried over anhydrous sodium sulfate and concentrated, and the crude product was separated and purified by silica gel column chromatography to obtain the light yellow liquid title compound **IN-1h** (7.8 g, yield 48%).

### Step 8: 3-bromo-5,5-dimethyl-5,6-dihydro-4H-pyrrolo[1,2-b]pyrazole IN-1i

Compound **IN-1h** (7.1 g, 52.1 mmol) was dissolved in dichloromethane (60 mL), N-bromosuccinimide (9.3 g, 52.2 mmol) was added, the reaction was carried out overnight at room temperature, and the reaction was complete shown by TLC. The reaction solution was added with water and extracted with dichloromethane, the organic phases were combined, washed with saturated salt water, dried over anhydrous sodium sulfate and concentrated, and the crude product was separated and purified by silica gel column chromatography to obtain the yellow liquid title compound **IN-1i** (7.8 g, yield 48%).

### Step 9: 5,5-dimethyl-3-(4,4,5,5-tetramethyl-1,3,2-dioxaborolan-2-yl)-5,6-dihydro-4H-pyrrolo[1,2-b]pyrazole IN-1

Compound **IN-1i** (5.5 g, 25.6 mmol) was dissolved in 1,4-dioxane (50 mL), bis(pinacolato)diboron (9.7 g, 38.2 mmol), potassium acetate (5.0 g, 51.0 mmol) and Pd(dppf)Cl₂ dichloromethane complex (244 mg, 0.3 mmol) were added, under nitrogen protection, the temperature was lifted to 85°C for reaction overnight, and the reaction was complete shown by TLC. The reaction solution was cooled to room temperature, added with water and extracted with ethyl acetate, the organic phases were combined, washed with saturated salt water, dried over anhydrous sodium sulfate and concentrated, and the crude product was separated and purified by silica gel column chromatography to obtain the light yellow liquid title compound **IN-1** (2.4 g, yield 36%).

¹H NMR (400 MHz, CDCl₃) δ 7.77 (s, 1H), 3.88 (s, 2H), 2.79 (s, 2H), 1.26 (s, 12H), 1.24 (s, 6H).

### Intermediate 2 5-chloro-4-(5,5-dimethyl-5,6-dihydro-4H-pyrrolo[1,2-b]pyrazol-3-yl)pyridin-2-amine IN-2

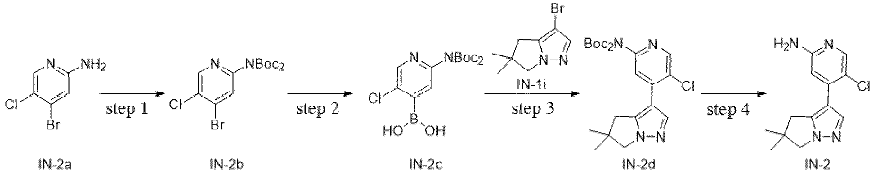

### Step 1: (4-bromo-5-chloropyridin-2-yl)iminodicarboxylic acid di-tert-butyl ester IN-2b

4-bromo-5-chloropyridin-2-amine **IN-2a** (3.0 g, 14.5 mmol) was dispersed in a mixed solvent (100 mL) of tert-butanol/acetone (1: 1), triethylamine (6.2 g, 61.3 mmol), di-tert-butyl dicarbonate (12.7 g, 58.2 mmol) and 4-dimethylaminopyridine (catalytic amount) were added, it was stirred at room temperature for 3 hours, and the complete reaction of the starting material was shown by TLC. The reaction solution was concentrated to remove the organic solvent, diluted with water and extracted with ethyl acetate, the organic phases were combined, washed with saturated salt water, dried with anhydrous sodium sulfate and concentrated, and the crude product was purified by silica gel column chromatography to obtain the white solid title compound **IN-2b** (7.5 g, crude product), which is directly used for the next step.

¹H NMR (400 MHz, DMSO-*d*₆) δ 8.65 (s, 1H), 8.03 (s, 1H), 1.41 (s, 18H).

### Step 2: (2-((di-tert-butoxycarbonyl)amino)-5-chloropyridin-4-yl)boronic acid IN-2c

Compound **IN-2b** (1.0 g, crude product) was dispersed in 1,4-dioxane (20 mL), and then bis(pinacolato)diboron (950 mg, 3.7 mmol), potassium acetate (730 mg, 7.4 mmol) and Pd(dppf)Cl₂ dichloromethane complex (catalytic amount) were added in sequence, under nitrogen protection, the temperature was lifted to 90°C, it was stirred for 1 hour, and the disappearance of the starting material was shown by TLC. The reaction solution was cooled to room temperature and filtered, the filter cake was washed with ethyl acetate, the filtrate was diluted with water and extracted with ethyl acetate, and the organic phases were combined, washed with water, washed with saturated salt water, dried over anhydrous sodium sulfate and concentrated to obtain the brown oil title compound **IN-2c** (2.5 g, crude product), which was used for the next step.

LC-MS: m/z=373.1[M+H]⁺

### Step 3: (5-chloro-4-(5,5-dimethyl-5,6-dihydro-4H-pyrrolo[1,2-b]pyrazol-3-yl)pyridin-2-yl)iminodicarbonic acid di-tert-butyl ester IN-2d

Compound **IN-1i** (215 mg, 1.0 mmol) and Compound **IN-2c** (746 mg, 2.0 mmol) were dispersed in 1,4-dioxane (15 mL) and water (5 mL), sodium carbonate (212 mg, 2.0 mmol) and Pd(dppf)Cl₂ (catalytic amount) were added in sequence at room temperature, after addition the temperature was lifted to 100°C, it was stirred for 1 hour under nitrogen protection, and the reaction was complete shown by TLC. The reaction solution was cooled to room temperature and filtered, the filter cake was washed multiple times with ethyl acetate, the filtrate was diluted with water and extracted with ethyl acetate, the organic phases were combined, washed with saturated salt water, dried over anhydrous sodium sulfate and concentrated, and the crude product was purified by silica gel column chromatography to obtain the white solid title compound **IN-2d** (398 mg, yield 86%).

### Step4: 5-chloro-4-(5,5-dimethyl-5,6-dihydro-4H-pyrrolo[1,2-b]pyrazol-3-yl)pyridin-2-amine IN-2

Compound **IN-2d** (398 mg, 0.86 mmol) was dissolved in dichloromethane (6 mL), trifluoroacetic acid (2 mL) was added, it was stirred at room temperature for 1 hour, and the complete reaction of the starting material was shown by TLC. The reaction solution was concentrated, added with saturated sodium bicarbonate aqueous solution and extracted with ethyl acetate, washed with saturated salt water, dried over anhydrous sodium sulfate and concentrated to obtain the yellow solid title compound **IN-2** (215 mg, crude product), which was directly used for the next step.

### Intermediate 3

### 7-(2-amino-5-chloropyridin-4-yl)-2,2-dimethyl-2,3-dihydro-1H-pyrrolizine-5-carbonitrile IN-3

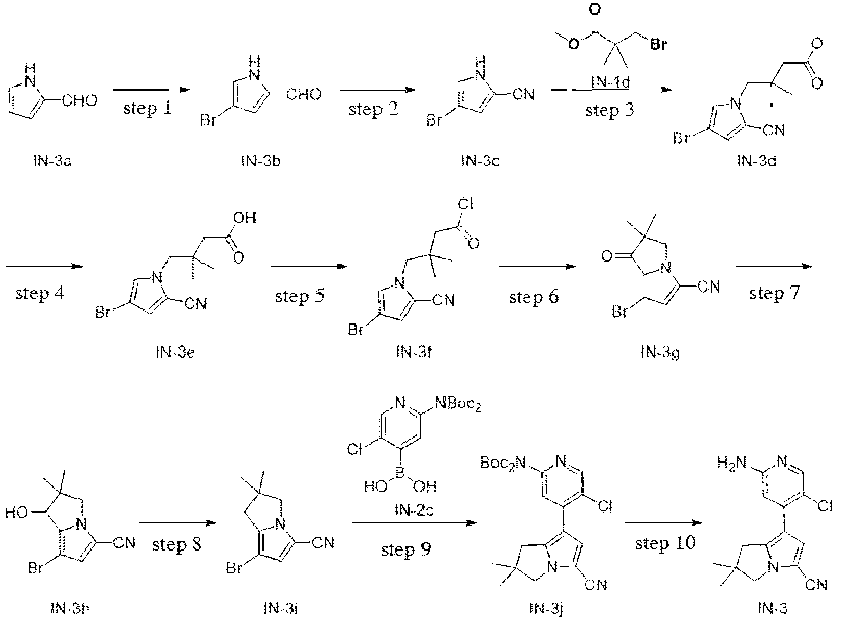

### Step 1: 4-bromo-1H-pyrrole-2-formaldehyde IN-3b

1*H*-pyrrole-2-formaldehyde **IN-3a** (10.0 g, 105.3 mmol) was dissolved in tetrahydrofuran (100 mL), under nitrogen protection, it was cooled to 0°C, *N*-bromosuccinimide (19.7 g, 110.5 mmol) was added, the reaction was maintained at 0°C for 30 minutes, and the disappearance of the starting material was shown by TLC. The reaction solution was concentrated to remove the organic solvent, diluted with water and extracted with ethyl acetate, the organic phases were combined, washed with saturated salt water, dried with anhydrous sodium sulfate and concentrated, and the crude product was purified by silica gel column to obtain the white solid title compound **IN-3b** (12.6 g, yield 69%).

LC-MS: m/z=174.0[M+H]⁺

### Step 2: 4-bromo-1H-pyrrole-2-carbonitrile IN-3c

Compound **IN-3b** (5.0 g, 28.7 mmol) was dissolved in water (150 mL), *O*-hydroxylamine sulfonic acid (11.4 g, 100.6 mmol) was added, it was stirred overnight at room temperature, and the disappearance of the starting material shown by TLC (the starting material and the product were distinguished by chromogenic agent for aldehyde or ketone). The reaction solution was cooled to 0°C, adjusted to pH=13-14 with a potassium hydroxide solution (4N) and extracted with dichloromethane, the organic phases were combined, washed with saturated salt water, dried over anhydrous sodium sulfate and concentrated, and the crude product was purified by silica gel column chromatography to obtain the white solid title compound **IN-3e** (4.2 g, yield 85.0%).

LC-MS: m/z=168.9[M-H]⁻

### Step 3: 4-(4-bromo-2-cyano-1H-pyrrol-1-yl)-3,3-dimethylbutyric acid IN-3d

Compound **IN-3c** (1.0 g, 5.8 mmol) was dispersed in *N,N-*dimethylformamide (15 mL), potassium carbonate (1.6 g, 11.6 mmol) and Compound **IN-1d** (1.7 g, 8.8 mmol) were added at room temperature, the temperature was lifted to 85°C, it was stirred overnight, and the complete reaction of the starting material was shown by TLC. The reaction solution was cooled to room temperature, diluted with water and extracted with ethyl acetate, the organic phases were combined, washed with water, washed with saturated salt water, dried over anhydrous sodium sulfate and concentrated, and the crude product was purified by silica gel column chromatography to obtain the yellow liquid title compound **IN-3d** (1.7 g, yield 97%).

### Step 4: 4-(4-bromo-2-cyano-1H-pyrrol-1-yl)-3,3-dimethylbutyric acid IN-3e

Compound **IN-3d** (1.7 g, 5.7 mmol) was dispersed in tetrahydrofuran (15 mL) and methanol (15 mL), it was cooled to 0°C, a sodium hydroxide aqueous solution (3 mL, 12 mmol, 4 M) was added, the temperature was slowly lifted to room temperature, it was stirred for 30 minutes, and the complete reaction of the starting material was shown by TLC. The reaction solution was concentrated to remove the organic solvent, diluted with water and extracted with ethyl acetate, the organic phase is discarded, the pH of the aqueous phase was adjusted to be acidic with dilute hydrochloric acid (1N), the aqueous phase was extracted with ethyl acetate, and the organic phases were combined, washed with saturated salt water, dried over anhydrous sodium sulfate and concentrated to obtain the yellow oil-like title compound **IN-3e** (1.4 g, crude product), which was directly used for the next step.

### Step 5: 4-(4-bromo-2-cyano-1H-pyrrol-1-yl)-3,3-dimethylbutyryl chloride IN-3f

Compound **IN-3e** (1.4 g, crude product) was dispersed in dichloromethane (30 mL), *N,N*-dimethylformamide (1 mL) was added at room temperature,it was cooled to 0°C, oxalyl chloride (980 mg, 7.7 mmol) was added dropwise, after addition the mixture was slowly lifted to room temperature, it was stirred for 30 minutes, and the complete reaction of the starting material was shown by TLC (methanol quenching point plate). The reaction solution was concentrated to obtain the yellow oil-like title compound **IN-3f** (1.5 g, crude product), which was directly used for the next step.

### Step 6: 7-bromo-2,2-dimethyl-1-oxo-2,3-dihydro-1H-pyrrolizine-5-carbonitrile IN-3g

Aluminum trichloride (1.4 g, 5.8 mmol) was dispersed in dichloromethane (15 mL), it was cooled to 0°C, a dichloromethane (15 mL) solution of Compound **IN-3f** (1.5 g, crude product) was added dropwise nitrogen protection, the temperature was naturally lifted to room temperature, it was stirred overnight, and the complete reaction of the starting material was shown by TLC. The reaction solution was poured into ice water for quenching, hydrochloric acid (1N) was added to adjust the pH to be acidic, it was extracted with dichloromethane, the organic phases were combined, washed with saturated salt water, dried over anhydrous sodium sulfate and concentrated, and the crude product was purified by silica gel column chromatography to obtain the white solid title compound **IN-3g** (900 mg, three-step yield 62%).

¹H NMR (400 MHz, CDCl₃) δ 7.00 (s, 1H), 4.17 (s, 2H), 1.38 (s, 6H).

### Step 7: 7-bromo-1-hydroxy-2,2-dimethyl-2,3-dihydro-1H-pyrrolizine-5-carbonitrile IN-3h

Compound **IN-3g** (870 mg, 3.4 mmol) was dispersed in methanol (15 mL), it was cooled to 0°C, sodium borohydride (330 mg, 8.7 mmol) was added, the temperature was lifted to room temperature, it was stirred for 1 hour, and the complete reaction of the starting material was shown by TLC. The reaction solution was quenched with water, concentrated to remove methanol and extracted with ethyl acetate, and the organic phases were combined, washed with saturated salt water, dried over anhydrous sodium sulfate and concentrated to obtain the title compound **IN-3h** (1.0 g, crude product), which was directly used for the next step.

### Step 8: 7-bromo-2,2-dimethyl-2,3-dihydro-1H-pyrrolizine-5-carbonitrile IN-3i

Compound **IN-3h** (1.0 g, crude product) was dispersed in trifluoroacetic acid (20 mL), it was cooled to 0°C and replaced with nitrogen, triethylsilane (1.4 g, 12 mmol) was added dropwise, after addition it was stirred at 0°C for 1 hour, and the complete reaction of the starting material was shown by TLC. The reaction solution was concentrated, the pH was adjusted to be alkaline with a saturated sodium bicarbonate aqueous solution, it was extracted ethyl acetate, the organic phases were combined, washed with saturated salt water, dried over anhydrous sodium sulfate and concentrated to obtain the yellow liquid title compound **IN-3i** (370 mg, crude product), which was directly used for the next step.

LC-MS: m/z=239.3[M+H] ⁺

### Step 9: (5-chloro-4-(5-cyano-2,2-dimethyl-2,3-dihydro-1H-pyrrolizin-7-yl)pyridin-2-yl)iminodicarbonic acid di-tert-butyl ester IN-3j

Compound **IN-2c** (250 mg, crude product) and Compound **IN-3i** (1.2 g, 1.6 mmol) were dispersed in 1,4-dioxane (5 mL) and water (2 mL), sodium carbonate (220 mg, 2.1 mmol) and Pd(dppf)Cl₂ (catalytic amount) were sequentially added, the temperature was lifted to 100°C, it was stirred for 1 hour under nitrogen protection, and the complete reaction of the starting material was shown by TLC. The reaction solution was cooled to room temperature and filtered, the filter cake was washed with ethyl acetate, the filtrate was diluted with water and extracted with ethyl acetate, the organic phases were combined, washed with saturated salt water, dried over anhydrous sodium sulfate and concentrated, and the crude product was purified by silica gel column chromatography to obtain the white solid title compound **IN-3j** (300 mg, three-step yield 26%).

LC-MS: m/z=387.2[M+H-Boc]⁺

### Step 10: 7-(2-amino-5-chloropyridin-4-yl)-2,2-dimethyl-2,3-dihydro-1H-pyrrolizine-5-carbonitrile IN-3

**IN-3j** (300 mg, 0.62 mmol) was dispersed in dichloromethane (6 mL), trifluoroacetic acid (3 mL) was added, it was stirred at room temperature for 1 hour, and the complete reaction of the starting material was shown by TLC. The reaction solution was concentrated, quenched with a saturated sodium bicarbonate aqueous solution and extracted with ethyl acetate, and the organic phases were combined, washed with saturated salt water, dried over anhydrous sodium sulfate and concentrated to obtain the yellow solid title compound **IN-3** (250 mg, crude product), which was directly used for the next step.

LC-MS: m/z=287.1[M+H]⁺

### Embodiment 1 3-(2-((5-chloro-4-(5,5-dimethyl-5,6-dihydro-4H-pyrrolo[1,2-b]pyrazol-3-yl)pyridin-2-yl)amino)-2-oxoethyl)be nzamide 1

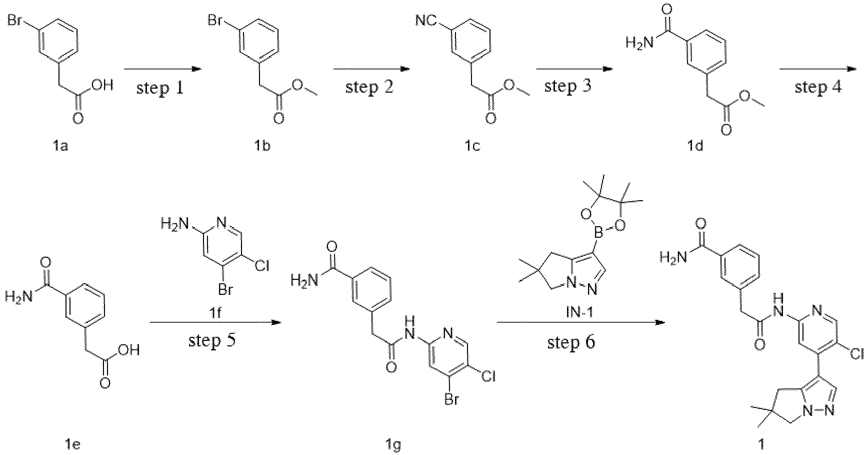

### Step 1: 2-(3-bromophenyl)acetic acid methyl ester 1b

3-bromophenylacetic acid **1a** (10.0 g, 46.5 mmol) was dissolved in methanol (80 mL), concentrated sulfuric acid (4.6 g, 46.5 mmol) was slowly added dropwise, the temperature was lifted to 60°C for reaction for 3 hours, and the reaction was complete shown by TLC. The reaction solution was cooled to room temperature, concentrated to remove most of methanol, added with water and extracted with ethyl acetate, the organic phases were combined, washed with saturated salt water, dried over anhydrous sodium sulfate and concentrated to obtain the light yellow transparent liquid title compound **1b** (10.8 g, crude product), which was directly used for the next step.

LC-MS: m/z=229.0/230.9[M+H]⁺

### Step 2: 2-(3-cyanophenyl)acetic acid methyl ester 1c

Compound **1b** (9.0 g, crude product) was dissolved in N-methylpyrrolidone (80 mL), zinc cyanide (9.2 g, 78.4 mmol) and tetrakis(triphenylphosphine)palladium (1.15 g, 1.0 mmol) were added, the temperature was lifted to 160°C for reaction for 8 hours under nitrogen protection, and the reaction was complete shown by TLC. The reaction solution was cooled to room temperature, added with water and extracted with ethyl acetate, and the organic phases were combined, washed with saturated salt water, dried over anhydrous sodium sulfate and concentrated to obtain the light yellow transparent liquid title compound **1c** (5.0 g, crude product), which was directly used for the next step.

LC-MS: m/z=176.0[M+H]⁺

¹H NMR (400 MHz, CDCl₃) δ 7.58-7.51 (m, 3H), 7.44 (t, *J =* 7.6 Hz, 1H), 3.71 (s, 3H), 3.66 (s, 2H).

### Step 3: 2-(3-carbamoylphenyl)acetic acid methyl ester 1d

Compound **1c** (5.0 g, crude product) was dissolved in dimethyl sulfoxide (50 mL), hydrogen peroxide (10 mL, 30%) and potassium carbonate (600 mg, 4.3 mmol) were added, the reaction was carried out overnight at room temperature, and the reaction was complete shown by TLC. The reaction solution was added with water and extracted with dichloromethane, and the organic phases were combined, washed with saturated salt water, dried over anhydrous sodium sulfate and concentrated to obtain the white-like solid title compound **1d** (5.5 g, crude product), which was directly used for the next step.

### Step 4: 3-formamidophenylacetic acid 1e

Compound **1d** (1.0 g, crude product) was dissolved in tetrahydrofuran (5 mL), sodium hydroxide (416 mg, 10.4 mmol) in water (3 mL) solution and methanol (1 mL) were added in sequence, the reaction was carried out at room temperature for 1 hour, and the reaction was complete shown by TLC. The reaction solution was added with water, adjust to pH=4-5 with hydrochloric acid (4N) and extracted with ethyl acetate, and the organic phases were combined, washed with saturated salt water, dried over anhydrous sodium sulfate and concentrated to obtain the white-like solid title compound **1e** (600 mg, crude product), which was directly used for the next step.

LCMS: m/z=180.1[M+H]⁺

### Step 5: 3-(2-((4-bromo-5-chloropyridin-2-yl)amino)-2-oxoethyl)benzamide 1g

Compound **1e** (600 mg, crude product) was dissolved in dichloromethane (10 mL), 1-(3-dimethylaminopropyl)-3-ethylcarbodiimide (980 mg, 5.1 mmol) was added, it was stirred at room temperature for 1 hour, 4-bromo-5-chloropyridin-2-amine **1f** (704 mg, 3.4 mmol) was added, the temperature was lifted to 30°C, it was stirred for 2 days, and a large amount of the starting material was remained shown by TLC. The reaction solution was added with water and extracted with dichloromethane, the organic phases were combined, washed with saturated salt water, dried over anhydrous sodium sulfate and concentrated, and the crude product was purified by silica gel column chromatography to obtain the light yellow solid title compound **1g** (105 mg, crude product), which was directly used for the next step.

### Step 6: 3-(2-((5-chloro-4-(5,5-dimethyl-5,6-dihydro-4H-pyrrolo[1,2-b]pyrazol-3-yl)pyridin-2-yl)amino)-2-oxoethyl)be nzamide 1

Compound **1g** (160 mg, crude product) was dissolved in a mixed solvent of 1,4-dioxane (6 mL) and water (2 mL), sodium carbonate (91 mg, 0.86 mmol), Intermediate **IN-1** (170 mg, 0.65 mmol) and Pd(dppf)Cl₂ dichloromethane complex (33 mg, 0.04 mmol) wer added in sequence at room temperature, under nitrogen protection, the temperature was lifted to 90°C for reaction for 3 hours, and the reaction was complete shown by TLC. The reaction solution was added with water and extracted with dichloromethane, the organic phases were combined, washed with saturated salt water and dried over anhydrous sodium sulfate, and the crude product was purified by Prep-TLC to obtain the white solid title compound **1** (29 mg, six-step yield 1%). LC-MS: m/z=423.9[M+H]⁺

¹H NMR (400 MHz, DMSO-d₆) δ10.92 (s, 1H), 8.38 (s, 1H), 8.22 (s, 1H), 8.00 (s, 1H), 7.96 (s, 1H), 7.85 (s, 1H), 7.76 (d, *J* = 7.6 Hz, 1H), 7.49 (d, *J* = 7.6 Hz, 1H), 7.40 (t, *J* = 7.6 Hz, 1H), 7.34 (s, 1H), 3.92 (s, 2H), 3.80 (s, 2H), 2.86 (s, 2H), 1.24 (s, 6H). (95.31% purity by HPLC)

### Embodiment 2

(S)-3-(1-((5-chloro-4-(5,5-dimethyl-5,6-dihydro-4*H*-pyrrolo[1,2-*b*]pyrazol-3-yl)pyridin-2-yl)amino)-1-oxopro pan-2-yl)benzamide (assumed) **2-1**
(R)-3-(1-((5-chloro-4-(5,5-dimethyl-5,6-dihydro-4*H*-pyrrolo[1,2-*b*]pyrazol-3-yl)pyridin-2-yl)amino)-1-oxopro pan-2-yl)benzamide (assumed) **2-2**

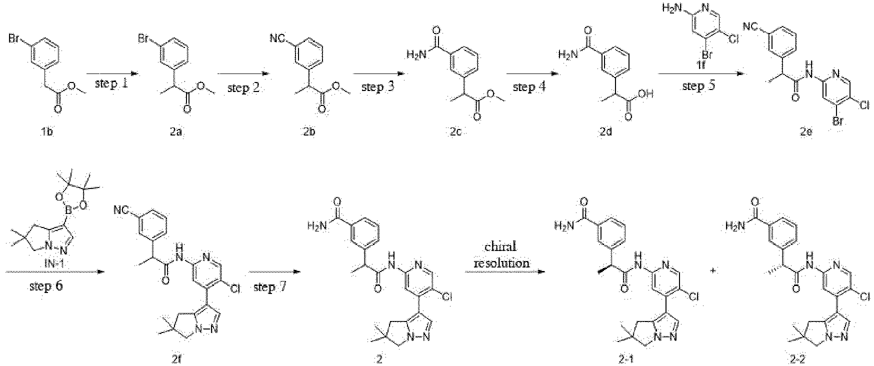

### Step 1: 2-(3-bromophenyl)propanoic acid methyl ester 2a

Compound **1b** (5.0 g, 21.83 mmol) was dissolved in anhydrous tetrahydrofuran (50 mL), under nitrogen protection, the temperature was lowered to -78°C, lithium diisopropylamide (13 mL, 26 mmol, 2M tetrahydrofuran solution) was added dropwise, after addition the reaction was carried out at -60°C for 1 hour, methyl iodide (3.1 g, 21.84 mmol) was added dropwise, after addition the temperature was lifted to room temperature for reaction for 1 hour, and the reaction was complete shown by TLC. The reaction solution was quenched with water and extracted with ethyl acetate, the organic phases were combined, washed with saturated salt water, dried over anhydrous sodium sulfate and concentrated, and the crude product was separated and purified by silica gel column chromatography to obtain the yellow liquid title compound **2a** (1.6 g, yield 30%).

### Step 2: 2-(3-cyanophenyl)propanoic acid methyl ester 2b

Compound **2a** (1.7 g, 6.99 mmol) was dissolved in N-methylpyrrolidone (10 mL), zinc cyanide (1.3 g, 11.07 mmol) and tetrakis(triphenylphosphine)palladium (140 mg, 0.12 mmol) were added at room temperature, the temperature was lifted to 150°C for reaction for 5 hours, and the reaction was complete shown by TLC. The reaction solution was cooled to room temperature, quenched with water and extracted with ethyl acetate, the organic phases were combined, washed with saturated salt water, dried with anhydrous sodium sulfate and concentrated, and the crude product was separated and purified by silica gel column chromatography to obtain the yellow liquid title compound **2b** (1.0 g, yield 77%).

### Step 3: 2-(3-formamidophenyl)propanoic acid methyl ester 2e

Compound **2b** (1.0 g, 5.28 mmol) was dissolved in dimethyl sulfoxide (10 mL), potassium carbonate (110 mg, 0.80 mmol) and hydrogen peroxide (2.0 mL, 30%) were added, the reaction was carried out for 1 hour at room temperature, and the reaction was complete shown by TLC. The reaction solution was quenched with water and extracted with ethyl acetate, the organic phases were combined, washed with saturated salt water, dried over anhydrous sodium sulfate and concentrated, and the crude product was separated and purified by silica gel column chromatography to obtain the yellow liquid title compound **2c** (1.0 g, yield 91%).

LC-MS: m/z=208.2 [M+H]⁺

### Step 4: 2-(3-formamidophenyl)propanoic acid methyl ester 2d

Compound **2c** (1.0 g, 4.82 mmol) was dissolved in tetrahydrofuran/methanol/water (12 mL/2 mL/6 mL), sodium hydroxide (0.5 g, 12.50 mmol) was added, it was stirred at room temperature for 1 hour, and the reaction was complete shown by TLC. The reaction solution was quenched with water and extracted with ethyl acetate, the organic phase was discarded, the aqueous phase was adjusted to pH=3 with dilute hydrochloric acid (1N) and extracted with ethyl acetate, and the organic phases were combined, washed with saturated salt water, dried over anhydrous sodium sulfate and concentrated to obtain the white solid title compound **2d** (910 mg, crude product), which was directly uesd for the next step.

### Step 5: N-(4-bromo-5-chloropyridin-2-yl)-2-(3-cyanophenyl)propionamide 2e

Compound **2d** (241 mg, crude product) and Compound **1f** (373 mg, 1.80 mmol) were dissolved in ethyl acetate (5 mL), 1-propylphosphonic anhydride (1.8 g, 2.83 mmol, 50% ethyl acetate solution) and pyridine (455 mg, 5.75 mmol) were added at room temperature, the temperature was lifted to 85°C for reaction for 3 hours, and the reaction was complete shown by TLC. The reaction solution was cooled to room temperature, quenched with water and extracted with ethyl acetate, the organic phases were combined, washed with saturated salt water, dried over anhydrous sodium sulfate and concentrated, and the crude product was separated and purified by silica gel column chromatography to obtain the white solid title compound **2e** (200 mg, two-step yield 43%).

LC-MS: m/z=364.0 [M+H]⁺

### Step 6: N-(5-chloro-4-(5,5-dimethyl-5,6-dihydro-4H-pyrrolo[1,2-b]pyrazol-3-yl)pyridin-2-yl)-2-(3-cyanophenyl)propi onamide 2f

Compound **2e** (250 mg, 0.68 mmol) and Intermediate **IN-1** (233 mg, 0.89 mmol) were dissolved in 1,4-dioxane (5 mL) and water (2.5 mL), potassium carbonate (276 mg, 2.00 mmol) and Pd(dppf)Cl₂ dichloromethane complex (57 mg, 0.07 mmol) were added at room temperature, the temperature was lifted to 100°C for reaction overnight under nitrogen protection, and the reaction was complete shown by TLC. The reaction solution was cooled to room temperature, quenched with water and extracted with ethyl acetate, the organic phases were combined, washed with saturated salt water, dried over anhydrous sodium sulfate and concentrated, and the crude product was purified by silica gel column chromatography to obtain the white solid title compound **2f** (90 mg, yield 31%).

### Step 7: (S)-3-(1-((5-chloro-4-(5,5-dimethyl-5,6-dihydro-4H-pyrrolo[1,2-b]pyrazol-3-yl)pyridin-2-yl)amino)-1-oxopro pan-2-yl)benzamide (assumed) 2-1 &

### (R)-3-(1-((5-chloro-4-(5,5-dimethyl-5,6-dihydro-4H-pyrrolo[1,2-b]pyrazol-3-yl)pyridin-2-yl)amino)-1-oxopro pan-2-yl)benzamide (assumed) 2-2

Compound **2f** (90 mg, 0.21 mmol) was dissolved in dimethyl sulfoxide (5 mL), potassium carbonate (50 mg, 0.36 mmol) and hydrogen peroxide (1 mL, 30%) were added, the reaction was carried out for 1 hour at room temperature, and the reaction was complete shown by TLC. The reaction solution was quenched with water and extracted with ethyl acetate, the organic phases were combined, washed with saturated salt water, dried over anhydrous sodium sulfate and concentrated, and the crude product was separated and purified by silica gel column chromatography to obtain the white solid compound **2** (81 mg, yield 88%), and chirally resolved (DAICEL AD-H, 30*250mm, 5um, 30mL/min, EtOH:Hexane=40:60) to obtain the title compound **2-1** (RT 34.5 min) (13.7 mg, yield: 17%) and the title compound **2-2** (RT 39.7 min) (16.2 mg, yield 20%).

### Compound 2-1

LC-MS: m/z=438.2[M+H]^{+ 1}H NMR(400 MHz, DMSO-d₆) δ 10.85 (s, 1H), 8.35 (s, 1H), 8.23 (s, 1H), 7.98 (s, 2H), 7.94 (s, 1H), 7.76 (d, *J* = 7.6 Hz, 1H), 7.56 (d, *J* = 8.0 Hz, 1H), 7.41 (t, *J* = 7.6 Hz, 1H), 7.36 (s, 1H), 4.10 (q, *J* = 7.2 Hz, 1H), 3.94 (s, 2H), 2.88 (s, 2H), 1.46 (d, *J* = 6.8 Hz, 3H), 1.27 (s, 6H). (98.78% purity by HPLC)

### Compound 2-2

LC-MS: m/z=438.2[M+H]⁺

¹H NMR(400 MHz, DMSO-*d₆*) δ 10.85 (s, 1H), 8.35 (s, 1H), 8.23 (s, 1H), 7.98 (s, 2H), 7.94 (s, 1H), 7.76 (d, *J* = 7.6 Hz, 1H), 7.56 (d, *J* = 8.0 Hz, 1H), 7.41 (t, *J =* 7.6 Hz, 1H), 7.36 (s, 1H), 4.10 (q, *J =* 7.2 Hz, 1H), 3.94 (s, 2H), 2.88 (s, 2H), 1.46 (d, *J* = 6.8 Hz, 3H), 1.27 (s, 6H). (98.60% purity by HPLC)

### Embodiment 3

### 3-(2-((5-chloro-4-(5,5-dimethyl-5,6-dihydro-4H-pyrrolo[1,2-b]pyrazol-3-yl)pyridin-2-yl)amino)-2-oxoethyl)-N-methylbenzamide 3

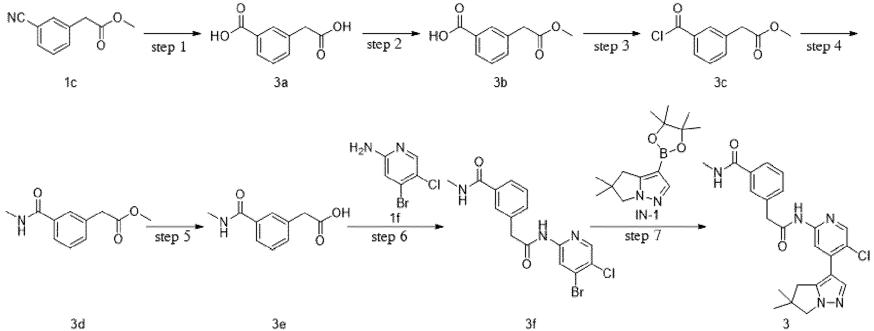

### Step 1: 3-(carboxymethyl)benzoic acid 3a

Compound **1c** (2.0 g, 11.42 mmol) and sodium hydroxide (1.83 g, 45.76 mmol) were dissolved in water (20 mL) and ethanol (5 mL), it was lifted to 75°C and reacted overnight, and the reaction was complete shown by TLC. The reaction solution was cooled to room temperature, quenched with water, extracted with ethyl acetate, the organic phase was discarded, the aqueous phase was adjusted to pH=3 with dilute hydrochloric acid (1N) and extracted with ethyl acetate, the organic phases were combined, washed with saturated salt water, dried over anhydrous sodium sulfate and concentrated to obtain the white solid title compound **3a** (1.8 g, crude product), which was directly used for the next step.

### Step 2: 3-(2-methoxy-2-oxoethyl)benzoic acid 3b

Thionyl chloride (36 mg, 0.30 mmol) was added to a solution of Compound **3a** (1.8 g, 10.0 mmol) in methanol (5 mL), it was stirred at room temperature overnight, and the disappearance of the starting material was shown by TLC. The reaction solution was quenched with water and extracted with ethyl acetate, and the organic phases were combined, washed with saturated salt water, dried over anhydrous sodium sulfate and concentrated to obtain the white solid title compound **3b** (1.6 g, crude product), which was directly used for the next step.

¹H NMR (400 MHz, CDCl₃) δ 13.00 (s, 1H), 7.88-7.84 (m, 2H), 7.54-7.44 (m, 2H), 3.79 (s, 2H), 3.63 (s, 3H).

### Step 3: 2-(3-(chlorocarbonyl)phenyl)acetic acid methyl ester 3c

Thionyl chloride (5 mL, 0.07 mmol) was added to a toluene (15 mL) solution of Compound **3b** (600 mg, crude product), the temperature was lifted to 85°C, it was stirred for 3 hours, and the disappearance of the starting material was shown by TLC. The reaction solution was concentrated to obtain the title compound **3c** (656 mg, crude product), which was directly used for the next step.

### Step 4: 2-(3-(methylcarbamoyl)phenyl)acetic acid methyl ester 3d

Compound **3c** (328 mg, crude product) and methylamine hydrochloride (125 mg, 1.85 mmol) were dissolved in dichloromethane (3 mL), triethylamine (0.5 mL, 3.60 mmol) was added, it was stirred at room temperature for 30 minutes, and the reaction was complete shown by TLC. The reaction solution was quenched with water and extracted with ethyl acetate, the organic phases were combined, washed with saturated salt water, dried over anhydrous sodium sulfate and concentrated to obtain the colorless oily liquid title compound **3d** (230 mg, crude product), which was directly used for the next step.

LC-MS: m/z=208.0 [M+H]⁺

### Step 5: 2-(3-(methylcarbamoyl)phenyl)acetic acid 3e

Compound **3d** (230 mg, crude product) was dissolved in a mixed solvent of tetrahydrofuran (3 mL) and water (3 mL), sodium hydroxide (67 mg, 1.67 mmol) was added, it was stirred overnight at room temperature, and the reaction was complete shown by TLC. The reaction solution was quenched with water, adjusted to pH-3 by dilute hydrochloric acid (1N) and extracted with ethyl acetate, and the organic phases were combined, washed with saturated salt water, dried over anhydrous sodium sulfate and concentrated to obtain the white solid title compound **3e** (180 mg, crude product), which was directly used for the next step.

### Step 6: 3-(2-((4-bromo-5-chloropyridin-2-yl)amino)-2-oxoethyl)-N-methylbenzamide 3f

Compound **3e** (180 mg, crude product) and compound **1f** (200 mg, 0.96 mmol) were dissolved in ethyl acetate (20 mL), pyridine (379 mg, 4.79 mmol) was added at room temperature, 1-propylphosphonic anhydride (2.05 g, 3.22 mmol, 50% ethyl acetate solution) was added dropwise, after addition the reaction was carried out overnight at room temperature, and the reaction was complete shown by TLC. The reaction solution was quenched with water and extracted with ethyl acetate, the organic phases were combined, washed with water, washed with saturated salt water, dried with anhydrous sodium sulfate and concentrated, and the crude product was separated and purified by silica gel column chromatography to obtain the white solid title compound **3f** (210 mg, six-step yield 28.8%).

¹H NMR (400 MHz, DMSO-*d₆*) δ 11.18 (s, 1H), 8.52 (s, 1H), 8.48 (s, 1H), 8.44-8.43 (m, 1H), 7.81 (s, 1H), 7.71 (d, *J* = 7.6 Hz, 1H), 7.48-7.39 (m, 2H), 3.81 (s, 2H), 2.78 (d, *J =* 4.4 Hz, 3H).

### Step 7:

### 3-(2-((5-chloro-4-(5,5-dimethyl-5,6-dihydro-4H-pyrrolo[1,2-b]pyrazol-3-yl)pyridin-2-yl)amino)-2-oxoethyl)-N-methylbenzamide 3

Compound **3f** (100 mg, 0.26 mmol) and Intermediate **IN-1** (100 mg, 0.38 mg) were dissolved in 1,4-dioxane/water (2mL/0.5mL), sodium carbonate (40 mg, 0.38 mmol) and Pd(dppf)Cl₂ (18 mg, 0.025 mmol) were added at room temperature, the temperature was lifted to 80°C for reaction for two hours under nitrogen protection, and the reaction was complete shown by TLC. The reaction solution was cooled to room temperature, quenched with water and extracted with ethyl acetate, the organic phases were combined, washed with water, washed with saturated salt water, dried over anhydrous sodium sulfate and concentrated, and the crude product was separated and purified byPrep-TLC to obtain the white solid title compound **3** (32 mg, yield 29%).

LC-MS: m/z=438.2 [M+H]^{+ 1}H NMR(400 MHz, DMSO-d₆) δ 10.95 (s, 1H), 8.45-8.43 (m, 1H), 8.38 (s, 1H), 8.22 (s, 1H), 8.00 (s, 1H), 7.82 (s, 1H), 7.71 (d, *J* = 7.6 Hz, 1H), 7.48 (d, *J* = 7.6 Hz, 1H), 7.41 (t, *J* = 8.0 Hz, 1H), 3.92 (s, 2H), 3.80 (s, 2H), 2.86 (s, 2H), 2.78 (d, *J* = 4.8 Hz, 3H), 1.24 (s, 6H). (95.32% purity by HPLC)

### Embodiment 4

### N-(5-chloro-4-(5,5-dimethyl-5,6-dihydro-4H-pyrrolo[1,2-b]pyrazol-3-yl)pyridin-2-yl)-2-(3-cyanophenyl)aceta mide 4

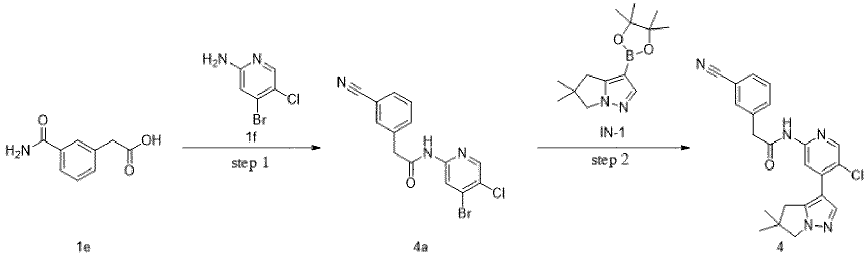

### Step 1: N-(4-bromo-5-chloropyridin-2-yl)-2-(3-cyanophenyl)acetamide 4a

Compound **1e** (347 mg, 1.94 mmol) was dissolved in ethyl acetate (10 mL), Compound **1f** (331 mg, 1.60 mmol), 1-propylphosphonic anhydride (4.10 g, 6.44 mmol, 50% ethyl acetate solution) and pyridine (764 mg, 9.66 mmol) were added at room temperature, the temperature was lifted to 85°C for reaction for 2 hours, and the reaction was complete shown by TLC. The reaction solution was cooled to room temperature, quenched with water and extracted with ethyl acetate, the organic phases were combined, washed with saturated salt water, dried with anhydrous sodium sulfate and concentrated, and the crude product was separated and purified by silica gel column chromatography to obtain the white solid title compound **4a** (537 mg, yield 68%).

### Step 2: N-(5-chloro-4-(5,5-dimethyl-5,6-dihydro-4H-pyrrolo[1,2-b]pyrazol-3-yl)pyridin-2-yl)-2-(3-cyanophenyl)aceta mide 4

Compound **4a** (100 mg, 0.28 mmol) was dissolved in 1,4-dioxane (6 mL) and water (2 mL), Intermediate **IN-1** (112 mg, 0.43 mmol), potassium carbonate (79 mg, 0.57 mmol) and Pd(dppf)Cl₂ dichloromethane complex (catalytic amount) were added at room temperature, the temperature was lifted to 95°C for reaction overnight under nitrogen protection, and the reaction was complete shown by TLC. The reaction solution was cooled to room temperature, quenched with water and extracted with ethyl acetate, the organic phases were combined, washed with saturated salt, dried over anhydrous sodium sulfate and concentrated, and the crude product was separated and purified by Prep-TLC to obtain the white solid title compound **4** (38 mg, yield 33%).

LC-MS: m/z=406.1 [M+H] ⁺

¹H NMR(400 MHz, DMSO-d₆) δ10.95 (s, 1H), 8.38 (s, 1H), 8.19 (s, 1H), 7.99 (s, 1H), 7.79 (s, 1H), 7.74 (d, *J* = 7.6 Hz, 1H), 7.68 (d, *J* = 8.0 Hz, 1H), 7.55 (t, *J* = 8.0 Hz, 1H), 3.92 (s, 2H), 3.85 (s, 2H), 2.95 (s, 2H), 1.24 (s, 6H). (97.23% purity by HPLC)

### Embodiment 5

### N-(5-chloro-4-(5,5-dimethyl-5,6-dihydro-4H-pyrrolo[1,2-b]pyrazol-3-yl)pyridin-2-yl)-2-(pyridin-4-yl)acetami de 5

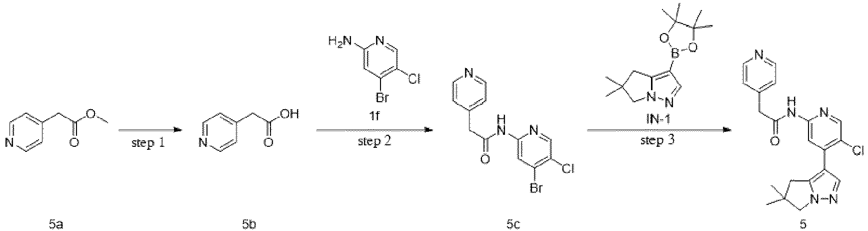

### Step 1: 2-(pyridin-4-yl)acetic acid 5b

Methyl 4-pyridylacetate **5a** (2.0 g, 13.23 mmol) was dissolved in tetrahydrofuran/water (10 mL/5 mL), sodium hydroxide (1.32 g, 33.01 mmol) was added, and it was stirred at room temperature for 2 hours. The reaction was complete shown by TLC. The reaction solution was quenched with water, the aqueous phase was extracted with ethyl acetate, the organic phase was discarded, the aqueous phase was adjusted to pH= 3 with dilute hydrochloric acid (1N) and extracted with ethyl acetate, the organic phases were combined, washed with saturated salt water, dried over anhydrous sodium sulfate and concentrated to obtain the white solid title compound **5b** (1.40 g, crude product), which was directly used for the next step.

### Step 2: N-(4-bromo-5-chloropyridin-2-yl)-2-(pyridin-4-yl)acetamide 5c

Compound **5b** (300 mg, crude product) was dissolved in ethyl acetate (20 mL), Compound **1f** (379 mg, 1.83 mmol), pyridine (868 mg, 10.97 mmol) and 1-propylphosphonic anhydride (4.66 g, 7.32 mmol, 50% ethyl acetate solution) were added in sequence, and it was stirred at room temperature overnight. The reaction was complete shown by TLC. The reaction solution was quenched with water and extracted with ethyl acetate, the organic phases were combined, washed with saturated salt water, dried over anhydrous sodium sulfate and concentrated, the crude product was separated and purified by silica gel column chromatography to obtain the white solid title compound **5c** (633 mg, yield 89%),

LC-MS: m/z=326.0[M+H]⁺

### Step 3:

### N-(5-chloro-4-(5,5-dimethyl-5,6-dihydro-4H-pyrrolo[1,2-b]pyrazol-3-yl)pyridin-2-yl)-2-(pyridin-4-yl)acetami de 5

Compound **5c** (100 mg, 0.31 mmol) was dissolved in 1,4-dioxane/water (6 mL/2 mL), Intermediate **IN-1** (158 mg, 0.60 mmol), potassium carbonate (84 mg, 0.61 mmol) and Pd(dppf)Cl₂ dichloromethane complex (catalytic amount) were added at room temperature, and temperature was lifted to 95°C for reaction overnight under nitrogen protection. The reaction was complete shown by TLC. The reaction solution was cooled to room temperature and quenched with water, the organic phases were combined, washed with saturated salt water, dried over anhydrous sodium sulfate and concentrated, and the crude product was separated and purified by silica gel column chromatography to obtain the white solid title compound **5** (33 mg, yield 28.2%). LC-MS: m/z=382.2 [M+H]⁺

¹H NMR (400 MHz, CDCl₃) δ 8.82 (s, 1H), 8.65 (s, 2H), 8.26 (s, 1H), 8.20 (s, 1H), 8.11 (s, 1H), 7.46 (s, 2H), 3.95 (s, 2H), 3.85 (s, 2H), 2.95 (s, 2H), 1.34 (s, 6H). (97.54% purity by HPLC)

### Embodiment 6

### N-(5-chloro-4-(5,5-dimethyl-5,6-dihydro-4H-pyrrolo[1,2-b]pyrazol-3-yl)pyridin-2-yl)-2-(pyridin-3-yl)acetami de 6

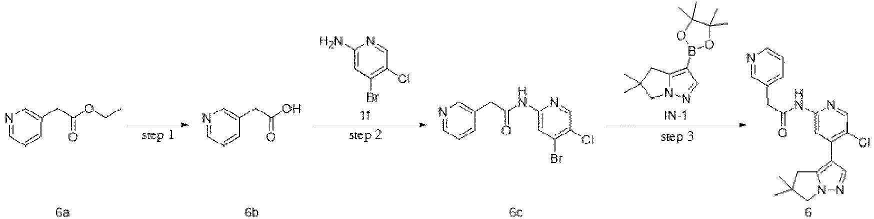

### Step 1: 3-pyridylacetic acid 6b

Ethyl 3-pyridylacetate **6a** (2.0 g, 12.11 mmol) was dissolved in tetrahydrofuran and water (10 mL/5 mL), sodium hydroxide (1.32 g, 33.01 mmol) was added, it was stirred at room temperature for 2 hours, and the reaction was complete shown by TLC. The reaction solution was neutralized with dilute hydrochloric acid (10.2 mL, 31.60 mmol, 3M) and concentrated to obtain a solid, a methanol/dichloromethane solution (10%) was added, the insoluble salt was removed by filtration, and the filtrate was concentrated to obtain the title compound **6b** (1.18 g, crude product), which was directly used for the next step.

### Step 2: N-(4-bromo-5-chloropyridin-2-yl)-2-(pyridin-3-yl)acetamide 6c

Compound **6b** (300 mg, crude product) was dissolved in ethyl acetate (20 mL), Compound **1f** (379 mg, 1.83 mmol), pyridine (868 mg, 10.97 mmol) and 1-propylphosphonic anhydride (4.66 g, 7.32 mmol, 50% ethyl acetate solution) were added in sequence at room temperature, it was stirred overnight at room temperature, and the reaction was complete shown by TLC. The reaction solution was quenched with water and extracted with ethyl acetate, the organic phases were combined, washed with saturated salt water, dried over anhydrous sodium sulfate and concentrated to obtain the title compound **6c** (590 mg, crude product), which was directly used for the next step.

### Step 3:

### N-(5-chloro-4-(5,5-dimethyl-5,6-dihydro-4H-pyrrolo[1,2-b]pyrazol-3-yl)pyridin-2-yl)-2-(pyridin-3-yl)acetami de 6

Compound **6c** (100 mg, crude product) was dissolved in 1,4-dioxane/water (6 mL/2 mL), under nitrogen protection, Intermediate **IN-1** (158 mg, 0.60 mmol), potassium carbonate (84 mg, 0.61 mmol) and Pd(dppf)Cl₂ dichloromethane complex (catalytic amount) were added in sequence at room temperature, the temperature was lifted to 95°C for reaction overnight, and the reaction was complete shown by TLC. The reaction solution was cooled to room temperature, quenched with water and extracted with ethyl acetate, the organic phases were combined, washed with saturated salt water, dried over anhydrous sodium sulfate and concentrated, and the crude product was chromatographed on silica gel column to obtain the white solid title compound 6 (33 mg, three-step yield 16%).

LC-MS: m/z=382.1 [M+H]⁺

¹H NMR(400 MHz, CDCl₃) δ 8.78 (s, 1H), 8.61 (d, *J =* 4.4 Hz, 1H), 8.42 (s, 1H), 8.23 (s, 1H), 8.21 (s, 1H), 8.10 (s, 1H), 7.89 (d, *J =* 8.0 Hz, 1H), 7.45 (dd, *J =* 7.6 Hz, 4.8 Hz, 1H), 3.95 (s, 2H), 3.87 (s, 2H), 2.94 (s, 2H), 1.33 (s, 6H). (97.14% purity by HPLC)

### Embodiment 7

### N-(5-chloro-4-(5,5-dimethyl-5,6-dihydro-4H-pyrrolo[1,2-b]pyrazol-3-yl)pyridin-2-yl)-2-(6-cyanopyridin-2-yl) acetamide 7

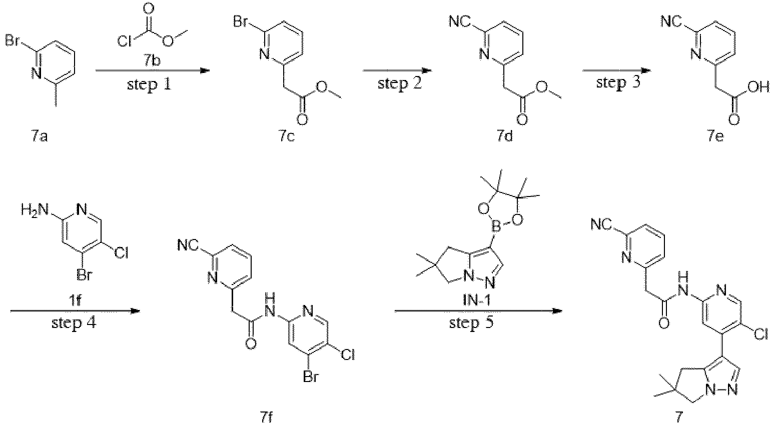

### Step 1: 2-(6-bromopyridin-2-yl)acetic acid methyl ester 7c

2-bromo-6-methylpyridine **7a** (5.0 g, 29.1 mmol) was dissolved in anhydrous tetrahydrofuran (30 mL), under nitrogen protection, the temperature was lowered to -50°C, lithium diisopropylamide (39 mL, 78.0 mmol, 2M tetrahydrofuran solution) was added dropwise, and methyl chloroformate **7b** (3.2 g, 33.9 mmol) was added dropwise, after the dropwise addition was completed, it was stirred at -50°C for 15 minutes, and the reaction was complete shown by TLC. The reaction solution was poured into water and extracted with ethyl acetate, the organic phases were combined, washed with saturated salt water, dried over anhydrous sodium sulfate and concentrated, and the crude product was separated and purified by silica gel column chromatography to obtain the light yellow solid title compound **7c** (1.2 g, yield 15%).

LC-MS: m/z=230.0 [M+H]⁺

¹H NMR (400 MHz, DMSO-*d₆*) δ 7.52 (t, *J =* 8.0 Hz, 1H), 7.40 (d, *J =* 8.0 Hz, 1H), 7.28 (d, *J =* 8.0 Hz, 1H), 3.84 (s, 2H), 3.82 (s, 3H).

### Step 2: 2-(6-cyanopyridin-2-yl)acetic acid methyl ester 7d

Compound **7c** (1.1 g, 4.8 mmol) was dissolved in *N,N*-dimethylformamide (10 mL) and water (1 mL), zinc cyanide (845 mg, 7.2 mmol), Pd(dppf)Cl₂ (200 mg, 0.3 mmol) and Pd₂(dba)₃ (270 mg, 0.3 mmol) (270 mg, 0.3 mmol) were added in sequence, the temperature was lifted to 90°C for reaction for 3 hours, and the reaction was complete shown by TLC. The reaction solution was cooled to room temperature, poured into water and extracted with ethyl acetate, the organic phases were combined, washed with saturated salt water, dried over anhydrous sodium sulfate and concentrated, and the crude product was separated and purified by silica gel column chromatography to obtain the light yellow liquid title compound **7d** (710 mg, yield 84%).

LC-MS: m/z=177.1 [M+H]⁺

### Step 3: 2-(6-cyanopyridin-2-yl)acetic acid 7e

Compound **7d** (600 mg, 3.4 mmol) was dissolved in tetrahydrofuran (6 mL), a water (2 mL) solution of sodium hydroxide (136 mg, 3.4 mmol) and methanol (2 mL) were added, the reaction was carried out for reaction for 1 hour at room temperature, and the reaction was complete shown by TLC. The reaction solution was poured into water, adjusted to pH 5-6 with dilute hydrochloric acid (2N) and extracted with ethyl acetate, and the organic phases were combined, washed with saturated salt water, dried over anhydrous sodium sulfate and concentrated to obtain the light yellow solid title compound **7e** (370 mg, crude product), which was directly used for the next step.

### Step 4: N-(4-bromo-5-chloropyridin-2-yl)-2-(6-cyanopyridin-2-yl)acetamide 7f

Compound **7e** (300 mg, crude product) was dissolved in ethyl acetate (5 mL), pyridine (506 mg, 6.4 mmol), 1-propylphosphonic anhydride (2.8 g, 4.3 mmol, 50% ethyl acetate solution) and 4-bromo-5-chloro-2-aminopyridine (294 mg, 1.4 mmol) were added in sequence at room temperature, the temperature was lifted to 85°C, it was stirred for 1 hour, and the reaction was complete shown by TLC. The reaction solution was cooled to room temperature, poured into water, adjusted to pH 8-9 with a saturated sodium bicarbonate solution and extracted with ethyl acetate, and the organic phases were combined, washed with saturated salt water, dried over anhydrous sodium sulfate and concentrated to obtain the light yellow solid title compound **7f** (600 mg, crude product), which was directly used for the next step.

### Step 5: N-(5-chloro-4-(5,5-dimethyl-5,6-dihydro-4H-pyrrolo[1,2-b]pyrazol-3-yl)pyridin-2-yl)-2-(6-cyanopyridin-2-yl) acetamide 7

Compound **7f** (155 mg, crude product) was dissolved in a mixed solvent of 1,4-dioxane (5 mL) and water (2.5 mL), Intermediate **IN-1** (149 mg, 0.57 mmol), potassium carbonate (180 mg, 1.3 mmol) and Pd(dppf)Cl₂ dichloromethane complex (33 mg, 0.03 mmol) were added, the temperature was lifted to 100°C for reaction overnight under nitrogen protection, and the reaction was complete shown by TLC. The reaction solution was cooled to room temperature, poured into water and extracted with ethyl acetate, and the organic phases were combined, washed with saturated salt water, dried over anhydrous sodium sulfate and concentrated to obtain the white solid title compound **7** (70 mg, yield 39%).

LC-MS: m/z=407.1 [M+H]⁺

¹H NMR (400 MHz, DMSO-d₆) δ 10.98 (s, 1H), 8.39 (s, 1H), 8.19 (s, 1H), 8.02-8.06 (m, 1H), 8.00 (s, 1H), 7.95 (d, *J =* 8.0 Hz, 1H), 7.76 (d, *J* = 8.0 Hz, 1H), 4.07 (s, 2H), 3.92 (s, 2H), 2.85 (s, 2H), 1.24 (s, 6H).

(99.04% purity by HPLC)

### Embodiment 8

### 2-(3-acetaminophenyl)-N-(5-chloro-4-(5,5-dimethyl-5,6-dihydro-4H-pyrrolo[1,2-b]pyrazol-3-yl)pyridin-2-yl)a cetamide 8

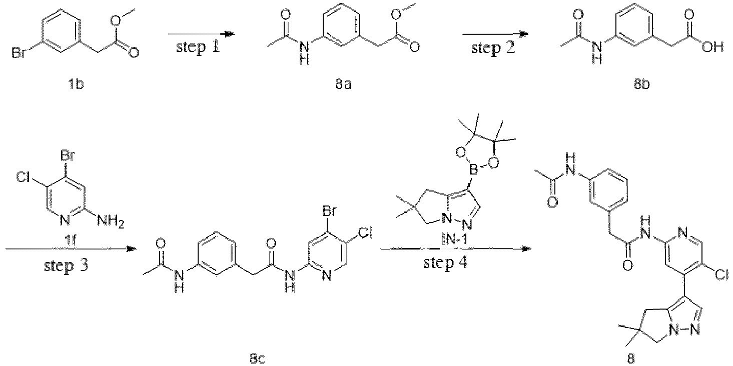

### Step 1: 2-(3-acetaminophenyl)acetic acid methyl ester 8a

Compound **1b** (1.5 g, crude product) was dissolved in 1,4-dioxane (20 mL), cesium carbonate (8.5 g, 26.1 mmol), acetamide (0.8 g, 13.1 mmol), Pd₂(dba)₃ (270 mg, 0.3 mmol) and Xphos (170 mg, 0.3 mmol) were added in sequence at room temperature, the temperature was lifted to 100°C for reaction for 2 hours under nitrogen protection, and the reaction was complete shown by TLC. The reaction solution was cooled to room temperature, added with water and extracted with ethyl acetate, the organic phases were combined, washed with saturated salt water, dried over anhydrous sodium sulfate and concentrated, and the crude product was separated and purified by silica gel column chromatography to obtain the light yellow liquid title compound **8a** (450 mg, two-step yield 33%).

LCMS: m/z=208.1 [M+H]⁺

### Step 2: 2-(3-acetaminophenyl)acetic acid 8b

Compound **8a** (450 mg, 2.2 mmol) was dissolved in tetrahydrofuran (5 mL), a water (3 mL) solution of sodium hydroxide (170 mg, 4.2 mmol) and methanol (1 mL) were added, the reaction was carried out for reaction for 1 hour at room temperature, and the reaction was complete shown by TLC. The reaction solution was added with water, adjusted to pH=4-5 with dilute hydrochloric acid (4N) and extracted with ethyl acetate, the organic phases were combined, washed with saturated salt water, dried over anhydrous sodium sulfate and concentrated to obtain the white-like solid title compound **8b** (250 mg, yield 60%).

¹H NMR (400 MHz, DMSO-*d₆*) δ 12.53-12.15 (m, 1H), 9.91 (s, 1H), 7.52-7.45 (m, 2H), 7.31-7.13 (m, 1H), 6.91 (d, *J =* 7.6 Hz, 1H), 3.51 (s, 2H), 2.02 (d, *J =* 7.8 Hz, 3H).

### Step 3: 2-(3-acetaminophenyl)-N-(4-bromo-5-chloropyridin-2-yl)acetamide 8c

Compound **8b** (500 mg, 2.6 mmol) was dissolved in ethyl acetate (10 mL), pyridine (0.8 g, 10.1 mmol), Compound **1f** (505 mg, 2.4 mmol) and 1-propylphosphonic anhydride (0.8 g, 10.4 mmol, 50% ethyl acetate solution) were added in sequence, it was stirred at room temperature overnight, and the starting material was remained shown by TLC. The reaction solution was added with water and extracted with ethyl acetate, the organic phases were combined, washed with saturated salt water, dried over anhydrous sodium sulfate and concentrated, and the crude product was separated and purified by silica gel column chromatography to obtain the light yellow solid title compound **8c** (180 mg, yield 18%).

LC-MS: m/z=381.9 [M+H]⁺

### Step 4: 2-(3-acetaminophenyl)-N-(5-chloro-4-(5,5-dimethyl-5,6-dihydro-4H-pyrrolo[1,2-b]pyrazol-3-yl)pyridin-2-yl)a cetamide 8

Compound **8c** (180 mg, 0.47 mol) was dissolved in a mixed solvent of 1,4-dioxane (6 mL) and water (2 mL), sodium carbonate (100 mg, 0.94 mmol), Intermediate **IN-1** (186 mg, 0.65 mmol) and Pd(dppf)Cl₂ dichloromethane complex (41 mg, 0.05 mmol) were added in sequence at room temperature, the temperature was lifted to 90°C for 3 hours under nitrogen protection, and the reaction was complete shown by TLC. The reaction solution was cooled to room temperature, slowly poured into water and extracted with ethyl acetate, the organic phases were combined, washed with saturated salt water, dried with anhydrous sodium sulfate and concentrated, and the crude product was separated and purified by silica gel column chromatography and purified by Prep-TLC to obtain the white solid title compound **8** (22 mg, yield 11%).

LC-MS: m/z =438.0[M+H]⁺

¹H NMR (400 MHz, DMSO-d₆) δ 10.87 (s, 1H), 9.91 (s, 1H), 8.37 (s, 1H), 8.23 (s, 1H), 8.00 (s, 1H), 7.55 (s, 1H), 7.47 (d, *J =* 8.0 Hz, 1H), 7.23 (t, *J =* 8.0 Hz, 1H), 7.01 (d, *J* = 7.6 Hz, 1H), 3.93 (s, 2H), 3.71 (s, 2H), 2.87 (s, 2H), 2.02 (s, 3H), 1.25 (s, 6H). (95.20% purity by HPLC)

### Embodiment 9

### N-(5-chloro-4-(5,5-dimethyl-5,6-dihydro-4H-pyrrolo[1,2-b]pyrazol-3-yl)pyridin-2-yl)-2-(3-(2-cyanoacetamido )phenyl)acetamide 9

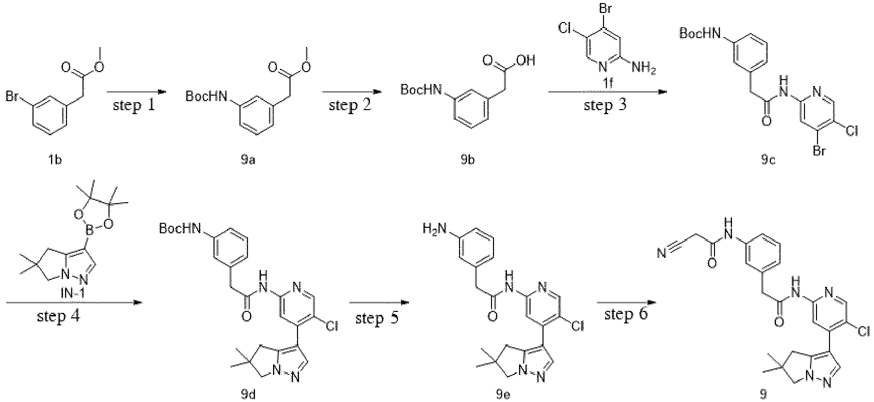

### Step 1: 2-(3-((tert-butoxycarbonyl)amino)phenyl)acetic acid methyl ester 9a

Compound **1b** (1.0 g, 4.36 mmol) and tert-butyl carbamate (0.77 g, 6.57 mmol) were dissolved in 1,4-dioxane (20 mL), cesium carbonate (2.85 g, 8.75 mmol), Pd₂(dba)₃ (120 mg, 0.13 mmol) and Xantphos (75 mg, 0.13 mmol) were added in sequence at room temperature, the temperature was lifted to 100°C for 5 hours under nitrogen protection, and the reaction was complete shown by TLC. The reaction solution was cooled to room temperature, quenched with water and extracted with ethyl acetate, the organic phases were combined, washed with saturated salt water, dried with anhydrous sodium sulfate and concentrated, the crude product was separated and purified by silica gel column chromatography to obtain the light yellow transparent liquid title compound **9a** (625 mg, yield 54%).

LC-MS: m/z=283.2 [M+Na]⁺

### Step 2: 2-(3-((tert-butoxycarbonyl)amino)phenyl)acetic acid 9b

Compound **9a** (625 mg, 2.36 mmol) was dissolved in methanol/water (10 mL/3 mL), sodium hydroxide (141 mg, 3.53 mmol) was added, the reaction was carried out for 2 hours at room temperature, and the reaction was complete shown by TLC. The reaction solution was quenched with water and extracted with ethyl acetate, the organic phase was discarded, the aqueous phase was adjusted to pH=3 with dilute hydrochloric acid (1N) and extracted with ethyl acetate, and the organic phases were combined, washed with saturated salt water, dried over anhydrous sodium sulfate and concentrated to obtain the light yellow transparent liquid title compound **9b** (666 mg, crude product), which was directly uesd for the next step.

LC-MS: m/z=274.1[M+Na]⁺

### Step 3: 3-(2-((4-bromo-5-chloropyridin-2-yl)amino)-2-oxoethyl)phenyl)carbamic acid tert-butyl ester 9c

Compound **9b** (666 mg, crude product) was dissolved in ethyl acetate (30 mL), Compound **1f** (458 mg, 2.21 mmol), 1-propylphosphonic anhydride (5.63 g, 8.85 mmol, 50% ethyl acetate solution) and pyridine (1.05 g, 13.27 mmol) were added in sequence at room temperature, the reaction was carried out overnight at room temperature, and the reaction was complete shown by TLC. The reaction solution was quenched with water and extracted with ethyl acetate, the organic phases were combined, washed with saturated salt water, dried over anhydrous sodium sulfate and concentrated, and the crude product was separated and purified by silica gel column chromatography to obtain the white solid title compound **9c** (830 mg, two-step yield 80%). LC-MS: m/z=442.0[M+H]⁺

### Step 4:

### (3-(2-((5-chloro-4-(5,5-dimethyl-5,6-dihydro-4H-pyrrolo[1,2-b]pyrazol-3-yl)pyridin-2-yl)amino)-2-oxoethyl)p henyl)carbamic acid tert-butyl ester 9d

Compound **9c** (200 mg, 0.45 mmol) and Intermediate **IN-1** (178 mg, 0.68 mmol) were dissolved in 1,4-dioxane (10 mL) and water (3 mL), potassium carbonate (125 mg, 0.90 mmol) and Pd(dppf)Cl₂ (catalytic amount) were added at room temperature, the temperature was lifted to 95°C for reaction overnight under nitrogen protection, and the reaction was complete shown by TLC. The reaction solution was cooled to room temperature, quenched with water and extracted with ethyl acetate, the organic phases were combined, washed with saturated salt water, dried over anhydrous sodium sulfate and concentrated, and the crude product was separated and purified by silica gel column chromatography to obtain the white solid title compound **9d** (78 mg, yield 35%).

LC-MS: m/z=496.2[M+H]⁺

### Step 5: 2-(3-aminophenyl)-N-(5-chloro-4-(5,5-dimethyl-5,6-dihydro-4H-pyrrolo[1,2-b]pyrazol-3-yl)pyridin-2-yl)aceta mide 9e

Compound **9d** (78 mg, 0.16 mmol) was dissolved in dichloromethane (6 mL), trifluoroacetic acid (1 mL) was added, the reaction was carried out for 1 hour at room temperature, and the reaction was complete shown by TLC. The reaction solution was quenched with water and extracted with ethyl acetate, the organic phase was discarded, the aqueous phase was adjusted to pH=9 with a saturated sodium carbonate aqueous solution and extracted with ethyl acetate, and the organic phases were combined, washed with salt water, dried over anhydrous sodium sulfate and concentrated to obtain the white solid title compound **9e** (44 mg, yield 70%). LC-MS: m/z=396.2[M+H]⁺

### Step 6: N-(5-chloro-4-(5,5-dimethyl-5,6-dihydro-4H-pyrrolo[1,2-b]pyrazol-3-yl)pyridin-2-yl)-2-(3-(2-cyanoacetamido )phenyl)acetamide 9

Compound **9e** (44 mg, 0.11 mmol) was dissolved in dichloromethane (5 mL), cyanoacetic acid (14 mg, 0.16 mmol) and EDCl (32 mg, 0.17 mmol) were added, it was stirred at room temperature for 2 hours, and the reaction was complete shown by TLC. The reaction solution was quenched with water and extracted with ethyl acetate, the organic phases were combined, washed with saturated salt water, dried over anhydrous sodium sulfate and concentrated, and the crude product was separated and purified by Prep-TLC to obtain the white solid title compound **9** (27.9 mg, yield 54%).

LC-MS: m/z=463.1[M+H]⁺

¹H NMR (400 MHz, DMSO-d₆) δ 10.89 (s, 1H), 10.31 (s, 1H), 8.37 (s, 1H), 8.22 (s, 1H), 8.00 (s, 1H), 7.52 (s, 1H), 7.45 (d, *J =* 7.8 Hz, 1H), 7.29 (t, *J =* 7.2 Hz, 1H), 7.07 (d, *J =* 8.0 Hz, 1H), 3.92 (s, 2H), 3.88 (s, 2H), 3.73 (s, 2H), 2.86 (s, 2H), 1.24 (s, 6H). (99.49% purity by HPLC)

### Embodiment 10

### 2-(3-acetaminophenyl)-N-(5-chloro-4-(5-cyano-2,2-dimethyl-2,3-dihydro-1H-pyrrolizin-7-yl)pyridin-2-yl)acet amide 10

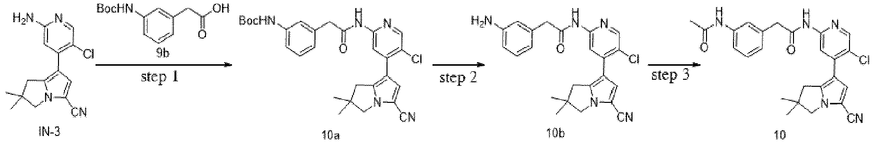

### Step 1: 3-(2-((5-chloro-4-(5-cyano-2,2-dimethyl-2,3-dihydro-1H-pyrrolyl-7-yl)pyrrolizin-2-yl)amino)-2-oxoethyl)phe nylcarbamic acid tert-butyl ester 10a

Compound **9b** (136 mg, 0.54 mmol) and Intermediate **IN-3** (130 mg, 0.45 mmol) were dissolved in ethyl acetate, 1-propylphosphonic anhydride (1.15 g, 1.81 mmol, 50% ethyl acetate solution) and pyridine (213 mg, 2.69 mmol) were added at room temperature, the temperature was lifted to 80°C for reaction for 2 hours, and the reaction was complete shown by TLC. The reaction solution was quenched with water and extracted with ethyl acetate, the organic phases were combined, washed with saturated salt water, dried over anhydrous sodium sulfate and concentrated, and the crude product was separated and purified by silica gel column chromatography to obtain the white solid title compound **10a** (200 mg, yield 71%).

LC-MS: m/z=520.2[M+H]⁺

### Step 2: 2-(3-aminophenyl)-N-(5-chloro-4-(5-cyano-2,2-dimethyl-2,3-dihydro-1H-pyrrolizin-7-yl)pyridin-2-yl)acetami de 10b

Compound **10a** (200 mg, 0.38 mmol) was dissolved in dichloromethane (6 mL), trifluoroacetic acid (3 mL) was added, the reaction was carried out for 1 hour at room temperature, and the reaction was complete shown by TLC. The reaction solution was quenched with water and extracted with ethyl acetate, the organic phase was discarded, the aqueous phase was adjusted to pH=9 with a saturated sodium carbonate aqueous solution and extracted with ethyl acetate, the organic phases were combined, washed with saturated salt water, dried over anhydrous sodium sulfate and concentrated to obtain the white solid title compound **10b** (152 mg, crude product), which was directly used for the next step.

### Step 3: 2-(3-acetaminophenyl)-N-(5-chloro-4-(5-cyano-2,2-dimethyl-2,3-dihydro-1H-pyrrolizin-7-yl)pyridin-2-yl)acet amide 10

Compound **10b** (80 mg, crude product) was dissolved in tetrahydrofuran (8 mL), potassium carbonate (39 mg, 0.28 mmol) and acetic anhydride (23 mg, 0.23 mmol) were added, the reaction was carried out for 3 hours at room temperature, and the reaction was complete shown by TLC. The reaction solution was quenched with water and extracted with ethyl acetate, the organic phases were combined, washed with saturated salt water, dried over anhydrous sodium sulfate and concentrated, and the crude product was separated and purified by Prep-TLC to obtain the white solid title compound **10** (62 mg, two-step yield 66%).

LC-MS: m/z=462.2 [M+H]^{+ 1}H NMR (400 MHz, DMSO-d₆) δ 10.93 (s, 1H), 9.98 (s, 1H), 8.39 (s, 1H), 8.19 (s, 1H), 7.57 (s, 1H), 7.47 (d, *J* = 8.0 Hz, 1H), 7.41 (s, 1H), 7.22 (t, *J* = 8.0 Hz, 1H), 7.00 (d, *J =* 7.6 Hz, 1H), 3.93 (s, 2H), 3.70 (s, 2H), 2.84 (s, 2H), 2.02 (s, 3H), 1.21 (s, 6H). (99.05% purity by HPLC)

### Embodiment 11

### (S)-N-(5-chloro-4-(5,5-dimethyl-5,6-dihydro-4H-pyrrolo[1,2-b]pyrazol-3-yl)pyridin-2-yl)-2-(pyridin-3-yl)prop ionamide (assumed) 11-1

### (R)-N-(5-chloro-4-(5,5-dimethyl-5,6-dihydro-4H-pyrrolo[1,2-b]pyrazol-3-yl)pyridin-2-yl)-2-(pyridin-3-yl)prop ionamide (assumed) 11-2

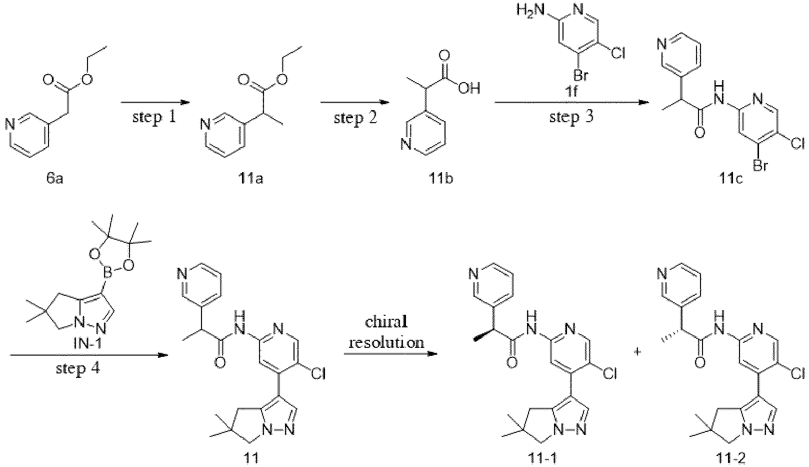

### Step 1: 2-(pyridin-3-yl)propanoic acid ethyl ester 11a

Ethyl 3-pyridylacetate **6a** (1.0 g, 6.05 mmol) was dissolved in tetrahydrofuran (10 mL), it was cooled to 0°C, potassium tert-butoxide (680 mg, 6.06 mmol) was added, the reaction was carried out at 0°C for 30 minutes, methyl iodide (3.70 g, 26.07 mmol) was added, the temperature was lifted to room temperature for reaction for 1 hour, and the reaction was complete shown by TLC. The reaction solution was quenched with water and extracted with ethyl acetate, the organic phases were combined, washed with saturated salt, dried over anhydrous sodium sulfate and concentrated, and the crude product was separated and purified by silica gel column chromatography to obtain the yellow oily liquid title compound **11a** (600 mg, yield 55%).

### Step 2: 2-(pyridin-3-yl)propionic acid 11b

Compound **11a** (0.6 g, 3.35 mmol) was dissolved in methanol (15 mL) and water (5 mL), sodium hydroxide (0.4 g, 10.00 mmol) was added, the reaction was carried out for 1 hour at room temperature, and the reaction was complete shown by TLC. The reaction solution was quenched with water and extracted with ethyl acetate, the organic phase was discarded, the aqueous phase was adjusted to pH=3 with dilute hydrochloric acid (1N) and extracted with ethyl acetate, and the organic phases were combined, washed with saturated salt water, dried over anhydrous sodium sulfate and concentrated to obtain the white solid title compound **11b** (0.5 g, crude product), which was directly used for the next step.

### Step 3: N-(4-bromo-5-chloropyridin-2-yl)-2-(pyridin-3-yl)propionamide 11c

Compound **11b** (0.5 g, crude product) and compound 1f (0.69 g, 3.32 mmol) were dissolved in ethyl acetate (20 mL), 1-propylphosphonic anhydride (2.1 g, 3.30 mmol, 50% ethyl acetate solution) and pyridine (0.78 g, 9.86 mmol) were added, the reaction was carried out for 2 hours at room temperature, and the reaction was complete shown by TLC. The reaction solution was quenched with water and extracted with ethyl acetate, the organic phases were combined, washed with saturated salt water, dried over anhydrous sodium sulfate and concentrated, and the crude product was separated and purified by silica gel column chromatography to obtain the white solid title compound **11c** (600 mg, two-step yield 53%).

### Step 4: (S)-N-(5-chloro-4-(5,5-dimethyl-5,6-dihydro-4H-pyrrolo[1,2-b]pyrazol-3-yl)pyridin-2-yl)-2-(pyridin-3-yl)prop ionamide (assumed) 11-1 &

### (R)-N-(5-chloro-4-(5,5-dimethyl-5,6-dihydro-4H-pyrrolo[1,2-b]pyrazol-3-yl)pyridin-2-yl)-2-(pyridin-3-yl)prop ionamide (assumed) 11-2

Compound **11c** (176 mg, 0.52 mmol) and Intermediate **IN-1** (150 mg, 0.57 mmol) were dissolved in 1,4-dioxane (4 mL) and water (2 mL), sodium carbonate (82 mg, 0.77 mmol) and d(dppf)Cl₂ (38 mg, 0.05 mmol) were added at room temperature, the temperature was lifted to 80°C for reaction for 2 hours, and the reaction was complete shown by TLC. The reaction solution was cooled to room temperature, quenched with water and extracted with ethyl acetate, the organic phases were combined, washed with saturated salt water, dried with anhydrous sodium sulfate and concentrated, the crude product was separated and purified by silica gel column chromatography to obtain the white solid compound **11** (44 mg, yield 22%), and chiral resolved (DAICEL AD-H, 30*250mm, 5um, 30mL/min, EtOH:Hexane=40:60) to obtain the title compound **11-1** (RT 31.7 min) (16 mg, yield 36%) and the title compound **11-2** (RT 44.2 min) (17 mg, yield 39%).

### Compound 11-1

LC-MS: m/z=396.2 [M+H]⁺

¹H NMR(400 MHz, CDCl₃) δ 9.52 (br, 1H), 9.27 (s, 1H), 8.66 (s, 1H), 8.34 (s, 1H), 8.23-8.18 (m, 2H), 8.06 (s, 1H), 7.74-7.66 (m, 1H), 4.53-4.38 (m, 1H), 3.95 (s, 2H), 2.94 (s, 2H), 1.67 (d, *J =* 6.4 Hz, 3H), 1.34 (s, 6H).

(100% purity by HPLC)

### Compound 11-2

LC-MS: m/z=396.2 [M+H]⁺

¹H NMR(400 MHz, CDCl₃) δ 8.96 (s, 1H), 8.66-8.60 (m, 2H), 8.21 (d, *J* = 10.4 Hz, 2H), 8.07 (s, 1H), 8.04 (d, *J* = 7.2 Hz, 1H), 7.52-7.50 (m, 1H), 4.17-4.07 (m, 1H), 3.95 (s, 2H), 2.95 (s, 2H), 1.65 (d, *J =* 6.4 Hz, 3H), 1.35 (s, 6H). (98.85% purity by HPLC)

### Embodiment 12

### N-(5-chloro-4-(5,5-dimethyl-5,6-dihydro-4H-pyrrolo[1,2-b]pyrazol-3-yl)pyridin-2-yl)-2-(6-methoxypyridin-3-yl)acetamide 12

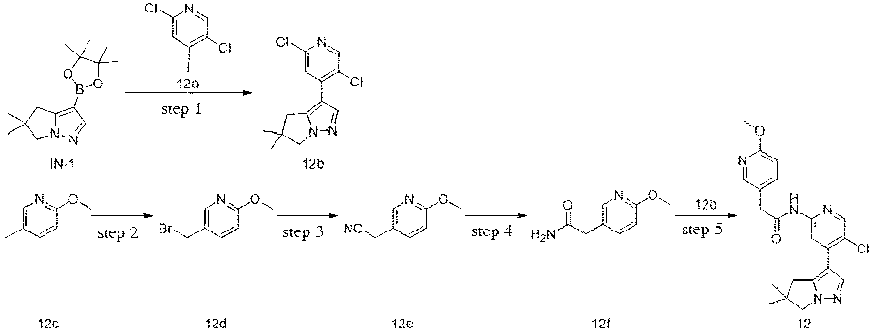

### Step 1: 3-(2,5-dichloropyridin-4-yl)-5,5-dimethyl -5,6-dihydro-4H-pyrrolo[1,2-b]pyrazole 12b

Intermediate **IN-1** (230 mg, 0.88 mmol) and 2,5-dichloro-4-iodopyridine **12a** (200 mg, 0.73 mmol) were dissolved in 1,4-dioxane (6 mL) and water (2mL), potassium carbonate (202 mg, 1.46 mmol), Pd(dppf)Cl₂ dichloromethane complex (50 mg, 0.06 mmol) were added, it was replaced with nitrogen three times, the temperature was lifted to 85°C, and it was stirred for 3 hours. The reaction was complete shown by TLC. The reaction solution was cooled to room temperature, quenched with water and extracted with ethyl acetate, the organic phases were combined, washed with saturated salt water, dried with anhydrous sodium sulfate and concentrated, and the crude product was purified by silica gel column chromatography to obtain the light yellow solid title compound **12b** (108 mg, yield 52%).

### Step 2: 5-(bromomethyl)-2-methoxypyridine 12d

2-methoxy-5-methylpyridine **12c** (2.0 g, 16.24 mmol) was dissolved in carbon tetrachloride (40 mL), *N*-bromosuccinimide (3.18 g, 17.87 mmol) and azodiisobutyronitrile (0.27 g, 1.64 mmol) were added at room temperature, the temperature was lifted to 70°C for reaction for 2 hours, and the reaction was complete shown by TLC. The reaction solution was concentrated, triturated with ethyl acetate and filtered, and the filter cake was washed and dried to obtain the yellow liquid title compound **12d** (2.9 g, yield 88%).

### Step 3: 2-(6-methoxypyridin-3-yl)acetonitrile 12e

Compound **12d** (3.0 g, 14.85 mmol) was dissolved in acetonitrile (30 mL), trimethylcyanosilane (2.50 g, 25.20 mmol) and potassium carbonate (2.79 g, 20.18 mmol) were added at room temperature, the temperature was lifted to 70°C for reaction for 2 hours, and the reaction was complete shown by TLC. The reaction solution was cooled to room temperature, quenched with water and extracted with ethyl acetate, the organic phases were combined, washed with saturated salt water, dried over anhydrous sodium sulfate and concentrated, and the crude product was separated and purified by silica gel column chromatography to obtain the colorless liquid title compound **12e** (450 mg, yield 21%).

### Step 4: 2-(6-methoxypyridin-3-yl)acetamide 12f

Compound **12e** (350 mg, 2.36 mmol) was dissolved in dimethyl sulfoxide (15 mL), potassium carbonate (653 mg, 4.72 mmol) and hydrogen peroxide (536 mg, 30%) were added at room temperature, the temperature was lifted to 50°C for reaction for 1 hour, and the reaction was complete shown by TLC. The reaction solution was cooled to room temperature, quenched with water and extracted with ethyl acetate, the organic phases were combined, washed with saturated salt water, dried with anhydrous sodium sulfate and concentrated, and the crude product was separated and purified by silica gel column chromatography to obtain the white solid title compound **12f** (95 mg, yield 24.2%).

¹H NMR (400 MHz, DMSO-*d₆*) δ 8.00 (d, *J* = 2.0 Hz, 1H), 7.58 (dd, *J* = 2.4 Hz, 8.8 Hz, 1H), 7.49 (br, 1H), 6.91 (br, 1H), 6.76 (d, *J* = 8.4 Hz, 1H), 3.82 (s, 3H), 3.32 (s, 2H).

### Step 5: N-(5-chloro-4-(5,5-dimethyl-5,6-dihydro-4H-pyrrolo[1,2-b]pyrazol-3-yl)pyridin-2-yl)-2-(6-methoxypyridin-3-yl)acetamide 12

Compound **12f** (33 mg, 0.20 mmol) and Compound **12b** (50 mg, 0.18 mmol) were dissolved in 1,4-dioxane (10 mL), cesium carbonate (118 mg, 0.36 mmol), Xphos (50 mg, 0.10 mmol) and Pd₂(dba)₃ (50 mg, 0.05 mmol) were added in sequence at room temperature, the temperature was lifted to 100°C for reaction for 3 hours under nitrogen protection, and the reaction was complete shown by TLC. The reaction solution was cooled to room temperature, quenched with water and extracted with ethyl acetate, the organic phases were combined, washed with saturated salt water, dried over anhydrous sodium sulfate and concentrated, and the crude product was purified by Prep-TLC to obtain the white solid title compound **12** (13 mg, yield 16%). LC-MS: m/z= 412.2 [M+H] ⁺

¹H NMR (400 MHz, DMSO-*d₆*) δ 10.90 (s, 1H), 8.38 (s, 1H), 8.21 (s, 1H), 8.10 (d, *J =* 2.0 Hz, 1H), 8.00 (s, 1H), 7.67 (dd, *J =* 2.4 Hz, 8.4 Hz, 1H), 6.80 (d, *J* = 8.4 Hz, 1H), 3.96 (s, 2H), 3.83 (s, 3H), 3.71 (s, 2H), 2.86 (s, 2H), 1.25 (s, 6H). (98.66% purity by HPLC)

### Embodiment 13

### (S)-N-(5-chloro-4-(5,5-dimethyl-5,6-dihydro-4H-pyrrolo[1,2-b]pyrazol-3-yl)pyridin-2-yl)-2-(6-cyanopyridin-2 -yl)propionamide (assumed) 13-1

### (R)-N-(5-chloro-4-(5,5-dimethyl-5,6-dihydro-4H-pyrrolo[1,2-b]pyrazol-3-yl)pyridin-2-yl)-2-(6-cyanopyridin-2 -yl)propionamide (assumed) 13-2

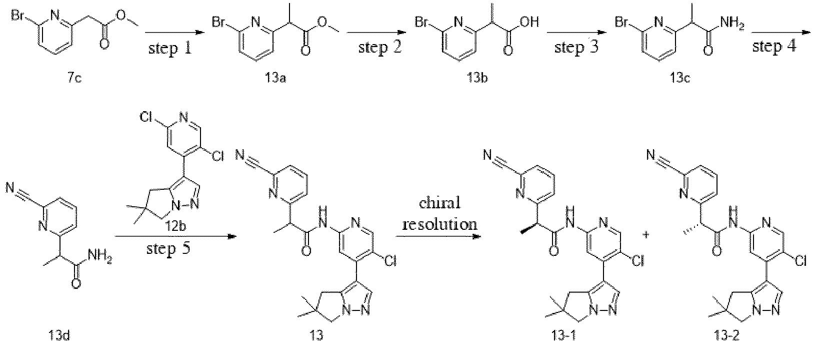

### Step 1: 2-(6-bromopyridin-2-yl)propanoic acid methyl ester 13a

Compound **7c** (5.4 g, 23.47 mmol) was dissolved in anhydrous tetrahydrofuran (40 mL), it was cooled to 0°C, potassium tert-butoxide (2.90 g, 25.8 mmol) was added, the reaction was carried out at 0°C for 30 minutes, iodomethane (3.33 g, 23.46 mmol) was added, the temperature was lifted to room temperature for reaction for 1 hour, and the reaction was complete shown by TLC. The reaction solution was quenched with dilute hydrochloric acid (1N) and extracted with ethyl acetate, the organic phases were combined, washed with saturated salt water, dried over anhydrous sodium sulfate and concentrated, and the crude product was separated and purified by silica gel column chromatography to obtain the white solid title compound **13a** (3.9 g, yield 68%).

### Step 2: 2-(6-bromopyridin-2-yl)propionic acid 13b

Compound **13a** (1.4 g, 5.74 mmol) was dissolved in methanol (10 mL) and water (5 mL), sodium hydroxide (0.46 g, 11.50 mmol) was added, the reaction was carried out for 2 hours at room temperature, and the reaction was complete shown by TLC. The reaction solution was quenched with water and extracted with ethyl acetate, the organic phase was discarded, the aqueous phase dilute hydrochloric acid (1N) was adjusted to pH=3 and extracted with ethyl acetate, and the organic phases were combined, washed with saturated salt water, dried over anhydrous sodium sulfate and concentrated to obtain the white solid title compound **13b** (1.1 g, crude product), which was directly used for the next step.

### Step 3: 2-(6-bromopyridin-2-yl)propionamide 13c

Compound **13b** (1.1 g, crude product) was dissolved in *N,N*-dimethylformamide (15 mL), ammonium chloride (0.77 g, 14.39 mmol), HATU (2.73 g, 7.18 mmol) and *N,N*-diisopropylethylamine (1.23 g, 9.52 mmol) were added in sequence, it was stirred at room temperature for 2 hours, and the reaction was complete shown by TLC. The reaction solution was quenched with water and extracted with ethyl acetate, the organic phases were combined, washed with saturated salt water, dried over anhydrous sodium sulfate and concentrated, and the crude product was separated and purified by silica gel column chromatography to obtain the white solid title compound **13c** (1.0 g, two-step yield 76%).

### Step 4: 2-(6-cyanopyridin-2-yl)propionamide 13d

Compound **13c** (1.6 g, 6.98 mmol) was dissolved in *N,N*-dimethylformamide (20 mL), zinc cyanide (1.64 g, 13.97 mmol) and tetrakis(triphenylphosphine)palladium (500 mg, 0.43 mmol) were added at room temperature, the temperature was lifted to 100°C for reaction overnight under nitrogen protection, and the reaction was complete shown by TLC. The reaction solution was cooled to room temperature, quenched with water and extracted with ethyl acetate, the organic phases were combined, washed with saturated salt water, dried with anhydrous sodium sulfate and concentrated, and the crude product was separated and purified by silica gel column chromatography to obtain the white solid title compound **13d** (640 mg, yield 52%).

LC-MS: m/z=176.2 [M+H]⁺

### Step 5:

### (S)-N-(5-chloro-4-(5,5-dimethyl-5,6-dihydro-4H-pyrrolo[1,2-b]pyrazol-3-yl)pyridin-2-yl)-2-(6-cyanopyridin-2 -yl)propanamide (assumed) 13-1 &

### (R)-N-(5-chloro-4-(5,5-dimethyl-5,6-dihydro-4H-pyrrolo[1,2-b]pyrazol-3-yl)pyridin-2-yl)-2-(6-cyanopyridin-2 -yl)propionamide (assumed) 13-2

Compound **13d** (94 mg, 0.54 mmol) and Compound **12b** (150 mg, 0.53 mmol) were dissolved in 1,4-dioxane (15 mL), cesium carbonate (346 mg, 1.06 mmol), Xphos (50 mg, 0.10 mmol) and Pd₂(dba)₃ (50 mg, 0.05 mmol) were added at room temperature, the temperature was lifted to 100°C for reaction for 3 hours under nitrogen protection, and the reaction was complete shown by TLC. The reaction solution was cooled to room temperature, quenched with water and extracted with ethyl acetate, the organic phases were combined, washed with saturated salt, dried over anhydrous sodium sulfate and concentrated, and the crude product was purified by Pre-TLC to obtain the white solid compound **13** (80 mg, yield 35%). The title compound **13-1** (RT 18.0 min) (15 mg, yield 19%) and the title compound **13-2** (RT 53.0 min) (16 mg, yield 20%) were obtained by chiral resolution (DAICEL AD-H, 30*250mm, 5um, 30mL/min, IPA:Hexane=40:60).

LC-MS: m/z=421.1 [M+H]⁺

### Compound 13-1

¹H NMR(400 MHz, DMSO-*d₆*) δ 10.96 (s, 1H), 8.38 (s, 1H), 8.21 (s, 1H), 8.06 (t, *J* = 7.6 Hz, 1H), 8.00 (s, 1H), 7.95 (d, *J* = 7.2 Hz, 1H), 7.83 (d, *J* = 8.0 Hz, 1H), 4.31 (q, *J =* 7.2 Hz, 1H), 3.94 (s, 2H), 2.88 (s, 2H), 1.51 (d, *J* = 6.8 Hz, 3H), 1.26 (s, 6H). (99.30% purity by HPLC)

### Compound 13-2

¹H NMR(400 MHz, DMSO-*d₆*) δ 10.96 (s, 1H), 8.38 (s, 1H), 8.21 (s, 1H), 8.05 (t, *J* = 7.6 Hz, 1H), 8.00 (s, 1H), 7.95 (d, *J* = 7.2 Hz, 1H), 7.83 (d, *J* = 8.0 Hz, 1H), 4.31 (q, *J* = 7.2 Hz, 1H), 3.93 (s, 2H), 2.88 (s, 2H), 1.51 (d, *J* = 7.2 Hz, 3H), 1.26 (s, 6H). (100% purity by HPLC)

### Embodiment 14

### (S)-2-(3-acetaminophenyl)-N-(5-chloro-4-(5,5-dimethyl-5,6-dihydro-4H-pyrrolo[1,2-b]pyrazol-3-yl)pyridin-2-yl)propionamide (assumed) 14-1

### (R)-2-(3-acetaminophenyl)-N-(5-chloro-4-(5,5-dimethyl-5,6-dihydro-4H-pyrrolo[1,2-b]pyrazol-3-yl)pyridin-2-yl)propionamide (assumed) 14-2

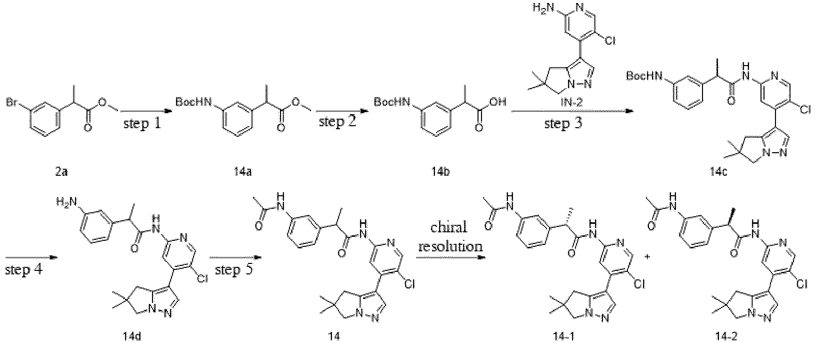

### Step 1: 2-(3-((tert-butoxycarbonyl)amino)phenyl)propanoic acid ethyl ester 14a

Compound **2a** (1.73 g, 7.12 mmol) was dissolved in 1,4-dioxane (30 mL), tert-butyl carbamate (1.25 g, 10.7 mmol), Pd₂(dba)₃ (327 mg, 0.36 mmol) and Xphos (509 mg, 1.07 mmol) were added at room temperature, the temperature was lifted to 100°C for reaction for 2 hours under nitrogen protection, and the reaction was complete shown by TLC. The reaction solution was cooled to room temperature, quenched with water and extracted with ethyl acetate, the organic phases were combined, washed with saturated salt water, dried over anhydrous sodium sulfate and concentrated, and the crude product was separated and purified by silica gel column chromatography to obtain the brown liquid title compound **14a** (1.6 g, yield 80%).

### Step 2: 2-(3-((tert-butoxycarbonyl)amino)phenyl)propionic acid 14b

Compound **14a** (1.6 g, 5.73 mmol) was dissolved in ethanol (8 mL) and water (4 mL), sodium hydroxide (0.46 g, 11.50 mmol) was added, it was stirred at room temperature for 1 hour, and the reaction was complete shown by TLC. The reaction solution was quenched with water and extracted with ethyl acetate, the organic phase was discarded, the aqueous phase was adjusted to pH=3 with dilute hydrochloric acid (1N) and extracted with ethyl acetate, and the organic phases were combined, washed with saturated salt water, dried over anhydrous sodium sulfate and concentrated to obtain the white solid title compound **14b** (1.0 g, crude product), which was directly used for the next step.

### Step 3:

### 3-(1-((5-chloro-4-(5,5-dimethyl-5,6-dihydro-4H-pyrrolo[1,2-b]pyrazol-3-yl)pyridin-2-yl)amino)-1-oxopropane -2-yl)phenyl)carbamic acid tert-butyl ester 14c

Compound 14b (250 mg, crude product) and Intermediate **IN-2** (247 mg, 0.94 mmol) were dissolved in ethyl acetate (8 mL), 1-propylphosphonic anhydride (2.4 g, 3.77 mmol, 50% ethyl acetate solution) and pyridine (473 mg, 5.98 mmol) were added at room temperature, the reaction was carried out overnight at room temperature, and the reaction was complete shown by TLC. The reaction solution was cooled to room temperature, quenched with water and extracted with ethyl acetate, the organic phases were combined, washed with saturated salt water, dried with anhydrous sodium sulfate and concentrated, and the crude product was separated and purified by silica gel column chromatography to obtain the white solid title compound **14c** (280 mg, yield 58%).

### Step 4:

### 2-(3-aminophenyl)-N-(5-chloro-4-(5,5-dimethyl-5,6-dihydro-4H-pyrrolo[1,2-b]pyrazol-3-yl)pyridin-2-yl)propi onamide 14d

Compound **14c** (280 mg, 0.55 mmol) was dissolved in dichloromethane (6 mL), trifluoroacetic acid (3 mL) was added, the reaction was carried out for 2 hours at room temperature, and the reaction was complete shown by TLC. The reaction solution was quenched with water and extracted with ethyl acetate, the organic phase was discarded, the aqueous phase was adjusted to pH=9 with saturated sodium carbonate aqueous solution and extracted with ethyl acetate, and the organic phases were combined, washed with saturated salt water, dried over anhydrous sodium sulfate and concentrated to obtain the white solid title compound **14d** (210 mg, crude product), which was directly used for the next step.

### Step 5:

### (S)-2-(3-acetaminophenyl)-N-(5-chloro-4-(5,5-dimethyl-5,6-dihydro-4H-pyrrolo[1,2-b]pyrazol-3-yl)pyridin-2-yl)propionamide (assumed) 14-1 &

### (R)-2-(3-acetaminophenyl)-N-(5-chloro-4-(5,5-dimethyl-5,6-dihydro-4H-pyrrolo[1,2-b]pyrazol-3-yl)pyridin-2-yl)propionamide (assumed) 14-2

Compound **14d** (210 mg, crude product) was dissolved in tetrahydrofuran (10 mL), acetic anhydride (72 mg, 0.71 mmol) and potassium carbonate (122 mg, 0.88 mmol) were added, the reaction was carried out for 2 hours at room temperature, and the reaction was complete shown by TLC. The reaction solution was quenched with water and extracted with ethyl acetate, the organic phases were combined, washed with saturated salt water, dried over anhydrous sodium sulfate and concentrated, and the crude product was separated and purified by Prep-TLC to obtain the white solid compound **14** (190 mg, yield 82%). The title compound **14-1** (RT 13.9 min) (60 mg, 32%) and the title compound **14-2** (RT 18.3 min) (60 mg, yield 32%) was obtained by chiral resolution (DAICEL AD-H, 30*250mm, 5um, 30mL/min, IPA:Hexane=40:60).

### Compound 14-1

LC-MS: m/z=452.2 [M+H]⁺

¹H NMR (400 MHz, DMSO-d₆) δ 10.81 (s, 1H), 9.92 (s, 1H), 8.34 (s, 1H), 8.23 (s, 1H), 7.98 (s, 1H), 7.58 (s, 1H), 7.48 (d, *J =* 7.6 Hz, 1H), 7.23 (t, *J =* 7.6 Hz, 1H), 7.07 (d, *J =* 7.6 Hz, 1H), 4.00 (q, *J* = 7.2 Hz, 1H), 3.93 (s, 2H), 2.88 (s, 2H), 2.01 (s, 3H), 1.39 (d, *J* = 6.8 Hz, 3H), 1.26 (s, 6H). (99.42% purity by HPLC)

### Compound 14-2

LC-MS: m/z=452.2 [M+H]⁺

¹H NMR (400 MHz, DMSO-d₆) δ 10.81 (s, 1H), 9.93 (s, 1H), 8.34 (s, 1H), 8.23 (s, 1H), 7.98 (s, 1H), 7.58 (s, 1H), 7.48 (d, *J =* 8.0 Hz, 1H), 7.23 (t, *J =* 8.0 Hz, 1H), 7.07 (d,*J* = 8.0 Hz, 1H), 4.00 (q, *J =* 6.8 Hz, 1H), 3.93 (s, 2H), 2.88 (s, 2H), 2.01 (s, 3H), 1.39 (d, *J* = 7.2 Hz, 3H), 1.26 (s, 6H). (99.12% purity by HPLC)

### Embodiment 15

### (S)-N-(5-chloro-4-(5,5-dimethyl-5,6-dihydro-4H-pyrrolo[1,2-b]pyrazol-3-yl)pyridin-2-yl)-2-(2-cyanopyridin-4 -yl)propionamide (assumed) 15-1

### (R)-N-(5-chloro-4-(5,5-dimethyl-5,6-dihydro-4H-pyrrolo[1,2-b]pyrazol-3-yl)pyridin-2-yl)-2-(2-cyanopyridin-4 -yl)propionamide (assumed) 15-2

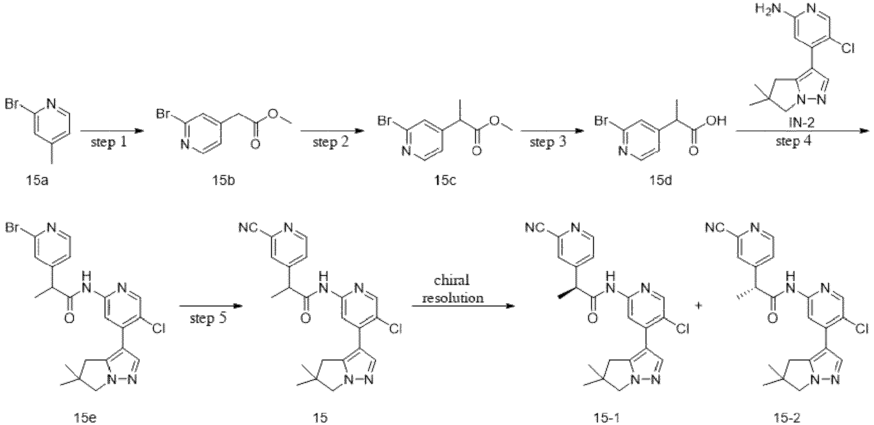

### Step 1: 2-(2-bromopyridin-4-yl) acetic acid methyl ester 15b

2-bromine-4-methylpyridine **15a** (10.0 g, 58.13 mmol) was dissolved in anhydrous tetrahydrofuran (150 mL), the temperature was lowered to -70°C, lithium diisopropylamine (87.2 mL, 174.4 mmol, 2M tetrahydrofuran solution) was slowly added, after addition the reaction was carried out at -70°C for 1 hour, methyl chloroformate (7.14 g, 75.56 mmol) was slowly added dropwise, the temperature was slowly lifted to room temperature for reaction for 1 hour, and the reaction was complete shown by TLC. The reaction solution was quenched with a saturated ammonium chloride aqueous solution and extracted with ethyl acetate, the organic phases were combined, washed with saturated salt water, dried over anhydrous sodium sulfate and concentrated, and the crude product was separated and purified by silica gel column chromatography to obtain the yellow liquid title compound **15b** (9.5 g, yield 71%).

¹H NMR (400 MHz, DMSO-*d₆*) δ 8.35 (d, *J =* 4.8 Hz, 1H), 7.62 (s, 1H), 7.39 (dd, *J =* 4.2, 1.2 Hz, 1H), 3.82 (s, 2H), 3.66 (s, 3H).

### Step 2: 2-(2-bromopyridin-4-yl)propanoic acid methyl ester 15c

Compound **15b** (6.0 g, 26.08 mmol) was dissolved in anhydrous tetrahydrofuran (60 mL), it was cooled to 0°C, potassium tert-butoxide (2.90 g, 25.84 mmol) was added, the reaction was carried out at 0°C for 30 minutes, iodomethane (3.70 g, 26.07 mmol) was added, the reaction was lifted to room temperature for reaction for 1 hour, and the reaction was complete shown by TLC. The reaction solution was quenched with water and extracted with ethyl acetate, the organic phases were combined, washed with saturated salt water, dried over anhydrous sodium sulfate and concentrated, and the crude product was separated and purified by silica gel column chromatography to obtain the yellow oily liquid title compound **15c** (4.4 g, yield 69%).

LC-MS: m/z=245.1 [M+H]⁺

### Step 3: 2-(2-bromopyridin-4-yl)propionic acid 15d

Compound **15c** (1.0 g, 4.10 mmol) was dissolved in tetrahydrofuran (8 mL) and water (4 mL), sodium hydroxide (0.33 g, 8.25 mmol) was added, the reaction was carried out for 1 hour at room temperature, and the reaction was complete shown by TLC. The reaction solution was quenched with water and extracted with ethyl acetate, the organic phase was discarded, the aqueous phase was adjusted to pH=3 with dilute hydrochloric acid (1N) and extracted with ethyl acetate, and the organic phases were combined, washed with saturated salt water, dried over anhydrous sodium sulfate and concentrated to obtain the white solid title compound **15d** (365 mg, crude product), which was directly used for the next step.

LC-MS: m/z=230.0[M+H]+

### Step 4:

### 2-(2-bromopyridin-4-yl)-N-(5-chloro-4-(5,5-dimethyl-5,6-dihydro-4H-pyrrolo[1,2-b]pyrazol-3-yl)pyridin-2-yl) propionamide 15e

Compound **15d** (365 mg, crude product) and Intermediate **IN-2** (380 mg, 1.45 mmol) were dissolved in ethyl acetate (20 mL), 1-propylphosphonic anhydride (3.7 g, 5.81 mmol, 50% ethyl acetate solution) and pyridine (687 mg, 8.68 mmol) were added, the reaction was carried out for 2 hours at room temperature, and the reaction was complete shown by TLC. The reaction solution was quenched with water and extracted with ethyl acetate, the organic phases were combined, washed with saturated salt water, dried over anhydrous sodium sulfate and concentrated, and the crude product was separated and purified by silica gel column chromatography to obtain the white solid title compound **15e** (610 mg, yield 89%).

### Step 5:

### (S)-N-(5-chloro-4-(5,5-dimethyl-5,6-dihydro-4H-pyrrolo[1,2-b]pyrazol-3-yl)pyridin-2-yl)-2-(2-cyanopyridin-4 -yl)propionamide (assumed) 15-1 &

### (R)-N-(5-chloro-4-(5,5-dimethyl-5,6-dihydro-4H-pyrrolo[1,2-b]pyrazol-3-yl)pyridin-2-yl)-2-(2-cyanopyridin-4 -yl)propionamide (assumed) 15-2

Compound **15e** (610 mg, 1.28 mmol) was dissolved in N,N-dimethylformamide (30 mL), zinc cyanide (456 mg, 3.88 mmol) and tetrakis(triphenylphosphine)palladium (100 mg, 0.09 mmol) were added at room temperature, the temperature was lifted to 100°C for reaction overnight under nitrogen protection, and the reaction was complete shown by TLC. The reaction solution was cooled to room temperature, quenched with water and extracted with ethyl acetate, the organic phases were combined, washed with saturated salt water, dried with anhydrous sodium sulfate and concentrated, and the crude product was separated and purified by silica gel column chromatography to obtain the white solid compound **15** (510 mg, yield 93%). Compound **15** (100 mg) was chirally resolved (NANOMICRO AD-H, 30*250mm, 5um, 30mL/min, EtOH:Hexane=40:60) to obtain the title compound **15-1** (RT 14.5 min) (36 mg, yield 36%) and the title compound **15-2** (RT 21.5 min) (32 mg, yield 32%).

### Compound 15-1

LC-MS: m/z=421.1 [M+H]⁺

¹H NMR (400 MHz, DMSO-*d₆*) δ 11.01 (s, 1H), 8.71 (s, 1H), 8.37-7.99 (m, 4H), 7.75 (s, 1H), 4.17 (s, 1H), 3.93 (s, 2H), 2.87 (s, 2H), 1.49 (s, 3H), 1.25 (s, 6H). (99.74% purity by HPLC)

### Compound 15-2

LC-MS: m/z=421.1 [M+H]⁺

¹H NMR (400 MHz, DMSO-d₆) δ 11.00 (s, 1H), 8.71 (s, 1H), 8.38-7.98 (m, 4H), 7.75 (s, 1H), 4.16 (s, 1H), 3.93 (s, 2H), 2.87 (s, 2H), 1.49 (s, 3H), 1.25 (s, 6H). (98.76% purity by HPLC)

### Embodiment 16

### (S)-N-(5-chloro-4-(5,5-dimethyl-5,6-dihydro-4H-pyrrolo[1,2-b]pyrazol-3-yl)pyridin-2-yl)-2-(2-methoxypyridi n-4-yl)propionamide (assumed) 16-1

### (R)-N-(5-chloro-4-(5,5-dimethyl-5,6-dihydro-4H-pyrrolo[1,2-b]pyrazol-3-yl)pyridin-2-yl)-2-(2-methoxypyridi n-4-yl)propionamide (assumed) 16-2

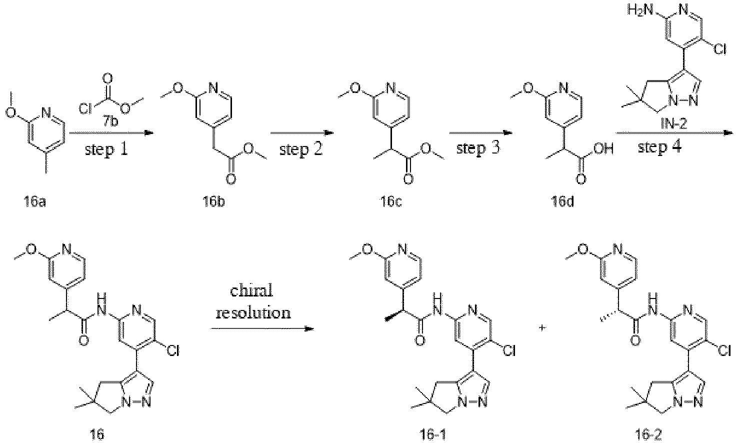

### Step 1: 2-(2-methoxypyridin-4-yl)acetic acid methyl ester 16b

2-methoxy-4-methylpyridine **16a** (2.0 g, 16.2 mmol) was dissolved in anhydrous tetrahydrofuran (20 mL), under nitrogen protection, the temperature was lowered to -60°C, lithium diisopropylamide (20.4 mL, 40.8 mmol, 2M tetrahydrofuran solution) was slowly added, it was stirred at -60°C for 2 hours, methyl chloroformate (2.0 g, 21.2 mmol) was added, after dropwise addition it was stirred at room temperature for 1 hour. The reaction was complete shown by TLC. The reaction solution was poured into water and extracted with ethyl acetate, the organic phases were combined, washed with saturated salt water, dried over anhydrous sodium sulfate and concentrated, and the crude product was chromatographed on silica gel column to obtain the yellow liquid title compound **16b** (1.3 g, yield 44%).

LC-MS: m/z=182.1 [M+H]⁺

¹HNMR (400 MHz, DMSO-*d₆*) δ 8.11 (d, *J =* 5.2 Hz, 1H), 6.91 (dd, *J* =1.2, 5.2 Hz, 1H), 6.74 (d, *J =* 0.8 Hz, 1H), 3.85 (s, 3H), 3.72 (s, 2H), 3.64 (s, 3H).

### Step 1: 2-(2-methoxypyridin-4-yl)propanoic acid methyl ester 16c

Compound **16b** (3.75 g, 20.70 mmol) was dissolved in anhydrous tetrahydrofuran (40 mL), it was cooled to 0°C, potassium tert-butoxide (2.32 g, 20.68 mmol) was added, the reaction was carried out at 0°C for 30 minutes, iodomethane (2.94 g, 20.71 mmol) was added, the reaction was lifted to room temperature for reaction for 1 hour, and the reaction was complete shown by TLC. The reaction solution was quenched with water and extracted with ethyl acetate, the organic phases were combined, washed with saturated salt water, dried over anhydrous sodium sulfate and concentrated, and the crude product was separated and purified by silica gel column chromatography to obtain the yellow oily liquid title compound **16c** (2.3 g, yield 57%).

### Step 3: 2-(2-methoxypyridin-4-yl)propionic acid 16d

Compound **16c** (500 mg, 2.56 mmol) was dissolved in methanol (10 mL) and water (5 mL), sodium hydroxide (210 mg, 5.25 mmol) was added, the reaction was carried out for 1 hour at room temperature, and the reaction was complete shown by TLC. The reaction solution was quenched with water and extracted with ethyl acetate, the organic phase was discarded, the aqueous phase was adjusted to pH=3 with dilute hydrochloric acid (1N) and extracted with ethyl acetate, the organic phases were combined, washed with saturated salt water, dried over anhydrous sodium sulfate and concentrated to obtain the white solid title compound **16d** (180 mg, yield 39%).

LC-MS: m/z=182.1[M+H]⁺

### Step 4:

### (S)-N-(5-chloro-4-(5,5-dimethyl-5,6-dihydro-4H-pyrrolo[1,2-b]pyrazol-3-yl)pyridin-2-yl)-2-(2-methoxypyridi n-4-yl)propionamide (assumed) 16-1 &

### (R)-N-(5-chloro-4-(5,5-dimethyl-5,6-dihydro-4H-pyrrolo[1,2-b]pyrazol-3-yl)pyridin-2-yl)-2-(2-methoxypyridi n-4-yl)propionamide (assumed) 16-2

Compound **16d** (180 mg, 0.99 mmol) and Intermediate **IN-2** (260 mg, 0.99 mmol) were dissolved in ethyl acetate (10 mL), 1-propylphosphonic anhydride (2.5 g, 3.93 mmol, 50% ethyl acetate solution) and pyridine (471 mg, 5.95 mmol) were added, the reaction was carried out for 2 hours at room temperature, and the reaction was complete shown by TLC. The reaction solution was quenched with water and extracted with ethyl acetate, the organic phases were combined, washed with saturated salt water, dried over anhydrous sodium sulfate and concentrated, and the crude product was separated and purified by silica gel column chromatography to obtain the white solid compound **16** (210 mg, yield 50%). The title compound **16-1** (14.0 min) (30 mg, yield 14%) and the title compound **16-2** (RT 18.0 min) (36 mg, yield 17%) were obtained by chiral resolution (NANOMICRO AD-H, 30*250mm, 5um, 30mL/min, EtOH:Hexane=40:60).

### Compound 16-1

LC-MS: m/z=426.1 [M+H]⁺

¹H NMR (400 MHz, DMSO-d₆) δ 10.90 (s, 1H), 8.36-8.00 (m, 4H), 7.01 (s, 1H), 6.82 (s, 1H), 4.04-4.83 (m, 6H), 2.87 (s, 2H), 1.41 (s, 3H), 1.26 (s, 6H). (99.64% purity by HPLC)

### Compound 16-2

LC-MS: m/z=426.1 [M+H]⁺

¹H NMR (400 MHz, DMSO-*d₆*) δ 10.89 (s, 1H), 8.36 (s, 1H), 8.20 (s, 1H), 8.09 (d, *J =* 4.4 Hz, 1H), 7.98 (s, 1H), 6.99 (d, *J* = 4.0 Hz, 1H), 6.81 (s, 1H), 4.07-3.97 (m, 1H), 3.93 (s, 2H), 3.82 (s, 3H), 2.87 (s, 2H), 1.41 (d, *J =* 6.0 Hz, 3H), 1.25 (s, 6H). (99.53% purity by HPLC)

### Embodiment 17

### (S)-N-(5-chloro-4-(5,5-dimethyl-5,6-dihydro-4H-pyrrolo[1,2-b]pyrazol-3-yl)pyridin-2-yl)-2-(6-(2-hydroxypro pyl-2-yl)pyridin-2-yl)propionamide (assumed) 17-1

### (R)-N-(5-chloro-4-(5,5-dimethyl-5,6-dihydro-4H-pyrrolo[1,2-b]pyrazol-3-yl)pyridin-2-yl)-2-(6-(2-hydroxypro pyl-2-yl)pyridin-2-yl)propionamide (assumed) 17-2

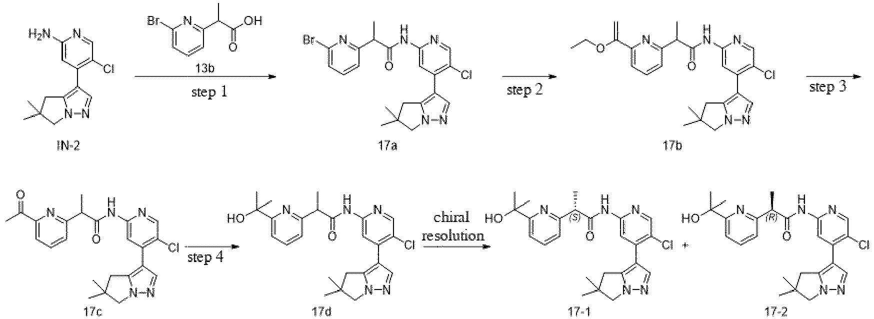

### Step 1:

### 2-(6-bromopyridin-2-yl)-N-(5-chloro-4-(5,5-dimethyl-5,6-dihydro-4H-pyrrolo[1,2-b]pyrazol-3-yl)pyridin-2-yl) propionamide 17a

Compound **13b** (437 mg, 1.90 mmol) and Intermediate **IN-2** (500 mg, 1.90 mmol) were dissolved in ethyl acetate (10 mL), 1-propylphosphonic anhydride (4.86 g, 7.64 mmol, 50% ethyl acetate solution) and pyridine (905 mg, 11.44 mmol) were added at room temperature, the temperature was lifted to 50°C for reaction for 2 hours, and the reaction was complete monitored by TLC. The reaction solution was added with water and extracted with ethyl acetate, the organic phases were combined, washed with water, washed with saturated salt water, dried over anhydrous sodium sulfate and concentrated, and the crude product was purified by silica gel column chromatography to obtain the title compound **17a** (700 mg, yield 52%).

### Step 2:

### N-(5-chloro-4-(5,5-dimethyl-5,6-dihydro-4H-pyrrolo[1,2-b]pyrazol-3-yl)pyridin-2-yl)-2-(6-(1-ethoxyvinyl)pyr idin-2-yl)propionamide 17b

Compound **17a** (430 mg, 0.91 mmol) and tributyl(1-ethoxyvinyl) tin (427 mg, 1.18 mmol) were dissolved in toluene (10 mL), bis-triphenylphosphine palladium (64 mg, 0.09 mmol) was added at room temperature, the temperature was lifted to 100°C for reaction overnight under nitrogen protection, and a small amount of the starting material was remained monitored by TLC. The reaction solution was cooled to room temperature, added with water and extracted with ethyl acetate, the organic phases were combined, washed with water, washed with saturated salt water, dried over anhydrous sodium sulfate and concentrated, and the crude product was purified by silica gel column chromatography to obtain the title compound **17b** (250 mg, yield 59%).

### Step 3:

### 2-(6-acetylpyridin-2-yl)-N-(5-chloro-4-(5,5-dimethyl-5,6-dihydro-4H-pyrrolo[1,2-b]pyrazol-3-yl)pyridin-2-yl) propionamide 17c

Compound **17b** (250 mg, 0.54 mmol) was dissolved in tetrahydrofuran (6 mL), dilute hydrochloric acid (1 mL, 3N) was added, the reaction was carried out for 1 hour at room temperature, and the reaction was complete monitored by TLC. The reaction solution was added with a saturated sodium bicarbonate solution to adjust the alkalinity and extracted with ethyl acetate, and the organic phases were combined, washed with water, washed with saturated salt water, dried over anhydrous sodium sulfate and concentrated to obtain the title compound **17c** (240 mg, crude product), which was directly used for the next step.

### Step 4:

### (S)-N-(5-chloro-4-(5,5-dimethyl-5,6-dihydro-4H-pyrrolo[1,2-b]pyrazol-3-yl)pyridin-2-yl)-2-(6-(2-hydroxypro pyl-2-yl)pyridin-2-yl)propionamide (assumed) 17-1 &

### (R)-N-(5-chloro-4-(5,5-dimethyl-5,6-dihydro-4H-pyrrolo[1,2-b]pyrazol-3-yl)pyridin-2-yl)-2-(6-(2-hydroxypro pyl-2-yl)pyridin-2-yl)propionamide (assumed) 17-2

Compound **17c** (240 mg, crude product) was dissolved in anhydrous tetrahydrofuran (8 mL), it was cooled to 0°C, methylmagnesium bromide (0.37 mL, 1.11 mmol, 3M) was added, the temperature was lifted to room temperature for reaction for 1 hour, and the reaction was complete monitored by TLC. The reaction solution was added with water and extracted with ethyl acetate, the organic phases were combined, washed with water, washed with saturated salt water, dried with anhydrous sodium sulfate and concentrated, and the crude product was purified by silica gel column chromatography to obtain the title compound **17** (55 mg, two-step yield 22%), and chirally resolved (DAICEL AD-H, 30*250mm, 5um, 30mL/min, IPA:Hexane=20:80) to obtain the title compound **17-1** (RT 31.26 min) (3.9 mg, yield 7.8%) and the title compound **17-2** (RT 38.94 min) (6.5 mg, yield 13%).

### Compound 17-1

LC-MS: m/z=454.2 [M+H]⁺

¹H NMR(400 MHz, DMSO-*d₆*) δ 10.76 (s, 1H), 8.35 (s, 1H), 8.23 (s, 1H), 8.00 (s, 1H), 7.75 (t, *J* = 7.6 Hz, 1H), 7.52 (d, *J* = 7.6 Hz, 1H), 7.27 (d, *J* = 7.6 Hz, 1H), 5.24 (s, 1H), 4.16 (q, *J* = 6.8 Hz, 1H), 3.94 (s, 2H), 2.88 (s, 2H), 1.49 (d, *J* = 7.2 Hz, 3H), 1.42 (d, *J* = 2.0 Hz, 6H), 1.26 (s, 6H). (99.96% purity by HPLC)

### Compound 17-2

LC-MS: m/z=454.2 [M+H]⁺

¹H NMR(400 MHz, DMSO-d₆) δ 10.75 (s, 1H), 8.35 (s, 1H), 8.23 (s, 1H), 8.00 (s, 1H), 7.75 (s, 1H), 7.54 (s, 1H), 7.28 (s, 1H), 5.24 (s, 1H), 4.17 (s, 1H), 3.94 (s, 2H), 2.88 (s, 2H), 1.49-1.43 (m, 9H), 1.26 (s, 6H).

(99.99% purity by HPLC)

### Embodiment 18

### (S)-N-(5-chloro-4-(5,5-dimethyl-5,6-dihydro-4H-pyrrolo[1,2-b]pyrazol-3-yl)pyridin-2-yl)-2-(2-(2-hydroxypro pyl-2-yl)pyridin-4-yl)propionamide (assumed) 18-1

### (R)-N-(5-chloro-4-(5,5-dimethyl-5,6-dihydro-4H-pyrrolo[1,2-b]pyrazol-3-yl)pyridin-2-yl)-2-(2-(2-hydroxypro pyl-2-yl)pyridin-4-yl)propionamide (assumed) 18-2

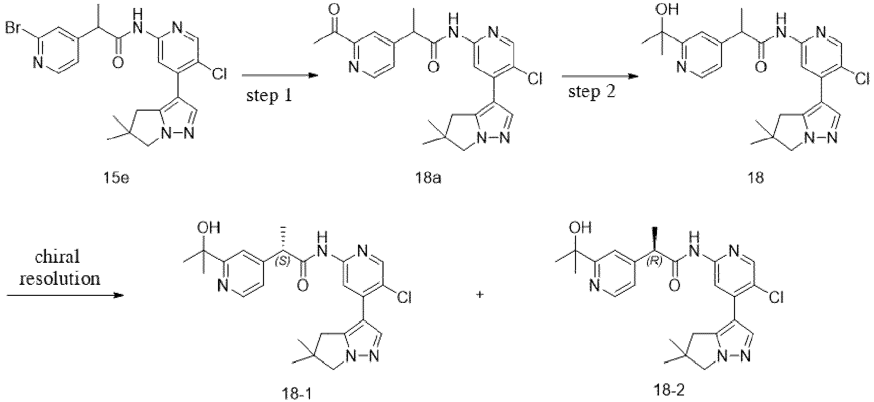

### Step 1: 2-(2-acetylpyridin-4-yl)-N-(5-chloro-4-(5,5-dimethyl-5,6-dihydro-4H-pyrrolo[1,2-b]pyrazol-3-yl)pyridin-2-yl) propionamide 18a

Compound **15e** (850 mg, 1.79 mmol) and tributyl (1-ethoxyethylene) tin (840 mg, 2.33 mmol) were dissolved in toluene (15 mL), Pd(PPh₃)₂Cl₂ (126 mg, 0.18 mmol) was added at room temperature, the temperature was lifted to 100°C for reaction overnight under nitrogen protection, and the reaction was complete shown by TLC. The reaction solution was cooled to room temperature, added with water and extracted with ethyl acetate, the organic phases were combined, dried over anhydrous sodium sulfate and concentrated, the residue was added with tetrahydrofuran to dissolve, added with dilute hydrochloric acid (2 mL, 3N), it was stirred at room temperature for 1 hour, and the reaction was complete shown by TLC. The reaction solution was extracted with ethyl acetate, the organic phases were combined, washed with water, washed with saturated salt water, dried over anhydrous sodium sulfate and concentrated, and the crude product was purified by silica gel column chromatography to obtain the title compound **18a** (493 mg, yield 62.9%).

### Step 2: (S)-N-(5-chloro-4-(5,5-dimethyl-5,6-dihydro-4H-pyrrolo[1,2-b]pyrazol-3-yl)pyridin-2-yl)-2-(2-(2-hydroxypro pyl-2-yl)pyridin-4-yl)propionamide (assumed) 18-1 &

### (R)-N-(5-chloro-4-(5,5-dimethyl-5,6-dihydro-4H-pyrrolo[1,2-b]pyrazol-3-yl)pyridin-2-yl)-2-(2-(2-hydroxypro pyl-2-yl)pyridin-4-yl)propionamide (assumed) 18-2

Compound **18a** (493 mg, 1.13 mmol) was dissolved in anhydrous tetrahydrofuran (10 mL), it was cooled to 0°C, methylmagnesium bromide (0.94 mL, 2.82 mmol, 3M tetrahydrofuran solution) was added, the temperature was lifted to room temperature for reaction for 1 hour, and part of the starting material was remained shown by TLC. The reaction solution was added with water and extracted with ethyl acetate, the organic phases were combined, washed with water, washed with saturated salt water, dried with anhydrous sodium sulfate and concentrated, and the crude product was purified by silica gel column chromatography to obtain the title compound **18** (120 mg, yield 23%). The title compound **18-1** (RT 8.86min) (10.0 mg, yield1.96%) and the title compound **18-2** (RT 12.65 min) (28 mg, yield 5.48%) were obtained by chiral resolution (DAICEL AD-H, 20*250mm, 5um, 30mL/min, IPA:Hexane=40:60).

### Compound 18-1

LC-MS: m/z = 454.2 [M+H]⁺

¹H NMR (400 MHz, DMSO-d₆) δ 10.94 (s, 1H), 8.42 (d, J= 4.8 Hz, 1H), 8.36 (s, 1H), 8.21 (s, 1H), 7.98 (s, 1H), 7.71 (s, 1H), 7.24 (dd, J= 4.8, 1.6 Hz, 1H), 5.23 (s, 1H), 4.08 (q, *J* = 7.2 Hz, 1H), 3.93 (s, 2H), 2.87 (s, 2H), 1.44-1.41 (m, 9H), 1.25 (d, *J* = 8.0 Hz, 6H). (97.9% purity by HPLC)

### Compound 18-2

LC-MS: m/z=454.2 [M+H]⁺

¹H NMR (400 MHz, DMSO-d₆) δ 10.94 (s, 1H), 8.42 (d, *J* = 4.8 Hz, 1H), 8.36 (s, 1H), 8.21 (s, 1H), 7.98 (s, 1H), 7.71 (s, 1H), 7.24 (dd, *J* = 3.6, 1.2 Hz, 1H), 5.23 (s, 1H), 4.08 (q, *J* = 7.2 Hz, 1H), 3.93 (s, 2H), 2.87 (s, 2H), 1.44-1.41 (m, 9H), 1.25 (s, 6H). (99.9% purity by HPLC)

### Embodiment 19

### (S)-2-([2,3'-bipyridine]-5-yl)-N-(5-chloro-4-(5,5-dimethyl-5,6-dihydro-4H-pyrrolo[1,2-b]pyrazol-3-yl)pyridin-2-yl)propionamide (assumed) 19-1

### (R)-2-([2,3'-bipyridine]-5-yl)-N-(5-chloro-4-(5,5-dimethyl-5,6-dihydro-4H-pyrrolo[1,2-b]pyrazol-3-yl)pyridin-2-yl)propionamide (assumed) 19-2

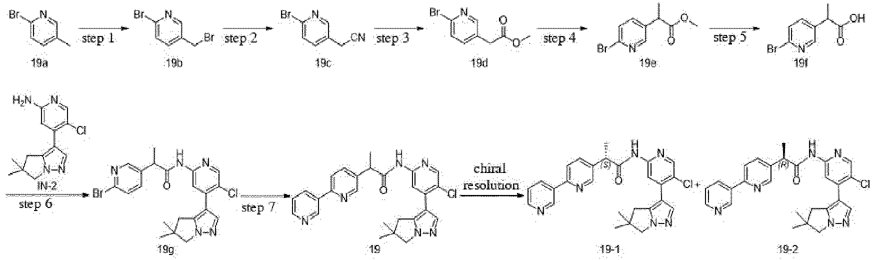

### Step 1: 2-bromo-5-(bromomethyl)pyridine 19b

2-bromine-5-methylpyridine **19a** (10.0 g, 58.13 mmol) was dissolved in carbon tetrachloride (100 mL), N-bromosuccinimide (10.9 g, 61.24 mmol) and dibenzoyl peroxide (200 mg, 0.83 mmol) were added at room temperature, the temperature was lifted to 80°C for reaction overnight. The reaction solution was cooled to room temperature and filtered, the filtrate was concentrated, and the crude product was purified by silica gel column chromatography to obtain the title compound **19b** (12g, yield 82.2%).

### Step 2: 2-(6-bromopyridin-3-yl)-acetonitrile 19c

Trimethylcyanosilane (7.1 g, 71.57 mmol) was dissolved in acetonitrile (200 mL), tetrabutylammonium fluoride (18.7 g, 71.52 mmol) was added at room temperature, it was stirred for 10 minutes, and an acetonitrile (100 mL) solution of Compound **19b** (12 g, 47.82 mmol) was added. The reaction was carried out at room temperature for 2 hours, and the reaction was complete shown by TLC. The reaction solution was added with a saturated sodium bicarbonate solution and extracted with ethyl acetate, the organic phases were combined, washed with saturated salt water, dried over anhydrous sodium sulfate and concentrated, and the crude product was purified by silica gel column chromatography to obtain the title compound **19c** (4.58 g, yield 48.6%).

### Step 3: 2-(6-bromopyridin-3-yl)acetic acid methyl ester 19d

Compound **19c** (4.58 g, 23.24 mmol) was dissolved in methanol (50 mL), thionyl chloride (6.9 g, 58.00 mmol) was added dropwise, the reaction was carried out overnight at room temperature, and the reaction was complete shown by TLC. The reaction solution was concentrated and purified by silica gel column chromatography to obtain the title compound **19d** (4.8 g, yield 89.8%).

### Step 4: 2-(6-bromopyridin-3-yl)propanoic acid methyl ester 19e

Compound **19d** (4.12 g, 17.91 mmol) was dissolved in tetrahydrofuran (50 mL), potassium tert-butoxide (2.42 g, 21.57 mmol) was added at room temperature, it was cooled to 0°C, iodomethane (2.43 g, 17.12 mmol) was added dropwise, after addition the reaction was carried out at room temperature for 1 hour, and the reaction was complete shown by TLC. The reaction solution was quenched with water and extracted with ethyl acetate, the organic phases were combined, washed with saturated salt water, dried over anhydrous sodium sulfate and concentrated, and the crude product was purified by silica gel column chromatography to obtain the title compound **19e** (2.2 g, yield 50.3%).

### Step 5: 2-(6-bromopyridin-3-yl)propionic acid 19f

Compound **19e** (1.3 g, 5.33 mmol) was dissolved in methanol/water (20 mL/10 mL), sodium hydroxide solid (450 mg, 11.25 mmol) was added, the reaction was carried out for 2 hours at room temperature, and the complete reaction of the starting material was monitored by TLC. The reaction solution was quenched with water, the organic phase was discarded, the aqueous phase was adjusted to pH about 3 with dilute hydrochloric acid (1N) and extracted with ethyl acetate, and the organic phases were combined, washed with saturated salt water, dried over anhydrous sodium sulfate and concentrated to obtain the white solid title compound **19f** (1.17 g, crude product), which was directly used for the next step.

### Step 6: 2-(6-bromopyridin-3-y1)-N-(5-chloro-4-(5,5-dimethyl-5,6-dihydro-4H-pyrrolo[1,2-b]pyrazol-3-yl)pyridin-2-yl) propionamide 19g

Compound **19f** (500 mg, crude product) was dissolved in ethyl acetate (10 mL), Intermediate **IN-2** (610 mg, 2.31 mmol), 1-propylphosphonic anhydride (5.89 g, 9.26 mmol, 50% ethyl acetate solution) and pyridine (1.1 g, 13.91 mmol) were added, the reaction was carried out at room temperature for 2 hours, and the complete reaction of the starting material was monitored by TLC. The reaction solution was quenched with water, the liquid was separated, the aqueous phase was extracted with ethyl acetate, the organic phases were combined, washed with saturated salt water, dried over anhydrous sodium sulfate, and the crude product was purified by silica gel column chromatography to obtain the title compound **19g** (670 mg, two-step yield 62.0%).

### Step 7: (S)-2-([2,3'-bipyridine]-5-yl)-N-(5-chloro-4-(5,5-dimethyl-5,6-dihydro-4H-pyrrolo[1,2-b]pyrazol-3-yl)pyridin-2-yl)propionamide (assumed) 19-1 &

### (R)-2-([2,3'-bipyridine]-5-yl)-N-(5-chloro-4-(5,5-dimethyl-5,6-dihydro-4H-pyrrolo[1,2-b]pyrazol-3-yl)pyridin-2-yl)propionamide (assumed) 19-2

Under nitrogen protection, Compound **19g** (100 mg, 0.21 mmol), 3-pyridine boronic acid (31 mg, 0.25 mmol), Pd(dppf)Cl₂ dichloromethane complex (17 mg, 0.02 mmol) and sodium carbonate (45 mg, 0.42 mmol) were dissolved in a mixed solution of 1,4-dioxane (5 mL)/water (1 mL), it was replaced with nitrogen three times, it was heated to 100°C and stirred for 3 hours, and the reaction was complete monitored by TLC. The reaction solution was cooled to room temperature, quenched with water and extracted with ethyl acetate, the organic phases were combined, washed with water, washed with saturated salt water, dried over anhydrous sodium sulfate and concentrated, and the crude product was purified by Prep-TLC to obtain the title compound **19** (71 mg, yield 71.5%). The compound **19-1** (RT 28.48 min) (25 mg, yield 25.2%) and the compound **19-2** (RT 46.28 min) (28 mg, yield 28.2%) were obtained by chiral resolution (DAICEL AD-H, 20*250mm, 5um, 30mL/min, IPA:Hexane=40:60).

### Compound 19-1

LC-MS: m/z=473.2[M+H]⁺

¹H NMR (400 MHz, DMSO-d₆) δ 10.96 (s, 1H), 9.23 (d, *J* = 1.6 Hz, 1H), 8.73 (d, *J* = 2.0 Hz, 1H), 8.62 (dd, *J* = 4.8, 1.6 Hz, 1H), 8.41-8.38 (m, 1H), 8.36 (s, 1H), 8.22 (s, 1H), 8.04 (d, *J* = 8.4 Hz, 1H), 7.98 (s, 1H), 7.93 (dd, J= 8.4, 2.4 Hz, 1H), 7.51 (dd, J= 7.2, 4.8 Hz, 1H), 4.16 (q, J= 7.2 Hz, 1H), 3.93 (s, 2H), 2.88 (s, 2H), 1.51 (d, *J* = 7.2 Hz, 3H), 1.26 (s, 6H). (99.81% purity by HPLC)

### Compound 19-2

LC-MS: m/z=473.2[M+H]⁺

¹H NMR (400 MHz, DMSO-d₆) δ 10.96 (s, 1H), 9.23 (d, *J* = 1.2 Hz, 1H), 8.73 (d, *J* = 2.0 Hz, 1H), 8.62 (dd, *J* = 3.6, 1.2 Hz, 1H), 8.41-8.39 (m, 1H), 8.36 (s, 1H), 8.22 (s, 1H), 8.04 (d, *J* = 8.0 Hz, 1H), 7.98 (s, 1H), 7.93 (dd, *J* = 8.0, 2.0 Hz, 1H), 7.52 (dd, *J* = 7.6, 5.2 Hz, 1H), 4.16 (q, *J* = 7.2Hz, 1H), 3.93 (s, 2H), 2.88 (s, 2H), 1.51 (d, *J* = 7.2 Hz, 3H), 1.26 (s, 6H). (99.89% purity by HPLC)

### Embodiment 20

### (S)-N-((5-(1-((5-chloro-4-(5,5-dimethyl-5,6-dihydro-4H-pyrrolo[1,2-b]pyrazol-3-yl)pyridin-2-yl)amino)-1-oxo propane-2-yl)-[2,3'-bipyridine]-6'-yl)methyl)acrylamide (assumed) 20-1

### (R)-N-([5-(1-((5-chloro-4-(5,5-dimethyl-5,6-dihydro-4H-pyrrolo[1,2-b]pyrazol-3-yl)pyridin-2-yl)amino)-1-oxo propane-2-yl)-[2,3'-bipyridine]-6'-yl)methyl)acrylamide (assumed) 20-2

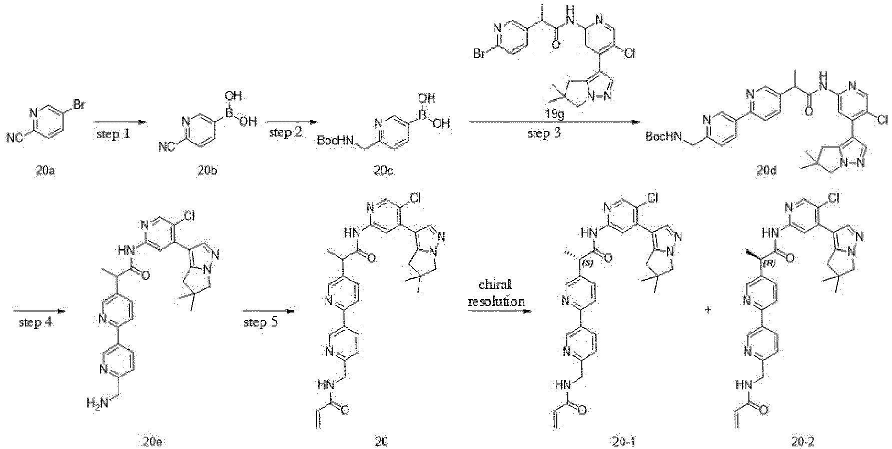

### Step 1: 2-cyanopyridine-5-boronic acid 20b

5-bromine-2-cyanopyridine **20a** (5.0 g, 27.32 mmol) and bis(pinacolato)diboron (13.9 g, 54.74 mmol) were dispersed in 1,4-dioxane (50 mL), potassium acetate (5.4 g, 55.02 mmol) and Pd(dppf)Cl₂ (500 mg, 0.68 mmol) were added at room temperature, the temperature was lifted to 100°C for reaction for 2 hours under nitrogen protection, and the reaction was complete shown by TLC. The reaction solution was cooled to room temperature and quenched with water, the liquid was separated and extracted with aqueous phase ethyl acetate, the organic phases were combined, washed with saturated salt water, dried with anhydrous sodium sulfate and concentrated, and the crude product was purified by silica gel column chromatography to obtain the title compound **20b** (3.5 g, yield 87%).

### Step 2: (6-((tert-butoxycarbonyl)amino)methyl)pyridin-3-yl)boronic acid 20c

Compound **20b** (3.5 g, 23.66 mmol) was dissolved in methanol (40 mL), di-tert-butyl dicarbonate (6.7 g, 30.70 mmol) and palladium/carbon (350 mg, 10%) were added, the reaction was carried out for 2 hours at room temperature in hydrogen atmosphere, and the reaction was complete shown by TLC. The reaction liquid was filtered with diatomite, and the filtrate was concentrated to obtain the title compound **20c** (4.8 g, crude product), which was directly used for the next step.

### Step 3: (5-(1-((5-chloro-4-(5,5-dimethyl-5,6-dihydro-4H-pyrrolo[1,2-b]pyrazol-3-yl)pyridin-2-yl)amino)-1-oxopropan e-2-yl)-[2,3'-bipyridine]-6'-yl)methyl)carbamic acid tert-butyl ester 20d

Compound **19g** (450 mg, 0.95 mmol) was dissolved in a mixed solvent of 1,4-dioxane (20 mL) and water (4 mL), Compound **20c** (335 mg, crude product), Pd(dppf)Cl₂ (69 mg, 0.09 mmol) and sodium carbonate (200 mg, 1.89 mmol) were added at room temperature, it was replaced with nitrogen three times, it was heated to 100°C for reaction for 5 hours, and the complete reaction of the starting material was monitored by TLC. The reaction solution was quenched with water, the liquid was separated, the aqueous phase was extracted with ethyl acetate, the organic phases were combined, washed with saturated salt water and dried over anhydrous sodium sulfate, and the crude product was purified by silica gel column chromatography to obtain the light yellow oil title compound **20d** (128 mg, yield 22%).

### Step 4: 2-(6'-(aminomethyl)-[2,3'-bipyridine]-5-yl)-N-(5-chloro-4-(5,5-dimethyl-5,6-dihydro-4H-pyrrolo[1,2-b]pyraz ol-3-yl)pyridin-2-yl)propionamide 20e

Compound **20d** (128 mg, 0.21 mmol) was dissolved in dichloromethane (5 mL), trifluoroacetic acid (1 mL) was added to react for 1 hour at room temperature, and the complete reaction of the starting material was monitored by TLC. The reaction solution was added with water, adjusted to pH=8 or so with a saturated sodium carbonate solution and extracted with ethyl acetate, the organic phases were combined, washed with saturated salt water, dried over anhydrous sodium sulfate and concentrated to obtain the title compound **20e** (108 mg, crude product), which was directly used for the next step.

### Step 5: (S)-N-((5-(1-((5-chloro-4-(5,5-dimethyl-5,6-dihydro-4H-pyrrolo[1,2-b]pyrazol-3-yl)pyridin-2-yl)amino)-1-oxo propane-2-yl)-[2,3'-bipyridine]-6'-yl)methyl)acrylamide (assumed) 20-1 &

### (R)-N-((5-(1-((5-chloro-4-(5,5-dimethyl-5,6-dihydro-4H-pyrrolo[1,2-b]pyrazol-3-yl)pyridin-2-yl)amino)-1-oxo propane-2-yl)-[2,3'-bipyridine]-6'-yl)methyl)acrylamide (assumed) 20-2

Compound **20e** (108 mg, crude product) was dissolved in a mixed system of dichloromethane (2 mL) and a saturated sodium bicarbonate aqueous solution (2 mL), it was cooled to 0°C, a dichloromethane (5 mL) solution of acryloyl chloride (14.4 mg, 0.16 mmol) was added dropwise, the reaction was carried out for 20 minutes at room temperature, and the complete reaction of the starting materials was monitored by TLC. The reaction solution was added with water, the liquid was separated, the aqueous phase was extracted with dichloromethane, the organic phases were combined, dried over anhydrous sodium sulfate and concentrated, and the crude product was separated by Prep-TLC to obtain the title compound **20** (60 mg, two-step yield 51%). Compound **20-1** (RT 16.57 min) (8.0 mg, yield 6.9%) and Compound **20-2** (RT 22.46 min) (11 mg, yield 9.4%) were obtained by chiral resolution (NANOMICRO AD-H, 30*250mm, 5um, 30mL/min, IPA:Hexane=40:60).

### Compound 20-1

LC-MS: m/z=556.3 [M+H]⁺

¹H NMR (400 MHz, DMSO-*d₆*) δ 10.95 (s, 1H), 9.16 (d, *J* = 1.6 Hz, 1H), 8.78 (t, *J* = 5.8 Hz, 1H), 8.72 (d, *J* = 2.0 Hz, 1H), 8.41 (dd, *J* = 8.0, 2.0 Hz, 1H), 8.36 (s, 1H), 8.22 (s, 1H), 8.02 (d, *J* = 8.0 Hz, 1H), 7.98 (s, 1H), 7.93 (dd, *J* = 8.4, 2.0 Hz, 1H), 7.42 (d, *J* = 8.4 Hz, 1H), 6.38 (dd, *J* = 10.4, 10.0 Hz, 1H), 6.16 (dd, *J* = 2.0, 2.4 Hz, 1H), 5.66 (dd, *J* = 2.0, 2.4 Hz, 1H), 4.51 (d, *J* = 5.6 Hz, 2H), 4.17 (q, *J* = 7.2 Hz, 1H), 3.93 (s, 2H), 2.88 (s, 2H), 1.51 (d, *J* = 7.2 Hz, 3H), 1.26 (s, 6H). (99.21% Purity by HPLC)

### Compound 20-2

LC-MS: m/z=556.3 [M+H]⁺

¹H NMR (400 MHz, DMSO-d₆) δ 10.95 (s, 1H), 9.17 (d, *J* = 1.6 Hz, 1H), 8.77 (t, *J* = 6.0 Hz, 1H), 8.72 (d, *J* = 2.0 Hz, 1H), 8.40 (dd, *J* = 2.0, 2.4 Hz, 1H), 8.36 (s, 1H), 8.22 (s, 1H), 8.03 (d, *J* = 8.2 Hz, 1H), 7.98 (s, 1H),

7.94 (dd, *J* = 2.0, 2.4 Hz, 1H), 7.42 (d, *J* = 8.4 Hz, 1H), 6.35 (dd, *J* = 10.0, 10.4 Hz, 1H), 6.16 (dd, *J* = 2.0, 2.2 Hz, 1H), 5.66 (dd, J= 2.0, 2.2 Hz, 1H), 4.51 (d, J= 5.6 Hz, 2H), 4.15 (q, J= 6.8 Hz, 1H), 3.93(s, 2H), 2.88 (s, 2H), 1.51 (d, *J* = 7.2 Hz, 3H), 1.26 (s, 6H). (99.93% Purity by HPLC)

### Embodiment 21

### (S)-N-(4-(5-cyano-2,2-dimethyl-2,3-dihydro-1H-pyrrolizin-7-yl)pyridin-2-yl)-2-(6-(2-hydroxypropan-2-yl)pyri din-2-yl)propionamide (assumed) 21-1

### (R)-N-(4-(5-cyano-2,2-dimethyl-2,3-dihydro-1H-pyrrolizin-7-yl)pyridin-2-yl)-2-(6-(2-hydroxypropan-2-yl)pyr idin-2-yl)propionamide (assumed) 21-2

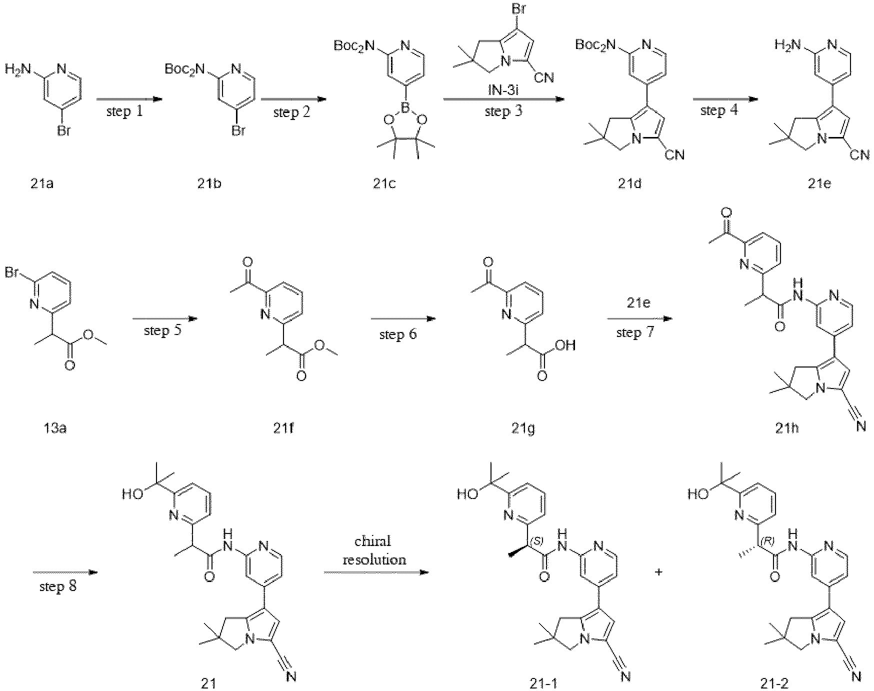

### Step 1: (4-bromopyridin-2-yl)iminodicarbonic acid di-tert-butyl ester 21b

2-amino-4-bromopyridine **21a** (2.0 g, 11.56 mmol) and di-tert-butyl dicarbonate (6.5 g, 29.78 mmol) were dissolved in dichloromethane (20 mL), 4-dimethylaminopyridine (122 mg, 0.1 mmol) was added, the reaction was carried out overnight at room temperature, and the reaction was complete shown by TLC. The reaction solution was added with water and extracted with dichloromethane, the organic phases were combined, washed with saturated salt water, dried over anhydrous sodium sulfate and concentrated, and the crude product was chromatographed on silica gel column to obtain the white solid title compound **21b** (3.5 g, yield 81%).

### Step 2: 4-(4,4,5,5-tetramethyl-1,3,2-dioxaborolan-2-yl)pyridin-2-yl)iminodicarbonic acid di-tert-butyl ester 21c

Under nitrogen protection, Compound **21b** (3.5 g, 9.38 mmol) and bis(pinacolato)diboron (3.57 g, 14.06 mmol) were dissolved in 1,4-dioxane (50 mL), potassium acetate (2.30 g, 23.44 mmol) and Pd(dppf)Cl₂ (343 mg, 0.47 mmol) were added at room temperature, the temperature was lifted to 90°C, it was stirred overnight, and the reaction was complete shown by TLC. The reaction solution was cooled to room temperature, added with water and extracted with ethyl acetate, the organic phases were combined, washed with saturated salt water, dried with anhydrous sodium sulfate and concentrated, and the crude product was purified by silica gel column chromatography to obtain the white solid title compound **21c** (2.7 g, yield 68%).

### Step 3: (4-(5-cyano-2,2-dimethyl-2,3-dihydro-1H-pyrrolizin-7-yl)pyridin-2-yl)iminodicarbonic acid di-tert-butyl ester 21d

Under nitrogen protection, Compound **21c** (500 mg, 1.19 mmol) and Intermediate **IN-3i** (340 mg, 1.42 mmol) were dissolved in 1,4-dioxane (6 mL) and water (2 mL), sodium carbonate (254 mg, 2.40 mmol) and Pd(dppf)Cl₂ (88 mg, 0.12 mmol) were added at room temperature, the temperatyre was lifted to 90°C for reaction for 3 hours, and the reaction was complete shown by TLC. The reaction solution was cooled to room temperature, added with water and extracted with ethyl acetate, the organic phases were combined, washed with saturated salt water, dried over anhydrous sodium sulfate and concentrated, and the crude product was purified by silica gel column chromatography to obtain the white solid title compound **21d** (380 mg, yield 70%).

### Step 4: 7-(2-aminopyridin-4-yl)-2,2-dimethyl-2,3-dihydro-1H-pyrrolizine-5-carbonitrile 21e

Compound **21d** (380 mg, 1.08 mmol) was dissolved in dichloromethane (9 mL), trifluoroacetic acid (3 mL) was added to react for 30 minutes at room temperature, and the reaction was complete shown by TLC. The reaction solution was quenched with a saturated aqueous sodium bicarbonate solution, extracted with dichloromethane, the organic phases were combined, washed with saturated salt water, dried over anhydrous sodium sulfate and concentrated, and the crude product was purified by silica gel column chromatography to obtain the white solid title compound **21e** (220 mg, yield 79%).

### Step 5: 2-(6-acetylpyridin-2-yl)propanoic acid methyl ester 21f

Under nitrogen protection, Compound **13a** (3.36 g, 13.88 mmol) was dissolved in toluene (40 mL), tributyl (1-ethoxyalkene) tin (6.52 g, 18.04 mmol) and Pd(PPh₃)₂Cl₂ (974 mg, 1.39 mmol) were added at room temperature, the temperature was lifted to 100°C for reaction overnight, and the reaction was complete shown by TLC. The reaction solution was cooled to room temperature, diluted with water and extracted with ethyl acetate, the organic phases were combined, dried over anhydrous sodium sulfate and concentrated, the residue was dissolved with tetrahydrofuran, added with hydrochloric acid (3 mL, 3N), ut was stirred at room temperature for 1 hour, and the reaction was complete shown by TLC. The reaction solution was extracted with ethyl acetate, the organic phases were combined, washed with water, washed with saturated salt, dried over anhydrous sodium sulfate and concentrated, and the crude product was purified by silica gel column chromatography to obtain the title compound **21f** (2.27 g, yield 79.6%).

### Step 6: 2-(6-acetylpyridin-2-yl)propionic acid 21g

Compound **21f** (2.27 g, 10.93 mmol) was dissolved in ethanol (20 mL), a water (5 mL) solution of sodium hydroxide (1.31 g, 32.8 mmol) was added, the reaction was carried out for 1 hour at room temperature, and the reaction was complete shown by TLC. The reaction solution was diluted with water and extracted with ethyl acetate, the organic phase was discarded, the aqueous phase was adjusted to pH=3 with dilute hydrochloric acid (3N) and extracted with dichloromethane, and the organic phases were combined, washed with water, washed with saturated salt water, dried over anhydrous sodium sulfate and concentrated to obtain the title compound **21g** (1.65 g, crude product), which was directly used for the next step.

### Step 7: 2-(6-acetylpyridin-2-yl)-N-(4-(5-cyano-2,2-dimethyl-2,3-dihydro-1H-pyrrolizin-7-yl)pyridin-2-yl)propionamid e 21h

Under nitrogen protection, Compound **21g** (340 mg, crude product) was dissolved in ethyl acetate (10 mL), Compound **21e** (260 mg, 1.35 mmol), 1-propylphosphonic anhydride (3.44 g, 5.4 mmol, 50% ethyl acetate solution) and triethylamine (818 mg, 8.1 mmol) were added at room temperature, the temperature was lifted to 60°C for reaction for 2 hours, and the reaction was complete shown by TLC. The reaction solution was diluted with water and extracted with ethyl acetate, the organic phases were combined, washed with saturated salt water, dried over anhydrous sodium sulfate and concentrated, and the crude product was purified by silica gel column chromatography to obtain the title compound **21h** (224 mg, two-step yield 23%).

### Step 8: (S)-N-(4-(5-cyano-2,2-dimethyl-2,3-dihydro-1H-pyrrolizin-7-yl)pyridin-2-yl)-2-(6-(2-hydroxypropan-2-yl)pyri din-2-yl)propionamide (assumed) 21-1 &

### (R)-N-(4-(5-cyano-2,2-dimethyl-2,3-dihydro-1H-pyrrolizin-7-yl)pyridin-2-yl)-2-(6-(2-hydroxypropan-2-yl)pyr idin-2-yl)propionamide (assumed) 21-2

Under nitrogen protection, Compound **21h** (351 mg, 0.82 mmol) was dissolved in anhydrous tetrahydrofuran (10 mL), the temperature was lowered to 0°C, methylmagnesium bromide (0.68 mL, 2.04 mmol, 3N tetrahydrofuran solution) was added dropwise, the reaction was carried out for 1 hour at room temperature, and the reaction was complete shown by TLC. The reaction solution was diluted with water and extracted with ethyl acetate, the organic phases were combined, washed with saturated salt water, dried over anhydrous sodium sulfate and concentrated, and the crude product was purified by silica gel column chromatography to obtain the title compound **21** (65 mg, yield 17.9%). Compound **21-1** (RT 9.96 min) (16 mg, yield 24.6%) and Compound **21-2** (RT 21.64 min) (20 mg, yield 30.7%) were obtained by chiral resolution (DAICEL AD-H, 20*250mm, 5um, 30mL/min, IPA:Hexane=40:60).

### Compound 21-1

LC-MS: m/z=444.3 [M+H]⁺

¹H NMR (400 MHz, DMSO-d₆) δ 10.59 (s, 1H), 8.22 (d, *J* = 5.2 Hz, 1H), 8.20 (s, 1H), 7.76 (t, *J* = 8.0 Hz, 1H), 7.53 (d, *J* = 7.6 Hz, 1H), 7.50 (s, 1H), 7.29 (d, *J* = 7.6 Hz, 1H), 7.23 (dd, *J* = 5.2, 1.2 Hz, 1H), 5.57-4.99 (m, 1H), 4.15 (q, *J* = 6.8 Hz, 1H), 3.90 (s, 2H), 2.96 (s, 2H), 1.50 (d, *J* = 7.2 Hz, 3H), 1.44 (s, 6H), 1.26 (d, *J* = 2.4 Hz, 6H). (96.98% purity by HPLC)

### Compound 21-2

LC-MS: m/z=444.3 [M+H]⁺

¹H NMR (400 MHz, DMSO-d₆) δ 10.59 (s, 1H), 8.22 (d, *J* = 5.2 Hz, 1H), 8.20 (s, 1H), 7.76 (t, *J* = 8.0 Hz, 1H), 7.53 (d, *J* = 7.6 Hz, 1H), 7.50 (s, 1H), 7.28 (d, *J* = 7.6 Hz, 1H), 7.23 (d, *J* = 4.0 Hz, 1H), 5.57-4.99 (m, 1H), 4.15 (q, *J* = 6.8 Hz, 1H), 3.90 (s, 2H), 2.94 (s, 2H), 1.50 (d, *J* = 7.2 Hz, 3H), 1.44 (s, 6H), 1.26 (d, *J* = 2.4 Hz, 6H). (98.44% purity by HPLC)

### Embodiment 22

### (S)-2-([2,3'-bipyridine]-5-yl)-N-(5-chloro-4-(6,6-dimethyl-6,7-dihydro-5H-pyrrolo[1,2-a]imidazol-3-yl)pyridin -2-yl)propionamide (assumed) 22-1

### (R)-2-([2,3'-bipyridine]-5-yl)-N-(5-chloro-4-(6,6-dimethyl-6,7-dihydro-5H-pyrrolo[1,2-a]imidazol-3-yl)pyridi n-2-yl)propionamide (assumed) 22-2

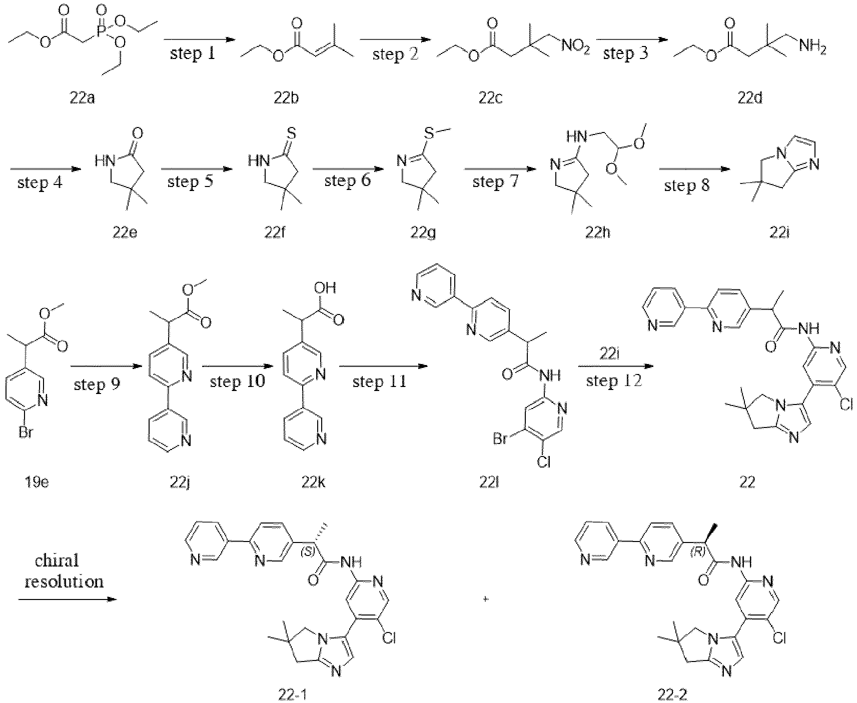

### Step 1: ethyl 3-methyl-2-butenoate 22b

Sodium hydride (8.2 g, 0.21 mol, 60%) was dissolved in tetrahydrofuran (100 mL), it was cooled to 0°C, triethyl phosphonoacetate **22a** (50.0 g, 0.22 mol) was added dropwise, it was stirred for 20 minutes, acetone (10.0 g, 0.17 mol) was added, it was stirred at room temperature for 3 hours, and the reaction was complete shown by TLC. The reaction solution was quenched with water and extracted with ethyl acetate, the organic phases were combined, washed with saturated salt water, dried over anhydrous sodium sulfate and concentrated, and the crude product was purified by silica gel column chromatography to obtain the title compound **22b** (13.0 g, yield 59%).

¹H NMR (400 MHz, CDCl₃) δ 5.67 (s, 1H), 4.14 (q, *J* = 7.2 Hz, 2H), 2.17 (s, 3H), 1.27 (s, 6H).

### Step 2: 3,3-dimethyl-4-nitrobutyric acid ethyl ester 22c

Compound **22b** (13.0 g, 0.10 mol) and nitromethane (52.0 g, 0.85 mol) were dissolved in acetonitrile (130 mL), 1,8-diazabicyclo(5.4.0)undec-7-ene was added at room temperature, the temperature was lifted to 60°C for reaction overnight. The reaction solution was concentrated, diluted with dilute hydrochloric acid (2N) and extracted twice with ethyl acetate, the organic phases were combined, washed with saturated salt water, dried with anhydrous sodium sulfate and concentrated, and the crude product was purified by silica gel column chromatography to obtain the title compound **22c** (8.3 g, yield 44%).

¹H NMR (400 MHz, CDCl₃) δ 4.54 (s, 2H),4.15 (q, *J* = 7.2 Hz, 2H), 2.45 (s, 2H), 1.27 (t, *J* = 7.2 Hz, 3H), 1.17 (s, 6H).

### Step 3: 4-amino-3,3-dimethylbutyric acid ethyl ester 22d

Compound **22c** (8.3 g, 43.87 mmol) was dissolved in methanol (100 mL), palladium/carbon (1.0 g, 10%) was added, the temperature was lifted to 40°C and it was stirred overnight under hydrogen atmosphere. The reaction solution was filtered with diatomite, and the filtrate was concentrated to obtain the title compound **22d** (5.6 g, crude product), which was directly used for the next step.

### Step 4: 4,4-dimethylpyrrolidin-2-one 22e

Compound **22d** (1.0 g, crude product) was dissolved in methanol (10 mL), triethylamine (3.18 g, 31.43 mmol) was added at room temperature, and the temperature was lifted to 80°C for reaction for 2 days. The reaction solution was concentrated, diluted with water and extracted with ethyl acetate, the organic phases were combined, washed with saturated salt water, dried over anhydrous sodium sulfate and concentrated, and the crude product was purified by silica gel column chromatography to obtain the title compound **22e** (300 mg, two-step yield 34%).

### Step 5: 4,4-dimethylpyrrolidin-2-thione 22f

Compound **22e** (300 mg, 2.65 mmol) was dissolved in toluene (10 mL), Lawesson reagent (644 mg, 1.59 mmol) was added at room temperature, and it was heated to 100°C for reaction for 2 hours. The reaction solution was cooled to room temperature,directly mixed with silica gel and purified by silica gel column chromatography to obtain the title compound **22f** (120 mg, yield 35%).

¹H NMR (400 MHz, CDCl₃) δ 8.26 (s, 1H), 3.37 (s, 2H), 2.70 (s, 2H), 1.19 (s, 6H).

### Step 6: 3,3-dimethyl-5-(methylthio)-3,4-dihydro-2H-pyrrole 22g

Compound **22f** (120 mg, 0.93 mmol) was dissolved in isopropanol (5 mL), iodomethane (528 mg, 3.72 mmol) was added, and it was stirred at room temperature overnight. The reaction solution was filtered, and the filter cake was washed twice with diethyl ether and dried to obtain the title compound **22g** (160 mg, crude product), which was directly used for the next step.

¹H NMR (400 MHz, DMSO-d₆) δ 3.71 (s, 2H), 3.09 (s, 2H), 2.73 (s, 3H), 1.16 (s, 6H).

### Step 7: N-(2,2-dimethoxyethyl)-3,3-dimethyl-3,4-dihydro-2H-pyrrole-5-amine 22h

Compound **22g** (160 mg, crude product) was dissolved in ethanol (10 mL), and aminoacetaldehyde dimethyl acetal (66 mg, 0.63 mmol) was added at room temperature, and the temperature was lifted to 70°C for reaction for 3 hours. The reaction solution was concentrated to obtain the title compound **22h** (230 mg, crude product), which was directly used for the next step.

### Step 8: 6,6-dimethyl-6,7-dihydro-5H-pyrrolo[1,2-a]imidazole 22i

Compound **22h** (230 mg, crude product) was dissolved in 1,4-dioxane (10 mL), hydrochloric acid/1,4-dioxane (2 mL, 4N) was added at room temperature, it was heated to 90°C for reaction for 3 hours. The reaction solution was cooled to room temperature, neutralized with a saturated sodium carbonate solution and extracted with ethyl acetate, the organic phases were combined, washed with saturated salt water, dried over anhydrous sodium sulfate and concentrated, and the crude product was purified by silica gel column chromatography to obtain the title compound **22i** (60 mg, three-step yield 47.6%).

¹H NMR (400 MHz, DMSO-d₆) δ 7.02 (d, *J* = 1.2 Hz, 1H), 6.84 (d, *J* = 0.8 Hz, 1H), 3.67 (s, 2H), 2.55 (s, 2H), 1.19 (s, 6H).

### Step 9: 2-([2,3'-bipyridine]-5-yl)propanoic acid methyl ester 22j

Compound **19e** (2.9 g, 11.88 mmol) was dissolved in 1,4-dioxane (25 mL) and water (5 mL), 3-pyridineboronic acid (1.47 g, 11.96 mmol) was added, sodium carbonate (2.53 g, 23.87 mmol) was added at room temperature, it was heated to 100°C for reaction overnight, and the reaction was complete monitored by TLC. The reaction solution was cooled to room temperature, added with water and extracted with ethyl acetate, the organic phases were combined, washed with saturated salt water, dried over anhydrous sodium sulfate and concentrated, and the crude product was purified by silica gel column chromatography to obtain the title compound **22j** (1.75 g, yield 61%).

### Step 10: 2-([2,3'-bipyridine]-5-yl)propionic acid 22k

Compound **22j** (1.7 g, 7.02 mmol) was dissolved in tetrahydrofuran (20 mL), a sodium hydroxide aqueous solution (3.6 mL, 14.4 mmol, 4M) was added, the reaction was carried out for 1 hour at room temperature, and the reaction was complete monitored by TLC. The reaction solution was concentrated, adjusted to pH=5-6 with dilute hydrochloric acid (2N) and extracted with ethyl acetate, the organic phases were combined, washed with saturated salt water, dried over anhydrous sodium sulfate and concentrated, and the crude product was purified by silica gel column chromatography to obtain the title compound **22k** (930 mg, yield 58%).

### Step 11: 2-([2,3'-bipyridine]-5-yl)-N-(4-bromo-5-chloropyridin-2-yl)propionamide 221

Compound **22k** (800 mg, 3.50 mmol) and 4-bromo-5-chloro-2-aminopyridine (880 mg, 4.24 mmol) were dissolved in ethyl acetate (20 mL), 1-propylphosphonic anhydride (8.90 g, 13.99 mmol, 50% ethyl acetate solution) and triethylamine (2.13 g, 21.05 mmol) were added at room temperature, it was heated to 70°C for reaction for 2 hours. The reaction solution was cooled to room temperature, quenched with water and extracted with ethyl acetate, the organic phases were combined, washed with saturated salt water, dried over anhydrous sodium sulfate and concentrated, and the crude product was purified by silica gel column chromatography to obtain the title compound **22l** (1.2 g, yield 82%).

LC-MS: m/z=417.0 [M+H]⁺

### Step 12: (S)-2-([2,3'-bipyridine]-5-yl)-N-(5-chloro-4-(6,6-dimethyl-6,7-dihydro-5H-pyrrolo[1,2-a]imidazol-3-yl)pyridin -2-yl)propionamide (assumed) 22-1 &

### (R)-2-([2,3'-bipyridine]-5-yl)-N-(5-chloro-4-(6,6-dimethyl-6,7-dihydro-5H-pyrrolo[1,2-a]imidazol-3-yl)pyridin -2-yl)propionamide (assumed) 22-2

Compound **22t** (1.2 g, 2.87 mmol) and Compound **22i** (700 mg, 5.14 mmol) were dissolved in N,N-dimethylaniline (10 mL), palladium acetate (200 mg, 0.89 mmol) and potassium acetate (1.01 g, 10.29 mmol) were added at room temperature, it was heated to 120°C for reaction overnight. The reaction solution was cooled to room temperature, quenched with water and extracted with ethyl acetate, the organic phases were combined, washed with saturated salt water, dried over anhydrous sodium sulfate and concentrated, and the crude product was purified by silica gel column chromatography to obtain the title compound **22** (120 mg, yield 9%). Compound **22-1** (RT 15.09 min) (48 mg, yield 3.5%) and Compound 22-2 (RT 24.06 min) (62 mg, yield 4.6%) was obtained by chiral resolution (NANOMICRO OD-H, 30*250mm, 5um, 30mL/min, IPA:Hexane=40:60).

### Compound 22-1

LC-MS: m/z=473.2 [M+H]⁺

¹H NMR (400 MHz, DMSO-d₆) δ 11.06 (s, 1H), 9.23 (s, 1H), 8.74 (s, 1H), 8.62 (d, J =1.2 Hz, 1H), 8.43-8.39 (m, 2H), 8.26 (s, 1H), 8.04 (d, J = 8.0 Hz, 1H), 7.94 (d, J = 6.8 Hz, 1H), 7.49(m, 2H),4.16 (q, J=6.4 Hz 1H), 3.89 (s 2H), 2.70 (s, 2H), 1.51 (d, J =6.4 Hz, 3H), 1.23 (s, 6H). (98.39% purity by HPLC)

### Compound 22-2

LC-MS: m/z=473.2 [M+H]⁺

¹H NMR (400 MHz, DMSO-d₆) δ 11.09 (s, 1H), 9.23 (s, 1H), 8.74 (s, 1H), 8.62 (d, J 1=1.2 Hz, 1H), 8.46-8.37 (m, 2H), 8.27 (s, 1H), 8.03 (d, J = 8.0 Hz, 1H), 7.94 (d, J= 2.2 Hz, 1H), 7.54(d, J =12.4 Hz, 2H), 4.18 (q, J=6.4 Hz 1H), 3.91 (s 2H), 2.74 (s, 2H), 1.52 (d, J =6.4 Hz, 3H), 1.23 (s, 6H). (97.67% purity by HPLC)

### Test Example 1: Testing of Inhibitory Effect of the Compounds on the Kinase Activity of CDK7/CDK9 in Vitro

The detection of the inhibitory effect of the compounds of the present disclosure on the kinase activity of CDK7/CDK9 in vitro was determined by the following method:
1) The compound was accurately weighed and dissolved in DMSO (Sigma, D2650) to a concentration of 100 mM for later use. The compound was diluted to 5-fold of a desired highest concentration, and diluted 4-fold into 6 concentration gradients, allowing the final concentration of the compound in the reaction system to be CDK7: 3000, 750, 188, 47, 12, 3 nM; CDK9: 100, 25, 6.25, 1.56, 0.39, 0.098 nM. 5 ul was taken to a 384-well plate (Corning, 4512).
2) Kinase reaction: 10uL protein kinase (CDK7: Eurofilns,14-476M; CDK9: Millipore,14-685M) solution was added to the 384-well plate (Corning, 4512) containing the compound, and allowed to stand at room temperature for 10 min; a mixed solution of ATP(Sigma, A7699) and substrate polypeptide (CDK7: 77uM ATP&0.2ug/uL CTD3 peptide (GL Biochem,SY356885); CDK9: 10uM ATP&0.2ug/uL CTD3 peptide (GL Biochem,SY356885)) was added, and allowed to stand at 28°C for a short time; and then a stop solution was added to terminate the reaction at 25 uL per well.
3) Detection: Data was collected by Caliper.
4) Calculation: The IC50 value was calculated by Graphpad prism 5.0 software according to the concentration of the compound and the corresponding signal value. Test Results: See Table 1.

Conclusion: The compounds of the present disclosure have a significant inhibitory effect on CDK7/9 kinase activity.

### Test Example 2: Inhibition Effect of the Compounds on the Proliferation of Mv4-11 Cells

The inhibition effect of the compounds of the present disclosure on the proliferation of Mv4-11 cells (ATCC:CRL-9591^{™}) in vitro was determined by the following method:
1) Cell inoculation: Mv4-11 cells with good growing state in logarithmic growth phase were inoculated into a 96-well plate at 20000 cells per well and 50 uL, and cultured at 37°C and under 5% CO₂ condition for 2-4 h.
2) Administration: The compound was diluted with 1640 medium containing 10% FBS and 1% PS: 0.4 uL of the compound with an initial concentration of 1 mM was placed in a dilution plate, each added with 199.6 uL of the above culture medium, and gradiently diluted 4-fold, so that the final concentration in the added cell was 1000, 250, 62.5, 15.625, 3.91, 0.98, 0.24, 0.06 nM. 50 µL of the medium containing the compound was sequentially added, and it was placed in a 37°C, 5% CO₂ cell incubator for 48 hours.
3) Detection: 10uL CCK8 (Dojindo, CK 04) solution was added to each well, incubated in 37°C. 5% CO₂ cell incubator for 2 hours, and then the OD450 value was read by Synegry H1(BioTek) multimode microplate reader.
4) Calculation: The IC50 value was calculated using Graphpad prism 5.0 software according to the concentration of the compound and the corresponding signal value. Test Results: See Table 1.

**TABLE 1 Inhibition of the Compounds of the Present Disclosure on the Kinase Activity of CDK7/9 and Mv4-11 cells IC50(nM)**

| Compound No. | CDK 9 | CDK 7 | Mv4-11 | Compound No. | CDK9 | CDK7 | Mv4-11 |
|---|---|---|---|---|---|---|---|
| AZD4573 | 2.8 | 109.2 | 4.2 | 13-1 | 44.4 | - | 148.3 |
| 1 | 3.0 | 96 | 7.4 | 13-2 | 3.0 | 328 | 4.8 |
| 2-1 | - | - | > 1000 | 14-1 | 2.3 | - | 10.1 |
| 2-2 | 2.7 | 19 | 1.6 | 14-2 | - | - | 136.2 |
| 3 | 4.0 | 103 | 3.9 | 15-1 | 5.9 | - | 11.2 |
| 4 | - | - | 10.7 | 15-2 | - | - | 160.9 |
| 5 | - | - | 26.0 | 16-1 | 3.4 | - | 6.4 |
| 6 | 3.1 | 197 | 9.9 | 16-2 | - | - | 468.9 |
| 7 | 1.7 | 118 | 8.2 | 18-2 | 3.0 | 74.0 | 25.0 |
| 8 | 3.4 | 116 | 19.6 | 19-1 | - | - | 13.9 |
| 9 | - | - | 13.0 | 20-1 | 8.4 | 25.1 | 3.7 |
| 11-1 | - | - | 893.1 | 20-2 | - | - | 849.8 |
| 11-2 | 2.9 | - | 13.0 | 22-1 | - | - | 2.6 |
| 12 | 5.2 | - | 12.2 | 22-2 | - | - | 78.1 |

| | | | | | | | |
|---|---|---|---|---|---|---|---|
| Conclusion: The compounds of the present disclosure have a significant inhibitory effect on CDK9. | | | | | | | |

### Test Example 3: Determination of the CDK7/CDK9 signaling pathway in Mv4-11 cells

The inhibition effect of the compounds of the present disclosure on CDK7/CDK9 protein downstream RNA pol II Ser 2/5 in Mv4-11 cells in vitro was determined by the following method:
1) Cell inoculation: Mv4-11 cells with good growing state in logarithmic growth phase were inoculated into a six-well plate with 2*105 cells/well, and cultured overnight at 37°C and under 5% CO₂ condition.
2) Administration: The compound was diluted with 2mL of 1640 medium containing 10% FBS and 1% PS. 2 ul of the medium containing the compound was added to the overnight cultured cells, and it was placed in a 37°C, 5% CO₂ cell incubator for 6 hours.
3) Protein extraction and quantification: Cell suspension was collected and centrifuged at 1000 g for 5 min, the cell culturing medium was discarded, and PBS was added for resuspension and centrifugation; it was repeated three times, the residual liquid was sucked dry, 80 uL cell lysate was added per well, it was placed to ice, shaked for 10 min on a shaker and centrifuged at 12000 g for 5 min, and the supernatant, ie, the total protein solution, was collected; the protein concentration was tested by BCA.
4) 12% SDS-PAGE electrophoresis was used to detect, after that trarsmembran was carried out at 100V voltage for 3h, blocking solution (Beyotime: P0235) was used to block for 15 min, TBST(Sangon Biotech: C520002) was used to wash the membrane three times, and the primary antibody (target protein CST:13499, internal reference Beyotime: AF1186) was incubated at 4°C overnight; the membrane was washed with TBST three times; the secondary antibody (Beyotime: A0208) was incubated at room temperature for 1 hour; the membrane was washed with TBST three times; ECL (Tanon: 180-501) was used for exposure and coloration.

The phosphorylation effect of compounds 2-2, 3, 11-2, 13-1, 13-2 and AZD4573 on RNA pol II Ser2/5 was shown in FIG. 1.

Conclusion: The compounds of the present disclosure such as 2-2, 3, 11-2, and 13-2 have a significant inhibitory effect on the phosphorylation of the CDK7 protein downstream RNA pol II Ser5 in acute myeloid leukemia (AML) Mv4-11 cells; the compounds such as 2-2, 11-2, and 13-1 have a significant inhibitory effect on the phosphorylation of CDK9 protein downstream RNA pol II Ser2.

The above are merely embodiments of the present disclosure, and the description thereof is relatively specific and detailed, but cannot be understood as a limitation to the patent scope of the present disclosure. It should be noted that a person of ordinary skill in the art may make several variations and improvements without departing from the concept of the present disclosure, and these are all within the protection scope of the present disclosure.

## Claims

1. A pyridine acetamide derivative, a pharmaceutically acceptable salt thereof, a tautomer thereof or a stereoisomer thereof, wherein the structure of the pyridine acetamide derivative is represented by formula (I): 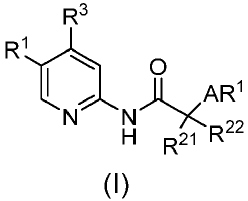 wherein:
R¹ is selected from hydrogen, halogen, cyano, substituted or unsubstituted C₁-C₃ alkyl, or substituted or unsubstituted C₁-C₃ alkoxy, and "substituted" refers to optionally substituted with 1-3 halogens;
R²¹ and R²² are independently selected from hydrogen, halogen, hydroxyl, cyano, amino, substituted or unsubstituted C₁-C₃ alkyl, and substituted or unsubstituted C₁-C₃ alkoxy, wherein substituted herein refers to optionally substituted with 1-3 halogens, hydroxyl groups, cyano groups, or amino groups;
AR¹ is selected from phenyl, 5-6 membered heteroaryl, 8-10 membered fused aryl, and 8-10 membered fused heteroaryl, wherein phenyl, 5-6 membered heteroaryl, 8-10 membered fused aryl, and 8-10 membered fused heteroaryl are optionally further substituted with one or more R^{a};
R^{a} is independently selected from C₁-C₃ alkyl, hydroxyl, halogen, cyano, amino, C₁-C₃ alkoxy, C₃-C₆ cycloalkyl, C₃-C₆ heterocyclyl, phenyl, 5-6 membered heteroaryl, S(O)R^{b1}, S(O)₂R^{b1}, S(O)NH₂, S(O)NHR^{b1}, S(O)NR^{b1}R^{b2}, S(O)₂NH₂, S(O)₂NHR^{b1}, S(O)₂NR^{b1}R^{b2}, NHS(O)R^{b1}, NR^{b1}S(O)R^{b2}, NHS(O)₂R^{b1}, NR^{b1}S(O)₂R^{b2}, C(O)R^{b1}, C(O)OR^{b1}, OC(O)R^{b1}, NHC(O)R^{b1}, NR^{b1}C(O)R^{b2}, NHC(O)OR^{b1}, NR^{b1}C(O)OR^{b2}, C(O)NH₂, C(O)NHR^{b1}, and C(O)NR^{b1}R^{b2}, wherein alkyl, alkoxy, cycloalkyl, heterocyclyl, phenyl, and 5-6 membered heteroaryl are optionally further substituted with 1-3 alkyl groups, hydroxyl groups, halogens, cyano groups, amino groups, alkoxy groups, acryloyl groups or acryloyl methylene groups;
R^{b1} and R^{b2} are independently selected from C₁-C₃ alkyl, 3-6 membered cycloalkyl or heterocyclyl, wherein alkyl, cycloalkyl, and heterocyclyl are optionally further substituted with 1-3 substituents selected from alkyl, hydroxyl, halogen, cyano, amino or alkoxy, or R^{b1} and R^{b2} together with the N atom to which they are bonded form a 3-7 membered heterocyclyl group;
R³ is selected from: 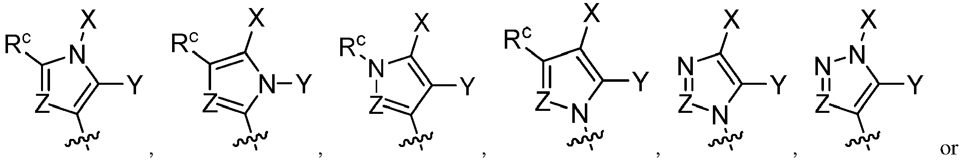 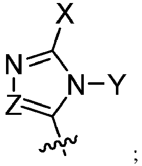
Z is N or CR^{c};
R^{c} is independently selected from hydrogen, halogen, cyano, C(O)NH₂, C(O)NHR^{b1}, C(O)NR^{b1}R^{b2}, C(O)R^{b} or C₁-C₃ alkyl, wherein alkyl is optionally further substituted with 1-3 substituents selected from alkyl, hydroxyl, halogen, cyano, amino or alkoxy;
X and Y together with the atoms to which they are bonded form a 5-7 membered heterocyclyl or cycloalkyl group, wherein the heterocyclic group comprises 1-2 heteroatoms selected from N, O, S; wherein the 5-7 membered heterocyclyl or cycloalkyl group is saturated or partially saturated, and the ring carbon or ring S atom is optionally further substituted with 1-3 R^{d};
R^{d} is independently selected from halogen, hydroxyl, cyano, =O or C₁-C₃ alkyl, wherein alkyl is optionally further substituted with 1-3 substituents selected from alkyl, hydroxyl, halogen, cyano, amino or alkoxy.

2. The pyridine acetamide derivative, the pharmaceutically acceptable salt thereof, the tautomer thereof or the stereoisomer thereof according to claim 1, wherein the structure of the pyridine acetamide derivative is represented by formula (II): 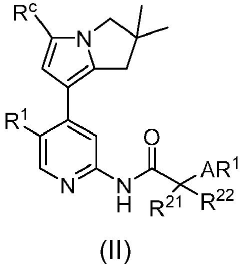 wherein R¹, R²¹, R²², AR¹, and R^{c} have the same defined ranges as in claim 1.

3. The pyridine acetamide derivative, the pharmaceutically acceptable salt thereof, the tautomer thereof or the stereoisomer thereof according to claim 1, wherein the structure of the pyridine acetamide derivative is represented by formula (III): 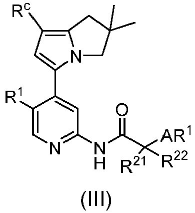 wherein R¹, R²¹, R²², AR¹, and R^{c} have the same defined ranges as in claim 1.

4. The pyridine acetamide derivative, the pharmaceutically acceptable salt thereof, the tautomer thereof or the stereoisomer thereof according to claim 1, wherein the structure of the pyridine acetamide derivative is represented by formula (IV): 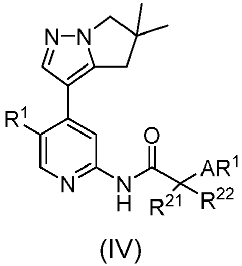 wherein R¹, R²¹, R²², and AR¹ have the same defined ranges as in claim 1.

5. The pyridine acetamide derivative, the pharmaceutically acceptable salt thereof, the tautomer thereof or the stereoisomer thereof according to claim 1, wherein the structure of the pyridine acetamide derivative is represented by formula (V): 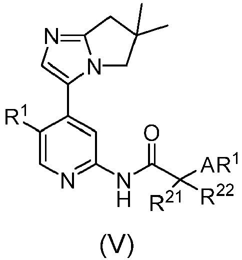 wherein R¹, R²¹, R²², and AR¹ have the same defined ranges as in claim 1.

6. The pyridine acetamide derivative, the pharmaceutically acceptable salt thereof, the tautomer thereof or the stereoisomer thereof according to claim 1,wherein the pyridine acetamide derivative is selected from any one of the following structures:

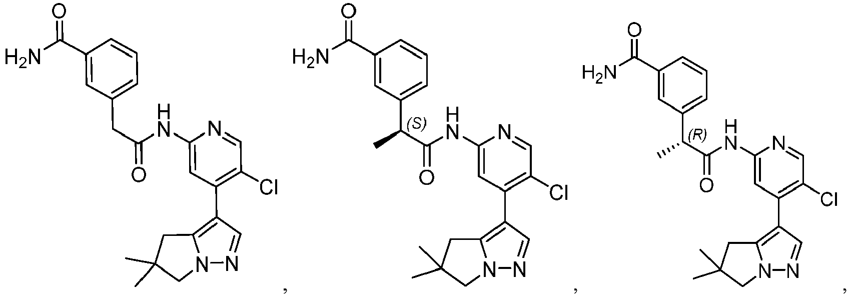

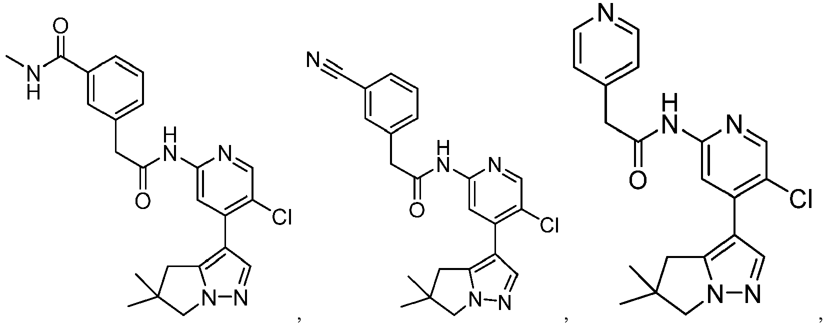

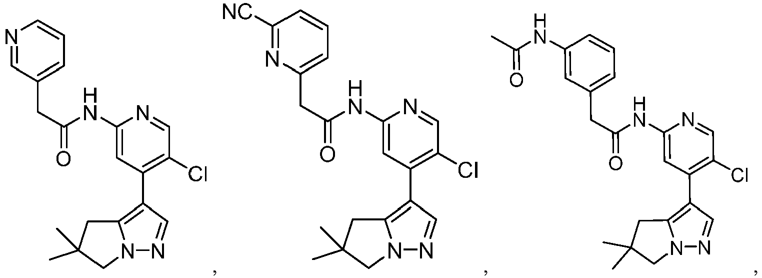

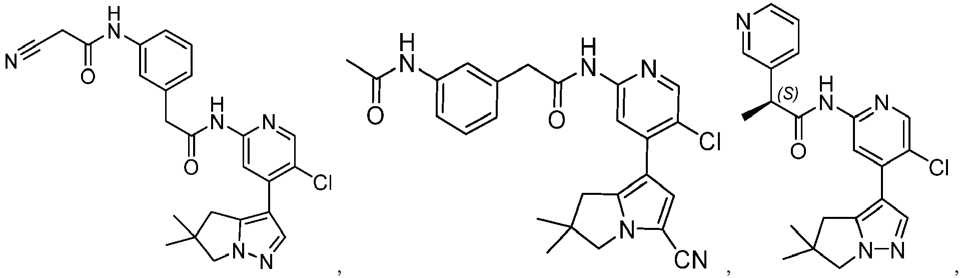

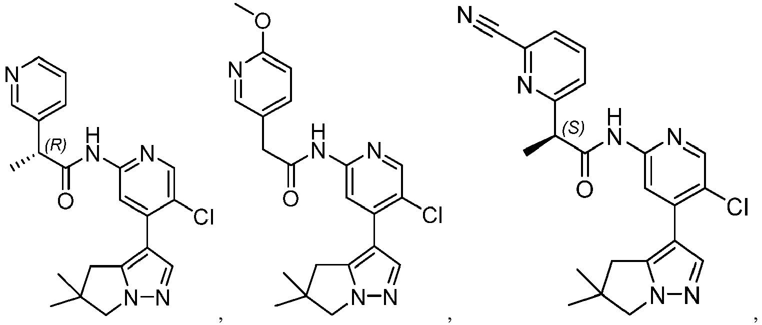

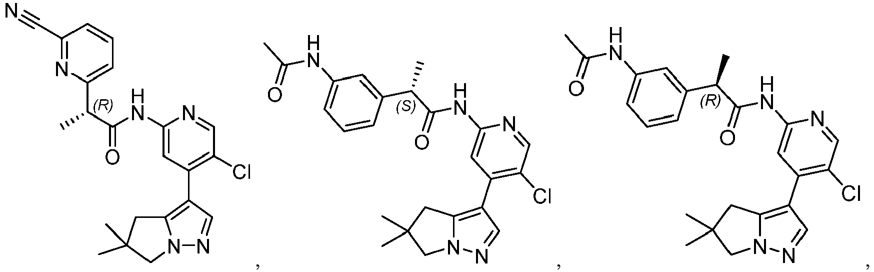

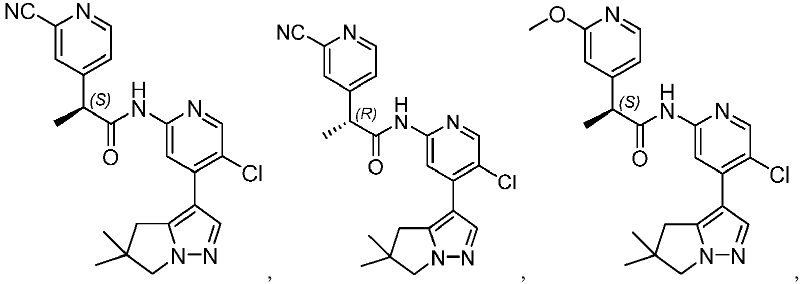

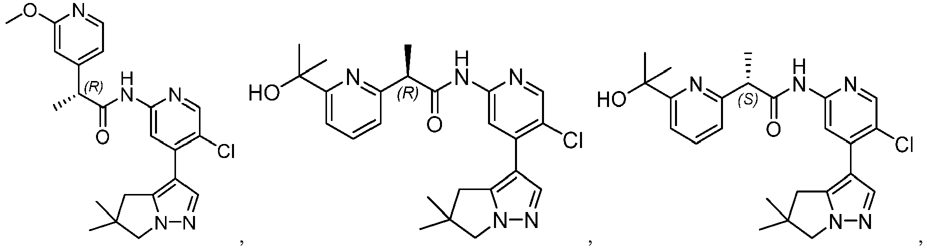

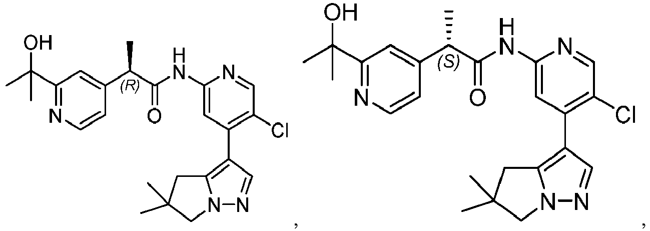

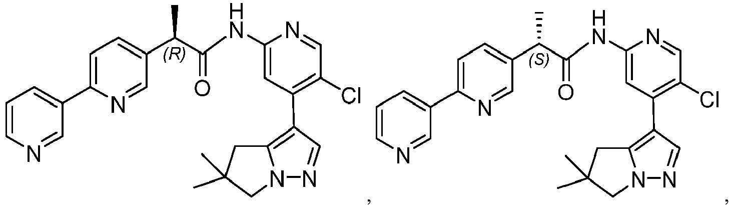

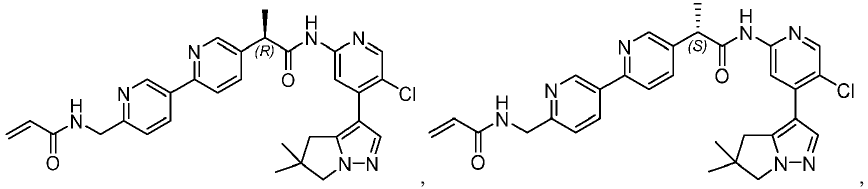

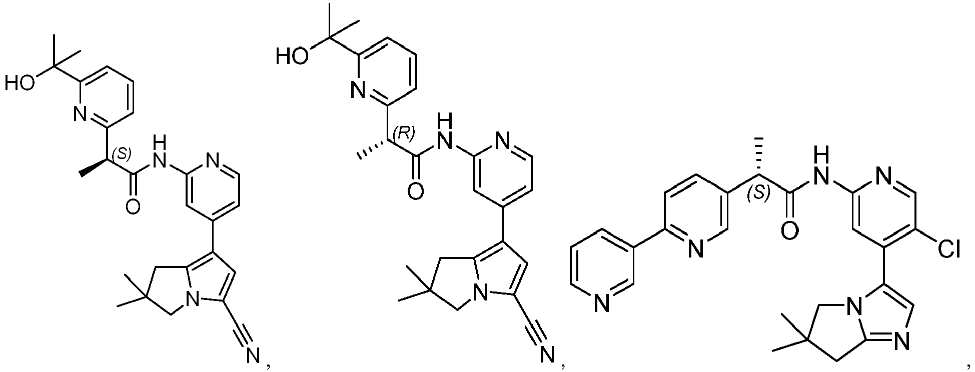

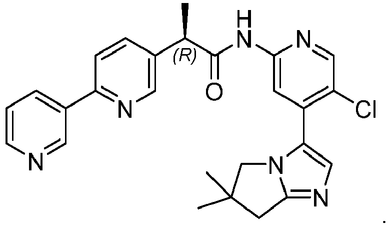

7. A preparation method of the pyridine acetamide derivative, the pharmaceutically acceptable salt thereof, the tautomer thereof or the stereoisomer thereof according to any one of claims 1-6, selected from one of the following three methods:
Method One: 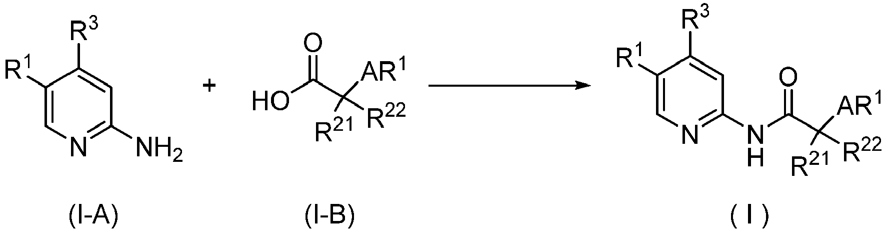
Method Two: 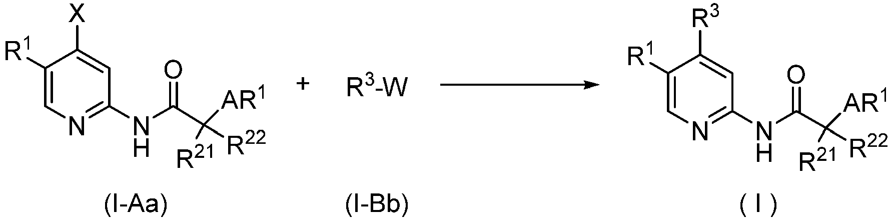
Method Three: 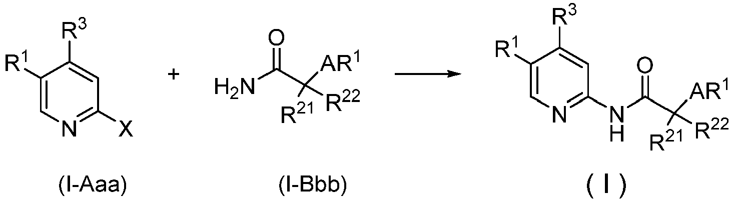 wherein W is 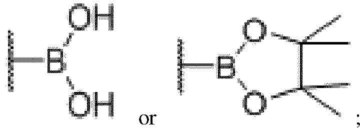 X is halogen; R¹, AR¹, R²¹, R²², and R³ have the same defined ranges as in claim 1.

8. A pharmaceutical composition, wherein the pharmaceutical composition comprises the pyridine acetamide derivative, the pharmaceutically acceptable salt thereof, the tautomer thereof or the stereoisomer thereof according to any one of claims 1-6; preferably, the pharmaceutical composition further comprises a pharmaceutically acceptable carrier and/or an excipient.

9. An application of the pyridine acetamide derivative, the pharmaceutically acceptable salt thereof, the tautomer thereof or the stereoisomer thereof according to any one of claims 1-6, or the pharmaceutical composition according to claim 8 in the preparation of medicaments for treating cancers, or in preparing inhibitors for CDK families;
preferably, the cancer is blood cancer, further preferably acute myeloid leukemia, multiple myeloma, chronic lymphocytic leukemia, follicular lymphoma, or solid tumor; still further preferably, the solid tumor is breast cancer, prostate cancer, ovarian cancer, hepatocellular carcinoma, pancreatic cancer, kidney cancer, gastric cancer, colorectal cancer or lung cancer.

10. A method for treating cancers by administering to a subject suffering from cancers an effective amount of the compound according to any one of claims 1-6.
